# EUROPEAN PATENT APPLICATION

(11) **EP 3 447 154 A1**
(43) Date of publication of application: **27.02.2019**
(21) Application number: 17460047.8
(22) Date of filing: 23.08.2017
(51) Int. Cl.: C12Q 1/6827, C12Q 1/6837

(54) **METHOD FOR DETECTION OF MUTATIONS, POLYMORPHISMS AND SPECIFIC DNA SEQUENCES ON DNA MATRICES WITH DNA IMAGING TECHNIQUES FOR THE USE IN MEDICAL DIAGNOSTICS AND FORENSIC GENETICS**

(71) Applicant: Instytut Genetyki Sadowej Jolanta Powierska - Czarny, 85-071 Bydgoszcz (PL)
(72) Inventor: Jakub, Czarny, 86-005 Kruszyn Kraje?ski (PL)
(74) Representative: Twardowska, Aleksandra

(57) **Abstract**

The invention relates to a method for the detection of mutations, polymorphisms and specific DNA sequences on DNA matrices with DNA IMAGING technology for use in medical diagnostics and forensic genetics, in particular the method relates to the detection of mutations in CFTR, BRCA1 and/or BRCA2 genes. The invention also relates to starters for use in the detection of mutations in CFTR, BRCA1 and BRCA2 genes.

## Description

The invention relates to the detection of mutations, polymorphisms and specific DNA sequences on DNA matrices with DNA IMAGING technology for use in medical diagnosis and forensic genetics, in particular the method relates to the detection of mutations in the CFTR, BRCA1 and/or BRCA2 genes. The invention also relates to starters for use in the detection of mutations in the CFTR, BRCA1 and BRCA2 genes.

### State of the art

In the course of numerous research a number of diseases which are based on inherited changes in the DNA, so called "mutations", have been identified. Said changes might be different in nature: from quantitative changes at the chromosome level to minor changes in genetic information. Even a change of a single nucleotide in the 6 billion base pair genetic information of a cell can lead to an increased risk of developing cancer or serious metabolic diseases. Some mutations can appear spontaneously, however, many of them occur in the general population with certain frequencies, in specific areas of the genome, and are responsible for specific disease symptopms.

For these reasons, methods for the detection of mutations are of fundamental importance in the diagnosis of genetic diseases. In the case of mutation detection methods, the most important method parameters include the following: sensitivity, specificity, possibility of multiplexing, i.e. detection of multiple mutations at the same time, and cost of analysis. In particular, it should be noted that in the case of such diseases as for example genetically determined susceptibility to breast cancer and ovarian cancer over a 100 mutations in several genes have been described.

According to the knowledge of the person skilled in the art, mutations in the DNA can be detected with non-specific methods, such as SSCP, DNA heteroduplexes analysis, temperature or denaturing gradient electrophoresis. These methods are currently going out of use due to their non-specificity and relatively small possibility of multiplexing.

A variant of PCR method for the detection of mutations has been developed, which consists in designing amplification starters at the mutation site, assuming that the mutation would cause incapability of DNA amplification, however, further research has shown that this assumption was wrong and significantly affected the specificity of the method. Also possibilities of multiplexing of the allelospecific method were limited. A different variation of the PCR reaction has shown much more specificity, estimated at 100%, namely real-time PCR, in which even a few mutations can be detected in one reaction through the use of fluorescent labeled probes with a silencing molecule binding to the area that contains the specific sequence. Possibility of multiplexing is a limitation of this method, which in the case of numerous mutations in multigenic disorders renders this method useless for genetic diagnostics.

DNA sequencing methods that employ base by base reading of nucleotide sequences, where not only known mutations can be detected but also those not described before, are described as most accurate. In a standard variant, DNA sequencing method does not allow for multiplexing. So called next generation DNA sequencing methods allow for mass determination of hundreds and thousands of DNA sequence fragments. Complexity, labouriousness, time consumption and high costs as well as sequencing errors occurring with this method are limitations to popularizing it in genetic diagnostics for standard use. Over the course of years many different molecular genetics methods dedicated for mutation detection have been developed, however, they have not found a broader application in genetic diagnostics.

Hybridization methods offer great possibilities for the detection of mutations in DNA, where the analyzed DNA fragments obtained with PCR amplification are subject to hybridization, that is, DNA sequence conditioned binding to short DNA fragments immobilized on a substrate. Basically there are two strategies for designing and detecting results in hybridization research. One approach assumes obtaining through amplification fluorescently labeled DNA fragments, which are hybridized with probes - normal type, unchanged (in genetics referred to as wild type) DNA fragments and probes containing a mutation.

The disadvantage of this approach is the need to control the conditions of the process of binding DNA fragments to the probes, so as to obtain high specificity and reliability of research. The specificity of binding DNA segments should be ensured by: temperature and ionic strength of the solution in which hybridisation is carried out. Another problem is ensuring access of DNA fragments to all areas on which the probes are located. This method has a very high potential of multiplexing, however, it has limited specificity and requires a long process of hybridization and a large quantity of analyzed and labeled DNA fragments.

The second approach is employing in research a variant of DNA sequencing, where on a solid substrate are bound short sections of DNA, located below the mutation site, and using a variant of DNA polymerase, which after joining to that probe a complementary DNA fragment inserts in the order conditioned by the matrix sequence one of four dideoxyanalogues nucleotide triphosphates, each of which labelled with a different fluorochrome. The reaction is single-stage and ends with the addition of only one nucleotide, whose type depends on the matrix sequence. The result is read with a matrix scanner using four different fluorescence excitation wavelengths and four different cut-off filters. The method requires a large amount of matrix to provide an appropriate signal level and a relatively large amount of reagents, because the whole reaction takes place in one stage.

The present invention provides a solution to said problems.

### Summary of the invention

Disclosed herein is a method for the detection of mutations, polymorphisms and specific DNA sequences on DNA matrices, which runs a reaction of binding oligonucleotides with sequences complementary to the 3' region directly preceding the detected sequence to a probe of such a sequence that after binding one nucleotide the mutated variant and the wild type variant would differ in the kind of the complement nucleotide successive in the 3' direction of the probe sequence, and then during matrix analysis bound molecules are amplified by PCR with thermostable DNA polymerase specific for dideoxynucleotide phosphates, which performs matrix sequence conditioned elongation by one nucleotide of oligonucleotides bound to the vehicle, in cycles of alternating denaturation and addition, after which matrix minisequencing is being performed on the collected and purified amplification products in an amplification reaction and based on that a multicolor scan is performed, which results in labeling mutations in the examined sequence.

In a preferred embodiment, the examined sequence is a mutation, particularly said mutation is a mutation in CFTR or BRCA1 and/or BRCA2 gene.

In a preferred embodiment of the invention, each probe at the 5' end has C12-AMINO modification and/or 18dT sequence preceding the proper oligonucleotide sequence.

For the detection of mutations in CFTR gene the following starters of SEQ ID NO: 235-288 are utilized, as shown in table 4.;
and for the detection of mutations in BRCA1 and BRCA2 genes the following starters of SEQ ID NO: 159-234 are utilized, as shown in table 3.

The invention is depicted in a drawing, where:
Fig. 1 shows a flow chart of an embodiment of the method of the invention,
Fig. 2 shows a matrix with submatrices for the analysis of separate samples,
Fig. 3 shows a matrix with an applied mask, forming 4 submatrices,

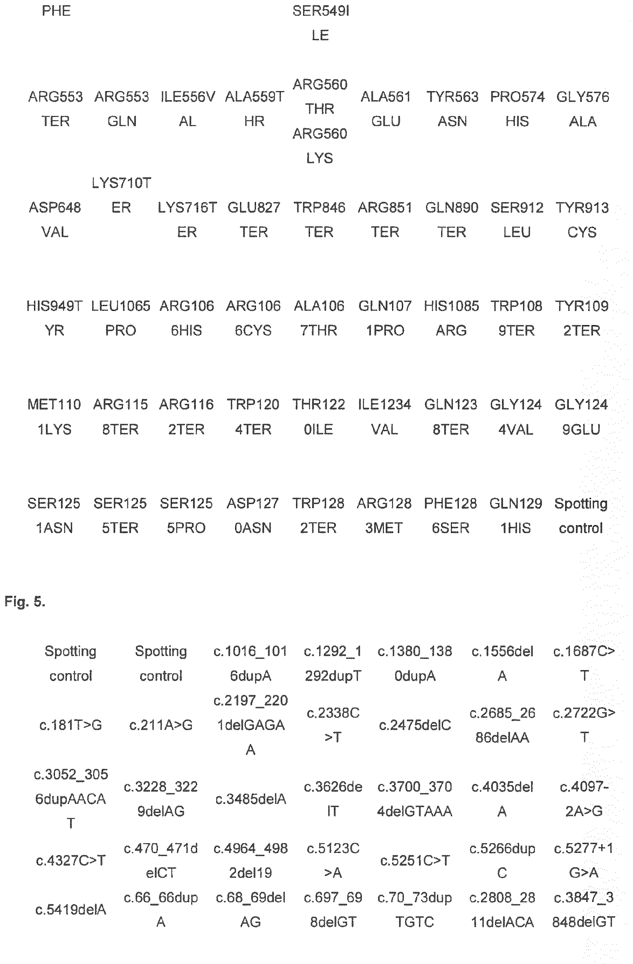

Fig. 4 and 5 show positioning of probes on the matrix,

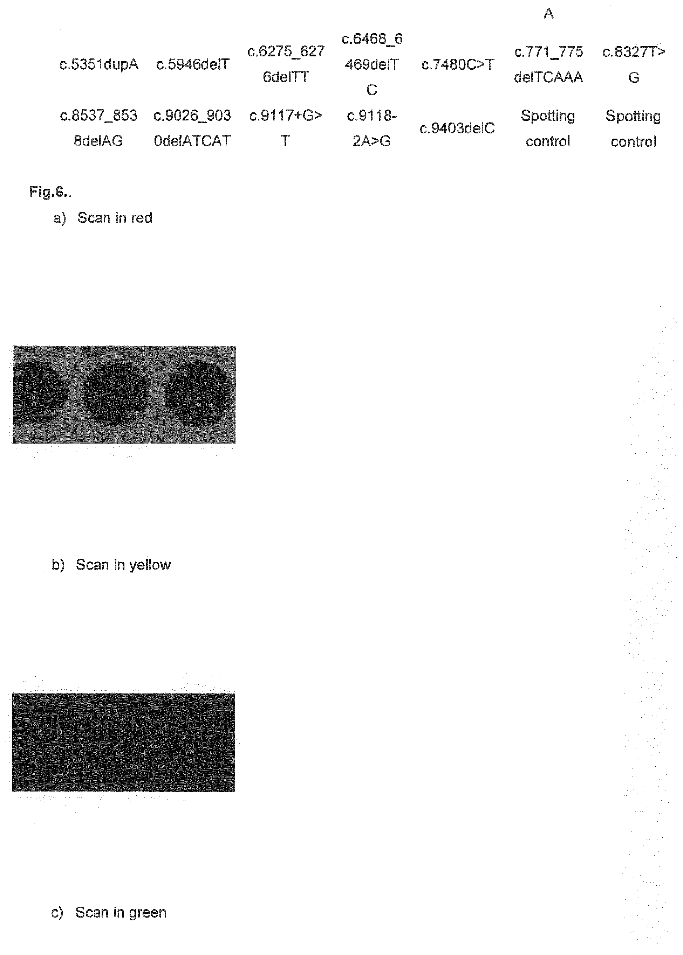

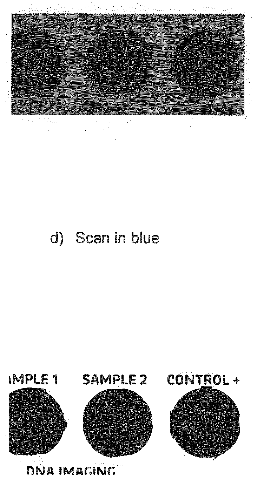

Fig. 6 shows quality control of matrix spotting - spotting controls are shown in red, in dedicated places,
Fig. 7 shows the results of multiplex amplification of BRCA1 gene fragments, of which
Fig. 7A. shows multiplex A: exons 2, 11.1 and 11.9 of BRCA1 gene and 9 and 11.1 of BRCA2 gene,
Fig. 7B. shows multiplex B: exons 5, 11.2, 11.10 of BRCA1 gene and 11.2 and 15 of BRCA2 gene,
Fig. 7C shows multiplex C: exons 8, 11.3 and 11.11 of BRCA1 gene and 11.3 and 1 of BRCA2 gene,
Fig. 7D shows multiplex D: exons 11. And 13 of BRCA1 gene and 11.4 and 20 of BRCA2 gene,
Fig. 7E shows multiplex E: exons 11.5 and 16 of BRCA1 gene and 11.5 and 23 of BRCA2 gene,
Fig. 7F shows multiplex F: exons 11.6 and 18 of BRCA1 gene and 11.6 and 24 of BRCA2 gene,
Fig. 7G shows multiplex G: exons 11.7 and 20 of BRCA1 gene and 11.7 and 25 of BRCA2 gene,
Fig. 7H shows multiplex H: exons 11.4, 11.12, 12 and 23 of BRCA1 gene,
Fig. 8. shows an example of matrix analysis of mutations in BRCA1/BRCA2 genes, mutation BRCA1 C>A c.68_69deIAG is marked.

### Detailed description of the invention

The essence of the invention is the use of thermostable DNA polymerase in matrix analysis, said polymerase being specific for dideoxynucleotide phosphates, and said polymerase performing matrix sequence conditioned elongation by one nucleotide of oligonucleotides bound to the vehicle, in 60 cycles of alternating denaturation and addition. In such conditions a small amount of matrix fragments is sufficient to obtain a reliable result identifying specific DNA sequences in the course of 2-3 hours without a need of sophisticated conditions control. Due to periodicity of the reaction, already high specificity of the reaction was increased even more, as the elongation of probes with a fluorescently labeled dideoxynucleotide takes place not once but 60 times, which significantly lowers the probability of error.

In more detail, the method of the invention comprises preparation of a matrix on a glass slide with SUPEREPOXY surface. Then, a 73 x 26 mm mask is prepared using 0.35 mm thick self-adhesive PVC foil, cut with a laser plotter and forming 4 separate areas on the slide: in each fragment of the foil a 14 mm diameter circle is cut out, 2.5 mm from the edge of the slide and at regular distances of 4 mm (from the edges of circles).

Said mask is then applied on the active surface of the slides. The matrices are printed with contact method on a matrix printer SpotBot 2 Array Printer (Arraylt) (sequences of oligonucleotide probes are shown in tables 1 and 3). To wash out unbond DNA, matrices are washed twice for 2 min. at room temperature (22-25°C) in 500 ml of 0.1% SDS solution and once in 500 ml of deionized water with intense mixing in a High Throughput Wash Station. Such prepared matrices are dried by centrifugation for 1 min., 500xg in a Microarray High Speed Centrifuge. To block background fluorescence, matrices are incubated for 60 min. at temperature of 60°C in 500 ml of Blocklt Buffer and are dried by centrifugation for 1 min., 500xg in a Microarray High Speed Centrifuge. To perform matrix spotting quality control, a scan of spotting control in red is performed, in dedicated areas. Additionally, scan in red, yellow, green and blue is performed.

Then the DNA is isolated on an automated workstation QIAsymphony (QIAGEN) with a reagent kit QIAsymphony DNA Investigator Kit (Qiagen) and the DNA concentration is determined by a fluorimetric method with a Qubit 2.0 apparatus (Life Technologies) using a Qubit ®dsDNA BR assay kit (Life Technologies). PCR amplification is performed in 8 multiplex PCR reactions in 50 µl volume, in 200 µl PCR tubes (amplification conditions in the text, PCR starter sequences in multiplex reactions are shown in tables 2 and 4). PCR products are assessed for throughput with capillary electrophoresis on a QIAxcelAdvanced device (QIAGEN).

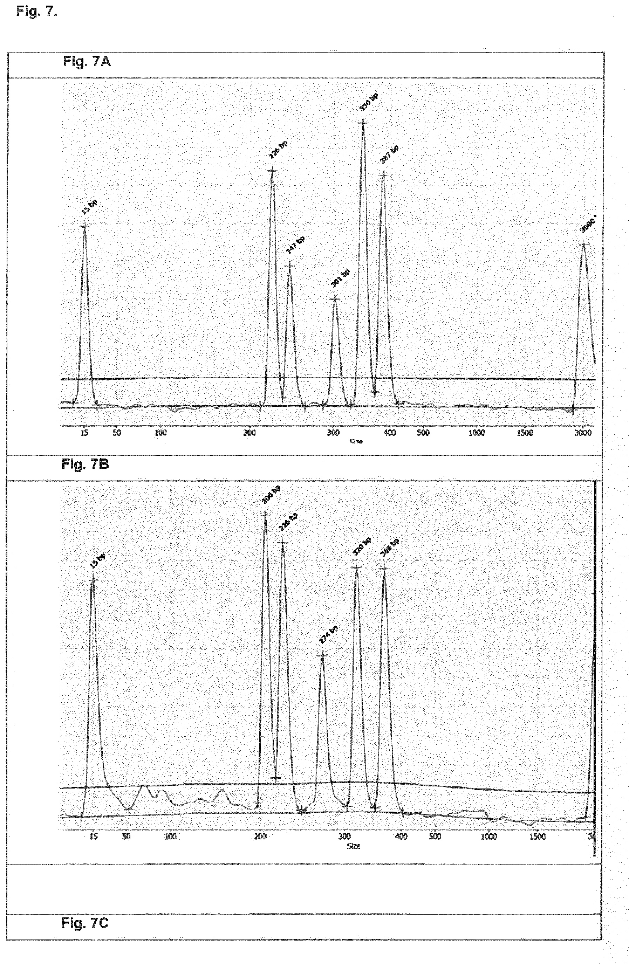

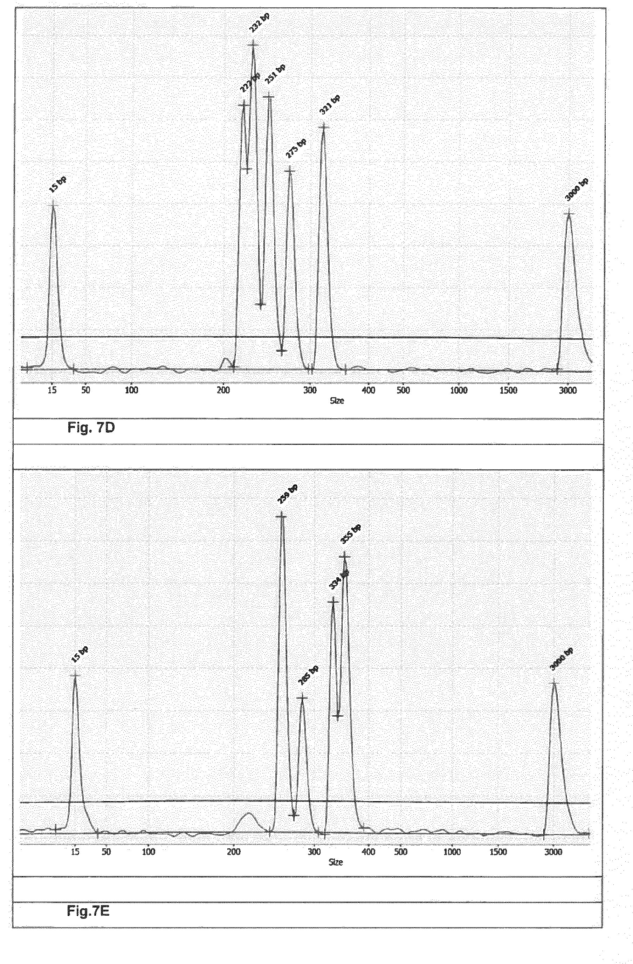

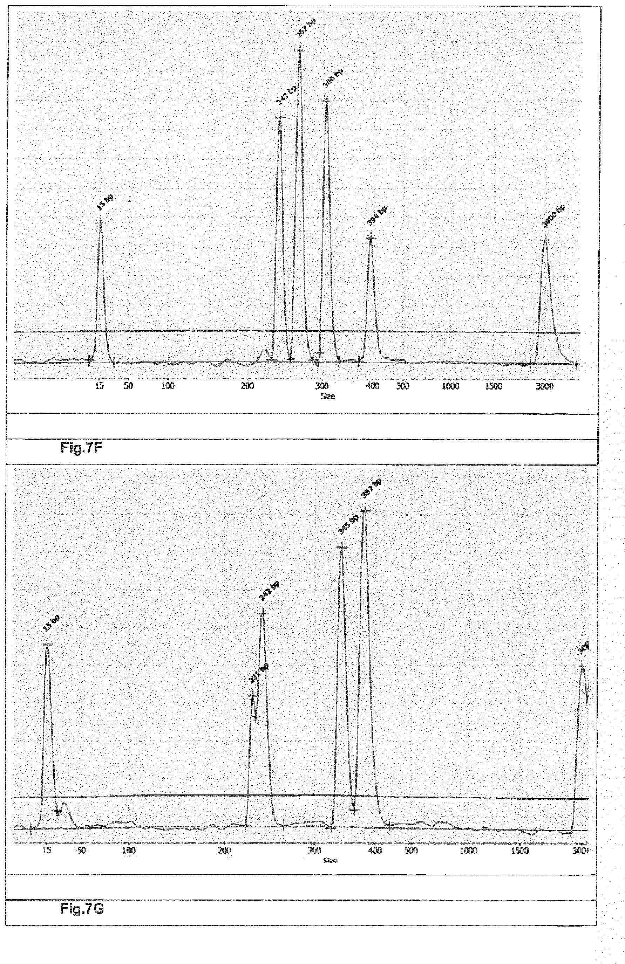

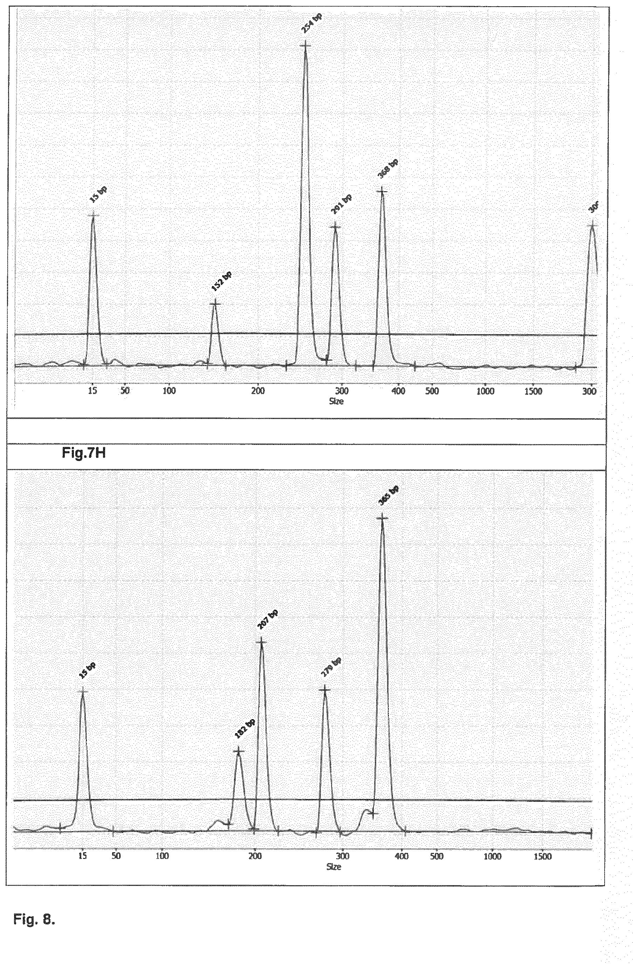

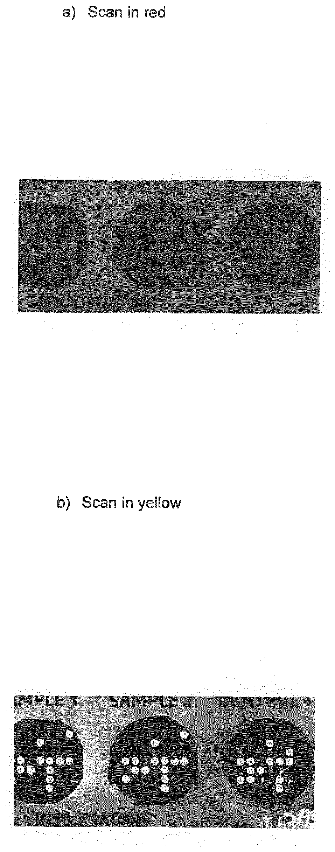

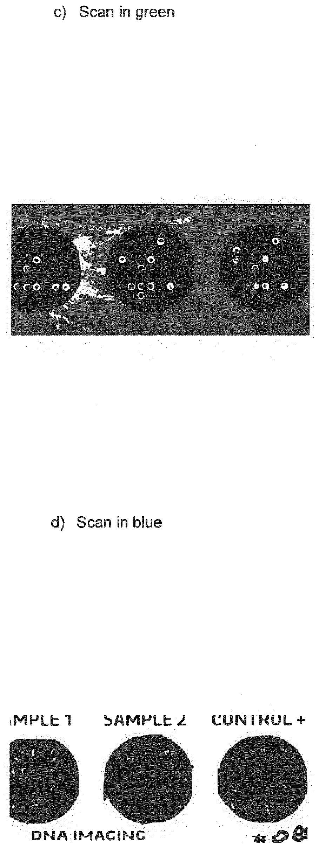

BRCA1 gene fragments multiplex amplification results are shown on figures 7A-G:
multiplex A: exons 2, 11.1 and 11.9 of BRCA1 gene and 9 and 11.1 of BRCA2 gene,
multiplex B: exons 5, 11.2, 11.10 of BRCA1 gene and 11.2 and 15 of BRCA2 gene,
multiplex C: exons 8, 11.3 and 11.11 of BRCA1 gene and 11.3 and 18 of BRCA2 gene,
multiplex D: exons 11.8 and 13 of BRCA1 gene and 11.4 and 20 of BRCA2 gene,
multiplex E: exons 11.5 and 16 of BRCA1 gene and 11.5 and 23 of BRCA2 gene,
multiplex F: exons 11.6 and 18 of BRCA1 gene and 11.6 and 24 of BRCA2 gene,
multiplex G: exons 11.7 and 20 of BRCA1 gene and 11.7 and 25 of BRCA2 gene,
multiplex H: exons 11.4, 11.12, 12 and 23 of BRCA1 gene.

Then, reaction mixtures are applied on submatrices and the matrix is sealed with PCR plates sealing foil cut to matrix size (76 x 26 mm). Cyclic reaction is performed in a thermocycler for amplification on microscope slides . After the amplification the sealing foil is taken off and the matrix is placed in a wash station and washed 2x2 min. in deionized water and then dried with centrifugation in a minicentrifuge for matrix centrifugation.

Then, minisequencing is performed, where the matrix is analyzed with a multicolor scanner GenePix 4300A (Molecular Devices). The presence of fluorescent signal in one color shows the presence of only one sequence variant, corresponding to said color. The presence of signal in two colors in one probe confirms the presence of a mutation.

### Working example of the invention:

4 round areas 9 mm in diameter are separated on SUPEREPOXY slides with 0.25 mm thick foil (submatrices), on which in predetermined places are deposited oligonucleotides with sequences complementary to the 3' region directly preceding a mutation undergoing detection. The probe has to be designed in such a way that after adding one nucleotide the mutated variant and the wild type variant differ in the kind of complementary nucleotide successive in the 3' direction of the probe sequence. At the 5' end each probe has C12-AMINO modification and 18dT sequence preceding the proper oligonucleotide sequence. A matrix preparation control of sequence 5'-C12-AMINO-(dT)₁₈-ACG TAC GTA CGT ACG TAC GTA CGT-Cy5-3' is deposited on every submatrix.

All 4 submatrices on the slide are printed with the same layout. After probe deposition, 12 hrs binding at room temperature and <30% humidity is performed followed by double washing in 0.1% SDS and double washing in water.

The following mutations and corresponding oligonucleotides (probes) for the diagnostics of hereditary mutations in BRCA1 and BRCA2 genes were selected - table 1:

**Table 1**

| gene | mutation | SNaPshot probe |
|---|---|---|
| BRCA1 | c.1016_1016dupA | GACTCCCAGCACAGAAAAAAA |
| | c.1292_1292dupT | ATTCTGGTTCTTCAGAGAAAATAGACTT |
| | c.1380_1380dupA | CAAATCAGTAGAGAGTAATATTGAAGACAAAATA |
| | c.1556delA | GCCTTCATCCTGAGGATTTTATCA |
| | c.1687C>T | CATGAGAATAAAACAAAAGGTGATTCTATT |
| | c.181T>G | CAGAAGAAAGGGCCTTCACAG |
| | c.211A>G | GTGTCCTTTATGTAAGAATGATATAACCAAA |
| | c.2197_2201delGAGAA | CAGCATTATTAGACACTTTAACTGTTTCTAGT |
| | c.2338C>T | GTACCTGGTACTGATTATGGCACT |
| | c.2475delC | CCAATGGATACTTAAAGCCTTCTGT |
| | c.2685_2686delAA | CACTCTGGGTCCTTAAAGAAACA |
| | c.2722G>T | CACTTTTGAATGTGAACAAAAGGAA |
| | c.3052_3056dupAACAT | CACTTTTGAATGTGAACAAAAGGAA |
| | c.3228_3229delAG | GAAAGAGAAATGGGAAATGAGAACAT |
| | c.3485delA | CATTCAAGCAGAACTAGGTAGAAACAG |
| | c.3626delT | CTGTTAGATGATGGTGAAATAAAGGAAG |
| | c.3700_3704delGTAAA | CGAAGAGGGGCCAAGAAAT |
| | c.4035delA | GCTTCCAACACTTGTTATTTGGTAAA |
| | c.4097-2A>G | CAAGGAATTGGTTTCAGATGATGA |
| | c.4327C>T | TTTTACATCTGAACCTCTGTTTTTGTTATTTA |
| | c.470_471delCT | TTCTGCCCTTGAGGACCTG |
| | c.4964_4982del19 | AACCAGTCTCAGTGTCCAACTCT |
| | c.5123C>A | GGAGATACATATGGATACACTCACAAAT |
| | c.5251C>T | CGGACACTGAAATATTTTCTAGGAATTG |
| | c.5266dupC | AGAAACCACCAAGGTCCAAAG |
| | c.5277+1G>A | AAAGCGAGCAAGAGAATCCC |
| | c.5419delA | CAACTTGAGGGAGGGAGCTTTA |
| | c.66_66dupA | TGACTTACCAGATGGGACACTCT |
| | c.68_69delAG | CTGACTTACCAGATGGGACACT |
| | c.697_698delGT | CTTGTGAATTTTCTGAGACGGAT |
| | c.70_73dupTGTC | GCTGACTTACCAGATGGGACA |
| BRCA2 | c.2808_2811delACAA | GGTTTTATATGGAGACACAGGTGATAA |
| | c.3847_3848delGT | GTTTAAGATAGAAAATCATAATGATAAAACT |
| | c.5351dupA | CAGTATTGAAGAATGTTGAAGATCAAAAAAA |
| | c.5946delT | GTGGGATTTTTAGCACAGCAAG |
| | c.6275_6276delTT | TTTGATTTAATCAGAACTGAGCATAGTC |
| | c.6468_6469delTC | CACTCTATTAAAGTTTCTCCATATCTCTC |
| | c.7480C>T | CAGAATGCCAGAGATATACAGGATATG |
| | c.771_775delTCAAA | CTGTGACAGACAGTGAAAACACAAA |
| | c.8327T>G | GAAGCCCCAGAATCTCTTATGT |
| | c.8537_8538delAG | GGATTATACATATTTCGCAATGAAAGAG |
| | c.9026_9030delATCAT | TGTTAACAGAAGGAAAGAGATACAGAATTT |
| | c.9117+G> T | AAAAAAAACTCAGTATCAACAACTACCG |
| | c.9118-2A>G | CATATGTTGAATTTTTGTTTTGTTTTCTGT |
| | c.9403delC | GACCAGAATCCAAATCAGGC |

The following mutations and corresponding oligonucleotides (probes) were selected for the diagnostics of CFTR gene mutations - table 2:

**Table 2**

| mutation | SNaPshot probe |
|---|---|
| GLU7TER | AGAGACCATGCAGAGGTCGCCTCTG |
| TRP57TER | GTCCCACTTTTTATTCTTTTGCAGAGAATG |
| GLY85GLU | GATGTTTTTTCTGGAGATTTATGTTCTATG |
| GLY91ARG | GTTCTATGGAATCTTTTTATATTTA |
| GLU92LYS | TTTCTCTGTTTTTCCCCTTTTGTAG |
| GLU92TER | |
| TYR109CYS | CTCTCTTACTGGGAAGAATCATAGCTTCCT |
| ASP110HIS | ACTGGGAAGAATCATAGCTTCCTAT |
| ARG117HIS | TATGACCCGGATAACAAGGAGGAAC |
| LEU206TRP | GCACATTTCGTGTGGATCGCTCCTT |
| GLU217GLY | GCACTCCTCATGGGGCTAATCTGGG |
| PHE311LEU | TGTGAGATACTTCAATAGCTCAGCCTTCTT |
| ARG334TRP | CCCTATGCACTAATCAAAGGAATCATCCTC |
| THR338ILE | GGAATCATCCTCCGGAAAATATTCA |
| ARG347PRO | ACCATCTCATTCTGCATTGTTCTGC |
| ARG347LEU | |
| ARG347HIS | |
| ALA349VAL | TCATTCTGCATTGTTCTGCGCATGG |
| ARG352GLN | ATTGTTCTGCGCATGGCGGTCACTC |
| GLN359LYS | CGGCAATTTCCCTGGGCTGTACAAA |
| ALA455GLU | ACCTTGCCTGCTCCAGTGGATCCAGCAACC |
| GLY458VAL | TAGAAAGAGGACAGTTGTTGGCGGTTGCTG |
| MET470VAL | TTATTTCCAGACTTCACTTCTAATG |
| GLY480CYS | GTGATTATGGGAGAACTGGAGCCTTCAGAG |
| SER492PHE | TTAAGCACAGTGGAAGAATTTCATTCTGTT |
| GLN493TER | CAGTGGAAGAATTTCATTCTGTTCT |
| ILE506VAL | TATGCCTGGCACCATTAAAGAAAAT |
| ILE507DEL | ATTATGCCTGGCACCATTAAAGAAAATATC |
| PHE508DEL | TATGCCTGGCACCATTAAAGAAAATATCAT |
| PHE508CYS | GGCACCATTAAAGAAAATATCATCT |
| VAL520PHE | CTATGATGAATATAGATACAGAAGC |
| CYS524TER | ATATAGATACAGAAGCGTCATCAAAGCATG |
| ALA534GLU | GTGGAATCACACTGAGTGGAGGTCAACGAG |
| GLY542TER | TGCAGAGAAAGACAATATAGTTCTT |
| SER549ASN | CTTGGAGAAGGTGGAATCACACTGA |
| SER549ILE | |
| SER549ARG | TCTTGGAGAAGGTGGAATCACACTG |
| GLY551ASP | GAAGGTGGAATCACACTGAGTGGAG |
| GLY551SER | CTTGGAGAAGGTGGAATCACACTGAGTGGA |
| GLN552TER | GGAGAAGGTGGAATCACACTGAGTGGAGGT |
| ARG553TER | TGGAATCACACTGAGTGGAGGTCAA |
| ARG553GLN | AAGGTGGAATCACACTGAGTGGAGGTCAAC |
| ILE556VAL | ACTGAGTGGAGGTCAACGAGCAAGA |
| ALA559THR | AGGTCAACGAGCAAGAATTTCTTTA |
| ARG560THR | CAACGAGCAAGAATTTCTTTAGCAA |
| ARG560LYS | |
| ALA561GLU | TGTAATTTAATTTCCATTTTCTTTTTAGAG |
| TYR563ASN | TTTAATTTCCATTTTCTTTTTAGAGCAGTA |
| PRO574HIS | AAGATGCTGATTTGTATTTATTAGACTCTC |
| GLY576ALA | TTGTATTTATTAGACTCTCCTTTTG |
| ASP648VAL | CAGACTTTAGCTCAAAACTCATGGGATGTG |
| LYS710TER | TCTATTCTCAATCCAATCAACTCTATACGA |
| LYS716TER | AACTCTATACGAAAATTTTCCATTGTGCAA |
| GLU827TER | GGCTTGGAAATAAGTGAAGAAATTAACGAA |
| TRP846TER | TATGGAGAGCATACCAGCAGTGACTACATG |
| ARG851TER | AGTGACTACATGGAACACATACCTT |
| GLN890TER | ATTTCCTATTTGCTTTACAGCACTCCTCTT |
| SER912LEU | GCAGTGATTATCACCAGCACCAGTT |
| TYR913CYS | GTGATTATCACCAGCACCAGTTCGT |
| HIS949TYR | CATACTCTAATCACAGTGTCGAAAATTTTA |
| LEU1065PRO | TTGTTACAAGCTTAAAAGGACTATGGACAC |
| ARG1066HIS | TTACAAGCTTAAAAGGACTATGGACACTTC |
| ARG1066CYS | AAGCTTAAAAGGACTATGGACACTT |
| ALA1067THR | ACAAGCTTAAAAGGACTATGGACACTTCGT |
| GLN1071PRO | GACTATGGACACTTCGTGCCTTCGGACGGC |
| HIS1085ARG | AAACTCTGTTCCACAAAGCTCTGAATTTAC |
| TRP1089TER | GCTCTGAATTTACATACTGCCAACT |
| TYR1092TER | TACATACTGCCAACTGGTTCTTGTA |
| MET1101LYS | CTGTCAACACTGCGCTGGTTCCAAA |
| ARG1158TER | AATGTTGTTATTTTTATTTCAGATG |
| ARG1162TER | CTGTTGGCATGTCAATGAACTTAAAGACTC |
| TRP1204TER | CACACGTGAAGAAAGATGACATCTG |
| THR1220ILE | GTCAAAGATCTCACAGCAAAATACA |
| ILE1234VAL | CATATTAGAGAACATTTCCTTCTCA |
| GLN1238TER | CATTTCCTTCTCAATAAGTCCTGGC |
| GLY1244VAL | GCAGTGATTATCACCAGCACCAGTT |
| GLY1249GLU | AGGTGGGCCTCTTGGGAAGAACTGGATCAG |
| SER1251ASN | TTGGGAAGAACTGGATCAGGGAAGA |
| SER1255TER | GGATCAGGGAAGAGTACTTTGTTAT |
| SER1255PRO | AGAACTGGATCAGGGAAGAGTACTTTGTTA |
| ASP1270ASN | GAACACTGAAGGAGAAATCCAGATC |
| TRP1282TER | GGGA TTCAA TAACTTTGCAACAGTG |
| ARG1283MET | GATTCAATAACTTTGCAACAGTGGA |
| PHE1286SER | CAATAACTTTGCAACAGTGGAGGAAAGCCT |
| GLN1291HIS | GGAAAGCCTTTGGAGTGATACCACA |
| ASN1303LYS | TTTTTTCTGGAACATTTAGAAAAAA |
| ASN1303HIS | |
| GLN1313TER | GGATCCCTATGAACAGTGGAGTGAT |
| TRP1316TER | CCTATGAACAGTGGAGTGATCAAGAAATAT |
| GLN1352HIS | GTGTCCTAAGCCATGGCCACAAGCA |
| 2-BP DEL, 394TT | TTCTGGAGATTTATGTTCTATGGAATCTTT |
| 1-BP DEL, 556A | ATTGTGAGGACACTGCTCCTACACCCAGCC |
| 1-BP DEL, 557T | GAGGACACTGCTCCTACACCCAGCCATTTT |
| 2-BP DEL, 936TA | CTTGTGATTACCTCAGAAATGATTGAAAAT |
| 1-BP INS, 989A | GGAAGAAGCAATGGAAAAAATGATTGAAAA |
| 1-BP DEL, 1078T | TAGCTCAGCCTTCTTCTTCTCAGGGTTCTT |
| 2-BP INS, 1154TC | CATCCTCCGGAAAATATTCACCACCATCTC |
| 1-BP DEL, 1213T | ACTCGGCAATTTCCCTGGGCTGTACAAACA |
| 1-BP DEL, 1215G | TCGGCAATTTCCCTGGGCTGTACAAACATG |
| 2-BP DEL, NT1221 | CCCTGGGCTGTACAAACATGGTATGACTCT |
| 2-BP DEL, 1609CA | AAGCACAGTGGAAGAATTTCATTCTGTTCT |
| 2-BP DEL, 1677TA | TATCATCTTTGGTGTTTCCTATGATGAATA |
| 1-BP INS, 2307A | AAGACTCCCTTACAAATGAATGGCATCGAA |
| 1-BP DEL, 2423G | TGATCAGCACTGGCCCCACGCTTCAGGCAC |
| 2-BP INS, 2566AT | CTCAGGCAAACTTGACTGAACTGGATATAT |
| 1-BP INS, 2869G | ATGCAGTGATTATCACCAGCACCAGTTCGT |
| 5-BP DUP, NT3320 | TCATCTTGTTACAAGCTTAAAAGGACTATG |
| 1-BP DEL, 3293A | ATTTCACAGGCAGGAGTCCAATTTTCACTC |
| 1-BP INS, 3622T | TTTCAGATGCGATCTGTGAGCCGAGTCTTT |
| 1-BP DEL, 3659C | TGACTGTCAAAGATCTCACAGCAAAATACA |
| 4-BP INS, NT3667 | TGCCAACAGAAGGTAAACCTACCAAGTCAA |
| 1-BP DEL, 3876A | ATAGGTGGGCCTCTTGGGAAGAACTGGATC |
| 1-BP INS, 3898C | TGGATCAGGGAAGAGTACTTTGTTATCAGC |

DNA fragments containing the analyzed regions of BRCA1 and BRCA2 genes are amplified by PCR starters in 8 multiplex reactions (A-H) - table 3:

**Table 3**

| gene | exon | name | sequence | PCR reaction | Concentration nM |
|---|---|---|---|---|---|
| BRCA1 | 2 | 1020014 | GACGTTGTCATTAGTTCTTTGG | A | 600 |
| | | 1020029 | GGTCAATTCTGTTCATTTGC | | 600 |
| | 5 | 1050017 | CTCTTAAGGGCAGTTGTGAG | B | 500 |
| | | 1050023 | ATGGTTTTATAGGAACGCTATG | | 500 |
| | 8 | 1080012 | TGTTAGCTGACTGATGATGGT | C | 700 |
| | | 1080026 | AACCCAGCAATTATTATTAAATAC | | 700 |
| | 11 | 1110012 | GATTTCCACCTCCAAGGTGTATGA | A | 600 |
| | | 1110024 | CATGGCTCCACATGCAAG | | 600 |
| | | 1110031 | CAGCTGAGAGGCATCCA | B | 800 |
| | | 1110043 | GGATTCTCTGAGCATGGCA | | 800 |
| | | 1110052 | GATCCCCTGTGTGAGAGAA | C | 400 |
| | | 1110069 | TTACTCTCTACTGATTTGGAGTGA | | 400 |
| | | 1110073 | AGTGATCCTCATGAGGCTTT | D | 600 |
| | | 1110084 | AGGCCTGATGTAGGTCTCC | | 600 |
| | | 1110096 | TACAAGAGCGTCCCCTCAC | E | 600 |
| | | 1110101 | TTTTCGAGTGATTCTATTGG | | 600 |
| | | 1110111 | GAGCAGAATGGTCAAGTGAT | F | 600 |
| | | 1110124 | CAAGCGCATGAATATGCCT | | 600 |
| | | 1110132 | TGTCAATCCTAGCCTTCCA | G | 500 |
| | | 1110142 | AAAGCCTTCTGTGTCATTTCT | | 500 |
| | | 1110151 | CCAAGGGACTAATTCATGGT | H | 700 |
| | | 1110162 | TTAACTGTCTGTACAGGCTTGAT | | 700 |
| | | 1110172 | GAAACAAAGTCCAAAAGTCACT | A | 400 |
| | | 1110181 | ACTTGATGGGAAAAAGTGG | | 400 |
| | | 1110191 | CATTACTCCAAATAAACATGGACT | B | 500 |
| | | 1110201 | TACTG GAG CCCACTTCATT | | 500 |
| | | 1110212 | TGGGAAATGAGAACATTCC | C | 500 |
| | | 1110221 | CAGGAAGACTTTGTTTATAGACC | | 500 |
| | | 1110231 | GCAGAACTAGGTAGAAACAGAGG | D | 500 |
| | | 1110242 | TTT CCTT AA TGTCA TTTTCAGCA | | 500 |
| | | 1110251 | TTAGATGATGGTGAAATAAAGGA | E | 600 |
| | | 1110261 | CAATGATAATAAATTCTCCTCTGT | | 600 |
| | | 1110271 | TAGCACCGTTGCTACCGAGT | F | 600 |
| | | 1110281 | TTTCTTCCAAGCCCGTT | | 600 |
| | | 1110292 | AGCCAGGGAGTTGGTCTG | G | 800 |
| | | 1110301 | CCCAAAAGCATAAACATTTAGC | | 800 |
| | 12 | 1120012 | GCGTTTATAGTCTGCTTTTACA | H | 600 |
| | | 1120021 | TGTCAGCAAACCTAAGAATGT | | 600 |
| | 13 | 1130011 | AATGGAAAGCTTCTCAAAGTA | D | 600 |
| | | 1130021 | ATGTTGGAGCTAGGTCCTTAC | | 600 |
| | 16 | 1160013 | AATTCTTAACAGAGACCAGAAC | E | 500 |
| | | 1160024 | AAAACTCTTTCCAGAATGTTGT | | 500 |
| | 18 | 1180015 | GGCTCTTTAGCTTCTTAGGAC | F | 500 |
| | | 1180026 | CTCAGACTCAAGCATCAGC | | 500 |
| | 20 | 1200011 | ATATGACGTGTCTGCTCCAC | G | 500 |
| | | 1200029 | AGTCTTACAAAATGAAGCGG | | 500 |
| | 23 | 1230014 | CAGAGCAAGACCCTGTCTC | H | 500 |
| | | 1230021 | GACATTTTAGCCATTCA | | 500 |
| BRCA2 | 9 | 2090017 | ATAACTGAAATCACCAAAAGTG | A | 600 |
| | | 2090021 | CTGTAGTTCAACTAAACAGAGG | | 600 |
| | 11 | 2110011 | AACCCATTTTCAAGAACTCTACCA | A | 500 |
| | | 2110023 | CTGAAGCTACCTCCAAAACTGTG | | 500 |
| | | 2110031 | CTTCAAGTAAATGTCATGATTCTGTC | B | 500 |
| | | 2110042 | CTGGCAGCAGTATATTTGTTATCT | | 400 |
| | | 2110051 | AACCAGAAAGAATAAATACT | C | 600 |
| | | 2110061 | TCCTCAACGCAAATATCTTCAT | | 600 |
| | | 2110071 | CACCTTGTGATGTTAGTTT | D | 600 |
| | | 2110081 | TTGGGATATTAAATGTTCTGGAGTA | | 600 |
| | | 2110091 | AAAGTAACGAACATTCAGACCA | E | 600 |
| | | 2110101 | CTGGGTTTCTCTTATCAACACG | | 600 |
| | | 2110111 | AGTCTTCACTATTCACCTACG | F | 600 |
| | | 2110121 | GTGAGACTTTGGTTCCTAAT | | 600 |
| | | 2110131 | TTCAACAAGACAAACAACAGT | G | 600 |
| | | 2110141 | TGTCAGTTCATCATCTTCCATAAA | | 600 |
| | 15 | 2150011 | GGCCAGGGGTTGTGCTTTTT | B | 500 |
| | | 2150021 | AGGATACTAGTTAATGAAATA | | 500 |
| | 18 | 2180011 | GCAGATACCCAAAAAGTGGC | C | 800 |
| | | 2180021 | TCTGGACCTCCCAAAAACTG | | 800 |
| | 20 | 2200011 | CACTGTGCCTGGCCTGATAC | D | 600 |
| | | 2200021 | ATGTTAAATTCAAAGTCTCTA | | 600 |
| | 23 | 2230011 | ACTTCTTCCATTGCATCTTTCTCA | E | 600 |
| | | 2230021 | AAAACAAAACAAAAATTCAACATA | | 600 |
| | 24 | 2240011 | GCAGCGACAAAAAAAACTCA | F | 600 |
| | | 2240021 | ATTTGCCAACTGGTAGCTCC | | 600 |
| | 25 | 2250011 | GCTTTCGCCAAATTCAGCTA | G | 600 |
| | | 2250021 | TACCAAAATGTGTGGTGATGC | | 600 |

Reactions are performed under the following conditions: PCR reaction volume: 50µl, 1x PCR buffer, MgCl₂ 2.5 mM, dNTPs 250 µM, BSA fraction V 0.16 mg/ml, Glycerol (10% w/v), 5U TaqPol, starters see table 3, and 10 µl DNA(1 ng/µl), in multiplex reactions of table 3. Reaction temperature profile: initial denaturation 95°C - 3 min., then 35 cycles: denaturation 94°C - 1 min., annealing 55°C - 1 min., elongation 72°C - 1 min., final elongation 60°C - 15 min.

DNA fragments containing the analyzed regions of CFTR gene amplified with PCR starters in 4 multiplex reactions (A-D) - table 4:

**Table 4**

| gene | exon | name | sequence | PCR reaction | concentration nM |
|---|---|---|---|---|---|
| CFTR | 1 | 3010011 | CAGCACTCGGCTTTTAAC | A | 700 |
| | | 3010021 | ATACACACGCCCTCCTCT | | 700 |
| | 2 | 3020011 | TCCAAATCTGTATGGAGACC | B | 600 |
| | | 3020021 | TGAATTTCTCTCTTCAACTAAACA | | 600 |
| | 3 | 3030011 | CAACTTATTGGTCCCACTTT | C | 800 |
| | | 3030021 | CACCTATTCACCAGATTTCG | | 800 |
| | 4 | 3040011 | TTGTAGGAAGTCACCAAAGC | D | 400 |
| | | 3040021 | TACGATACAGAATATATGTGCCA | | 400 |
| | 5 | 3050011 | TTGAAATTATCTAACTTTCCATTTT | A | 600 |
| | | 3050021 | CGCCTTTCCAGTTGTATAAT | | 600 |
| | 6 | 3060011 | GCTGTGCTTTTATTTTCCAG | B | 600 |
| | | 3060021 | ACTAAAGTGGGCTTTTTGAA | | 600 |
| | | 3060031 | CTTAAAACCTTGAGCAGTTCT | C | 600 |
| | | 3060041 | CAATATTGAAATTATTGGAACAAC | | 600 |
| | 7 | 3070011 | AGATCTTCCATTCCAAGATC | D | 500 |
| | | 3070021 | TGCAGCATTATGGTACATTA | | 500 |
| | 8 | 3080011 | AAGATGTAGCACAATGAGAGTATAAA | A | 700 |
| | | 3080021 | GAAAACAGTTAGGTGTTTAGAGCAA | | 700 |
| | 9 | 3090011 | TGGGGAATTATTTGAGAAAG | B | 400 |
| | | 3090021 | CTTCCAGCACTACAAACTAGAAA | | 400 |
| | 10 | 3010011 | GCGTGATTTGATAATGACCT | C | 500 |
| | | 3010021 | TGGGTAGTGTGAAGGGTTC | | 500 |
| | 11 | 3011011 | AGATTGAGCATACTAAAAGTGAC | D | 500 |
| | | 3011021 | TGCTTGCTAGACCAATAATTAG | | 500 |
| | 12 | 3012011 | CCAGGAAATAGAGAGGAAATG | A | 500 |
| | | 3012021 | CATACCAACAATGGTGAACA | | 500 |
| | 13 | 3013011 | GCTAAAATACGAGACATATTGC | B | 600 |
| | | 3013021 | ATTCTGTGGGGTGAAATACC | | 600 |
| | 14 | 3014011 | GGTGGCATGAAACTGTACT | C | 600 |
| | | 3014021 | ATACATCCCCAAACTATCTTAAT | | 600 |
| | | 3014031 | ATGGGAGGAATAGGTGAAGA | D | 800 |
| | | 3014041 | CAAAGTGGATTACAATACATACA | | 800 |
| | 15 | 3015011 | TGCCAAATAACGATTTCCTA | A | 600 |
| | | 3015021 | GTGGATCAGCAGTTTCATTT | | 600 |
| | 16 | 3016011 | TTGAGGAATTTGTCATCTTGT | B | 600 |
| | | 3016021 | GACTTCAACCCTCAATCAAA | | 600 |
| | 17 | 3017011 | CACTGACACACTTTGTCCAC | C | 500 |
| | | 3017021 | ATCGCACATTCACTGTCATA | | 500 |
| | | 3017031 | ATTTGCAATGTTTTCTATGG | D | 500 |
| | | 3017041 | TGCTTAGCTAAAGTTAATGAGTTC | 500 | 500 |
| | 18 | 3018011 | TTCATTTACGTCTTTTGTGC | A | 600 |
| | | 3018021 | GGTATATAGTTCTTCCTCATGC | | 600 |
| | 19 | 3019011 | GTGAAATTGTCTGCCATTCT | B | 800 |
| | | 3019021 | CAAGCAGTGTTCAAATCTCA | | 800 |
| | 20 | 3020011 | TGATCCCATCACTTTTACCT | C | 600 |
| | | 3020021 | TTTCTGGCTAAGTCCTTTTG | | 600 |
| | 21 | 3021011 | AGAACTTGATGGTAAGTACATG | D | 600 |
| | | 3021021 | CATTTCAGTTAGCAGCCTTA | | 600 |
| | 22 | 3022011 | TCTGAACTATCTTCTCTAACTGC | A | 500 |
| | | 3022021 | AATGATTCTGTTCCCACTGT | | 500 |
| | 23 | 3023011 | TTCTGTGATATTATGTGTGGTATT | B | 500 |
| | | 3023021 | AAGAATTACAAGGGCAATGA | | 500 |
| | 24 | 3024011 | CAGATCTCACTAACAGCCATT | C | 500 |
| | | 3024021 | TGTCAACATTTATGCTGCTC | | 500 |

Reactions are carried out under the following conditions: PCR reaction volume: 50 µl, 1x PCR buffer, MgCl₂ 2,5 mM, dNTPs 250 µM, BSA fraction V 0,16 mg/ml, glycerol (10% w/v), 5U TaqPol, starters cf. table 4, and 10 µl DNA (1 ng/µl), in multiplex reactions of table 4. Reactions temperature profile: initial denaturation 95°C - 3 min., followed by 35 cycles: denaturation 94°C - 1 min., annealing 55°C - 1 min., elongation 72°C - 1 min., final elongation 60°C - 15 min.

DNA for examination can be obtained according to many available protocols, according to user's preferences.

Each analysis involves amplification of 4 samples:: a positive control containing DNA of known genotype, a negative control containing water instead of DNA solution, and two examined samples, which fulfills sample double analysis criteria for a reliable result.

After the reaction is finished, reaction products of the individual multiplex reactions for the same sample are mixed together: in the case of mutation analysis in BRCA1 and BRCA2 genes, products of 8 multiplex PCR reactions are mixed together for each sample, in the case of CFTR gene, products of 4 multiplex PCR reactions are mixed together.

The mixed amplification products are subjected to purification from starters, ions, nucleotides and polymerase by a method which guarantees yield of at least 20 µl of purified product.

Reaction on the matrix is prepared in 4 test tubes (negative control, positive control, examined sample 1, examined sample 2) in a volume of 100 µl, said volume containing: 50 mM Tris-HCl, ph 9,5, 3 mM MgCl₂, 0,75 µm 7-Propargylamino-7-deaza-2',3'-dideoxyadenosine-5'thiphosphate-6-FAM, 5-Propargylamino-2',3'-dideoxycytidine+5'thiphosphate-5/6-TAMRA, 7-Propargylamino-7-deaza-2'3'dideoxyguanosine-5'triphosphate-6-ROX, 5-Propargylamino-2',3'-dideoxyuridine-5'triphosphate-6-JOE, 5U thermostable DNA polymerase adding dideoxynucleotides, and 20 µl of purified PCR product. Reaction mixtures are placed in proper matrix fields and sealed with foil. The matrix is placed in a thermocycler for amplification on a microscope slide and amplification is run under the following conditions: 35 cycles: denaturation 94°C - 1 min., annealing/elongation 50°C - 1 min., cover temperature 50°C.

After finished amplification, the sealing foil is removed and the matrix is placed in a wash station. The matrices are washed 2x 2 min. in deionized water and then dried by centrifugation in a minicentrifuge for matrix centrifugation.

The matrix is analyzed on a multicolor scanner. 4 scans are conducted under the following conditions:
- for 7-Propargylamino-7-deaza-2',3'- dideoxyadenosine-5'thiphosphate-6-FAM excitation wavelength: 492 nm, emission wavelength: 517 nm
- for 5-Propargylamino-2',3'-dideoxycytidine+5'thiphosphate-5/6-TAMRA excitation wavelength: 545 nm, emission wavelength: 575 nm
- for 7-Propargylamino-7-deaza-2'3'dideoxyguanosine-5'triphosphate-6-ROX excitation wavelength: 575 nm, emission wavelength: 600 nm
- for 5-Propargylamino-2',3'-dideoxyuridine-5'triphosphate-6-JOE excitation wavelength: 520 nm, emission wavelength: 548 nm

### CFTR gene mutations analysis

### 1. Matrix preparation

Oligonucleotides for matrix preparation were purchased from GENOMED S.A. Synthesis was carried out in 0.2 µmol scale, HPLC purified. Sequences of the individual probes are shown in table 5. Oligonucleotides were dissolved in water in an appropriate water volume for obtaining 200 µM concentration.

**Table 5. Nucleotide sequence of matrix probes for the detection of CFTR gene mutations.**

| mutation | probe |
|---|---|
| GLU7TER | 5'-C12-AMINO-(dT)₁₈-AGAGACCATGCAGAGGTCGCCTCTG-3' |
| TRP57TER | 5'-C12-AMINO-(dT)₁₈-GTCCCACTTTTTATTCTTTTGCAGAGAATG-3' |
| GLY85GLU | 5'-C12-AMINO-(dT)₁₈-GATGTTTTTTCTGGAGATTTATGTTCTATG-3' |
| GLY91ARG | 5'-C12-AMINO-(dT)₁₈-GTTCTATGGAATCTTTTTATATTTA-3' |
| GLU92LYS | 5'-C12-AMINO-(dT)₁₈-TTTCTCTGTTTTTCCCCTTTTGTAG-3' |
| GLU92TER | |
| TYR109CYS | 5'-C12-AMINO-(dT)₁₈-CTCTCTTACTGGGAAGAATCATAGCTTCCT-3' |
| ASP110HIS | 5'-C12-AMINO-(dT)₁₈-ACTGGGAAGAATCATAGCTTCCTAT-3' |
| ARG117HIS | 5'-C12-AMINO-(dT)₁₈-TATGAGCCGGATAACAAGGAGGAAC-3' |
| LEU206TRP | 5'-C12-AMINO-(dT)₁₈-GCACATTTCGTGTGGATCGCTCCTT-3' |
| GLU217GLY | 5'-C12-AMINO-(dT)₁₈-GCACTCCTCATGGGGCTAATCTGGG-3' |
| PHE311LEU | 5'-C12-AMINO-(dT)₁₈-TGTGAGATACTTCAATAGCTCAGCCTTCTT-3' |
| ARG334TRP | 5'-C12-AMINO-(dT)₁₈-CCCTATGCACTAATCAAAGGAATCATCCTC-3' |
| THR338ILE | 5'-C12-AMINO-(dT)₁₈-GGAATCATCCTCCGGAAAATATTCA-3' |
| ARG347PRO | 5'-C12-AMINO-(dT)₁₈-ACCATCTCATTCTGCATTGTTCTGC-3' |
| ARG347LEU | |
| ARG347HIS | |
| ALA349VAL | 5'-C12-AMINO-(dT)₁₈-TCATTCTGCATTGTTCTGCGCATGG-3' |
| ARG352GLN | 5'-C12-AMINO-(dT)₁₈-ATTGTTCTGCGCATGGCGGTCACTC-3' |
| GLN359LYS | 5'-C12-AMINO-(dT)₁₈-CGGCAATTTCCCTGGGCTGTACAAA-3' |
| ALA455GLU | 5'-C12-AMINO-(dT)₁₈-ACCTTGCCTGCTCCAGTGGATCCAGCAACC-3' |
| GLY458VAL | 5'-C12-AMINO-(dT)₁₈-TAGAAAGAGGACAGTTGTTGGCGGTTGCTG-3' |
| MET470VAL | 5'-C12-AMINO-(dT)₁₈-TTATTTCCAGACTTCACTTCTAATG-3' |
| GLY480CYS | 5'-C12-AMINO-(dT)₁₈-GTGATTATGGGAGAACTGGAGCCTTCAGAG-3' |
| SER492PHE | 5'-C12-AMINO-(dT)₁₈-TTAAGCACAGTGGAAGAATTTCATTCTGTT-3' |
| GLN493TER | 5'-C12-AMINO-(dT)₁₈-CAGTGGAAGAATTTCATTCTGTTCT-3' |
| ILE506VAL | 5'-C12-AMINO-(dT)₁₈-TATGCCTGGCACCATTAAAGAAAAT-3' |
| ILE507DEL | 5'-C12-AMINO-(dT)₁₈-ATTATGCCTGGCACCATTAAAGAAAATATC-3' |
| PHE508DEL | 5'-C12-AMINO-(dT)₁₈-TATGCCTGGCACCATTAAAGAAAATATCAT-3' |
| PHE508CYS | 5'-C12-AMINO-(dT)₁₈-GGCACCATTAAAGAAAATATCATCT-3' |
| VAL520PHE | 5'-C12-AMINO-(dT)₁₈-CTATGATGAATATAGATACAGAAGC-3' |
| CYS524TER | 5'-C12-AMINO-(dT)₁₈-ATATAGATACAGAAGCGTCATCAAAGCATG-3' |
| ALA534GLU | 5'-C12-AMINO-(dT)₁₈-GTGGAATCACACTGAGTGGAGGTCAACGAG-3' |
| GLY542TER | 5'-C12-AMINO-(dT)₁₈-TGCAGAGAAAGACAATATAGTTCTT-3' |
| SER549ASN | 5'-C12-AMINO-(dT)₁₈-CTTGGAGAAGGTGGAATCACACTGA-3' |
| SER549ILE | |
| SER549ARG | 5'-C12-AMINO-(dT)₁₈-TCTTGGAGAAGGTGGAATCACACTG-3' |
| GLY551ASP | 5'-C12-AMINO-(dT)₁₈-GAAGGTGGAATCACACTGAGTGGAG-3' |
| GLY551SER | 5'-C12-AMINO-(dT)₁₈-CTTGGAGAAGGTGGAATCACACTGAGTGGA-3' |
| GLN552TER | 5'-C12-AMINO-(dT)₁₈-GGAGAAGGTGGAATCACACTGAGTGGAGGT-3' |
| ARG553TER | 5'-C12-AMINO-(dT)₁₈-TGGAATCACACTGAGTGGAGGTCAA-3' |
| ARG553GLN | 5'-C12-AMINO-(dT)₁₈-AAGGTGGAATCACACTGAGTGGAGGTCAAC-3' |
| ILE556VAL | 5'-C12-AMINO-(dT)₁₈-ACTGAGTGGAGGTCAACGAGCAAGA-3' |
| ALA559THR | 5'-C12-AMINO-(dT)₁₈-AGGTCAACGAGCAAGAATTTCTTTA-3' |
| ARG560THR | 5'-C12-AMINO-(dT)₁₈-CAACGAGCAAGAATTTCTTTAGCAA-3' |
| ARG560LYS | |
| ALA561GLU | 5'-C12-AMINO-(dT)₁₈-TGTAATTTAATTTCCATTTTCTTTTTAGAG-3' |
| TYR563ASN | 5'-C12-AMINO-(dT)₁₈-TTTAATTTCCATTTTCTTTTTAGAGCAGTA-3' |
| PRO574HIS | 5'-C12-AMINO-(dT)₁₈-AAGATGCTGATTTGTATTTATTAGACTCTC-3' |
| GLY576ALA | 5'-C12-AMINO-(dT)₁₈-TTGTATTTATTAGACTCTCCTTTTG-3' |
| ASP648VAL | 5'-C12-AMINO-(dT)₁₈-CAGACTTTAGCTCAAAACTCATGGGATGTG-3' |
| LYS710TER | 5'-C12-AMINO-(dT)₁₈-TCTATTCTCAATCCAATCAACTCTATACGA-3' |
| LYS716TER | 5'-C12-AMINO-(dT)₁₈-AACTCTATACGAAAATTTTCCATTGTGCAA-3' |
| GLU827TER | 5'-C12-AMINO-(dT)₁₈-GGCTTGGAAATAAGTGAAGAAATTAACGAA-3' |
| TRP846TER | 5'-C12-AMINO-(dT)₁₈-TATGGAGAGCATACCAGCAGTGACTACATG-3' |
| ARG851TER | 5'-C12-AMINO-(dT)₁₈-AGTGACTACATGGAACACATACCTT-3' |
| GLN890TER | 5'-C12-AMINO-(dT)₁₈-ATTTCCTATTTGCTTTACAGCACTCCTCTT-3' |
| SER912LEU | 5'-C12-AMINO-(dT)₁₈-GCAGTGATTATCACCAGCACCAGTT-3' |
| TYR913CYS | 5'-C12-AMINO-(dT)₁₈-GTGATTATCACCAGCACCAGTTCGT-3' |
| HIS949TYR | 5'-C12-AMINO-(dT)₁₈-CATACTCTAATCACAGTGTCGAAAATTTTA-3' |
| LEU1065PRO | 5'-C12-AMINO-(dT)₁₈-TTGTTACAAGCTTAAAAGGACTATGGACAC-3' |
| ARG1066HIS | 5'-C12-AMINO-(dT)₁₈-TTACAAGCTTAAAAGGACTATGGACACTTC-3' |
| ARG1066CYS | 5'-C12-AMINO-(dT)₁₈-AAGCTTAAAAGGACTATGGACACTT-3' |
| ALA1067THR | 5'-C12-AMINO-(dT)₁₈-ACAAGCTTAAAAGGACTATGGACACTTCGT-3' |
| GLN1071PRO | 5'-C12-AMINO-(dT)₁₈-GACTATGGACACTTCGTGCCTTCGGACGGC-3' |
| HIS1085ARG | 5'-C12-AMINO-(dT)₁₈-AAACTCTGTTCCACAAAGCTCTGAATTTAC-3' |
| TRP1089TER | 5'-C12-AMINO-(dT)₁₈-GCTCTGAATTTACATACTGCCAACT-3' |
| TYR1092TER | 5'-C12-AMINO-(dT)₁₈-TACATACTGCCAACTGGTTCTTGTA-3' |
| MET1101LYS | 5'-C12-AMINO-(dT)₁₈-CTGTCAACACTGCGCTGGTTCCAAA-3' |
| ARG1158TER | 5'-C12-AMINO-(dT)₁₈-AATGTTGTTATTTTTATTTCAGATG-3' |
| ARG1162TER | 5'-C12-AMINO-(dT)₁₈-CTGTTGGCATGTCAATGAACTTAAAGACTC-3' |
| TRP1204TER | 5'-C12-AMINO-(dT)₁₈-CACACGTGAAGAAAGATGACATCTG-3' |
| THR1220ILE | 5'-C12-AMINO-(dT)₁₈-GTCAAAGATCTCACAGCAAAATACA-3' |
| ILE1234VAL | 5'-C12-AMINO-(dT)₁₈-CATATTAGAGAACATTTCCTTCTCA-3' |
| GLN1238TER | 5'-C12-AMINO-(dT)₁₈-CATTTCCTTCTCAATAAGTCCTGGC-3' |
| GLY1244VAL | 5'-C12-AMINO-(dT)₁₈-GCAGTGATTATCACCAGCACCAGTT-3' |
| GLY1249GLU | 5'-C12-AMINO-(dT)₁₈-AGGTGGGCCTCTTGGGAAGAACTGGATCAG-3' |
| SER1251ASN | 5'-C12-AMINO-(dT)₁₈-TTGGGAAGAACTGGATCAGGGAAGA-3' |
| SER1255TER | 5'-C12-AMINO-(dT)₁₈-GGATCAGGGAAGAGTACTTTGTTAT-3' |
| SER1255PRO | 5'-C12-AMINO-(dT)₁₈-AGAACTGGATCAGGGAAGAGTACTTTGTTA-3' |
| ASP1270ASN | 5'-C12-AMINO-(dT)₁₈-GAACACTGAAGGAGAAATCCAGATC-3' |
| TRP1282TER | 5'-C12-AMINO-(dT)₁₈-GGGATTCAATAACTTTGCAACAGTG-3' |
| ARG1283MET | 5'-C12-AMINO-(dT)₁₈-GATTCAATAACTTTGCAACAGTGGA-3' |
| PHE1286SER | 5'-C12-AMINO-(dT)₁₈-CAATAACTTTGCAACAGTGGAGGAAAGCCT-3' |
| GLN1291HIS | 5'-C12-AMINO-(dT)₁₈-GGAAAGCCTTTGGAGTGATACCACA-3' |
| ASN1303LYS | 5'-C12-AMINO-(dT)₁₈-TTTTTTCTGGAACATTTAGAAAAAA-3' |
| ASN1303HIS | |
| GLN1313TER | 5'-C12-AMINO-(dT)₁₈-GGATCCCTATGAACAGTGGAGTGAT-3' |
| TRP1316TER | 5'-C12-AMINO-(dT)₁₈-CCTATGAACAGTGGAGTGATCAAGAAATAT-3' |
| GLN1352HIS | 5'-C12-AMINO-(dT)₁₈-GTGTCCTAAGCCATGGCCACAAGCA-3' |
| 2-BP DEL, 394TT | 5'-C12-AMINO-(dT)₁₈-TTCTGGAGATTTATGTTCTATGGAATCTTT-3' |
| 1-BP DEL, 556A | 5'-C12-AMINO-(dT)₁₈-ATTGTGAGGACACTGCTCCTACACCCAGCC-3' |
| 1-BP DEL, 557T | 5'-C12-AMINO-(dT)₁₈-GAGGACACTGCTCCTACACCCAGCCATTTT-3' |
| 2-BP DEL, 936TA | 5'-C12-AMINO-(dT)₁₈-CTTGTGATTACCTCAGAAATGATTGAAAAT-3' |
| 1-BP INS, 989A | 5'-C12-AMINO-(dT)₁₈-GGAAGAAGCAATGGAAAAAATGATTGAAAA-3' |
| 1-BP DEL, 1078T | 5'-C12-AMINO-(dT)₁₈-TAGCTCAGCCTTCTTCTTCTCAGGGTTCTT-3' |
| 2-BP INS, 1154TC | 5'-C12-AMINO-(dT)₁₈-CATCCTCCGGAAAATATTCACCACCATCTC-3' |
| 1-BP DEL, 1213T | 5'-C12-AMINO-(dT)₁₈-ACTCGGCAATTTCCCTGGGCTGTACAAACA-3' |
| 1-BP DEL, 1215G | 5'-C12-AMINO-(dT)₁₈-TCGGCAATTTCCCTGGGCTGTACAAACATG-3' |
| 2-BP DEL,NT1221 | 5'-C12-AMINO-(dT)₁₈-CCCTGGGCTGTACAAACATGGTATGACTCT-3' |
| 2-BP DEL,1609CA | 5'-C12-AMINO-(dT)₁₈-AAGCACAGTGGAAGAATTTCATTCTGTTCT-3' |
| 2-BP DEL, 1677TA | 5'-C12-AMINO-(dT)₁₈-TATCATCTTTGGTGTTTCCTATGATGAATA-3' |
| 1-BP INS, 2307A | 5'-C12-AMINO-(dT)₁₈-AAGACTCCCTTACAAATGAATGGCATCGAA-3' |
| 1-BP DEL, 2423G | 5'-C12-AMINO-(dT)₁₈-TGATCAGCACTGGCCCCACGCTTCAGGCAC-3' |
| 2-BP INS, 2566AT | 5'-C12-AMINO-(dT)₁₈-CTCAGGCAAACTTGACTGAACTGGATATAT-3' |
| 1-BP INS, 2869G | 5'-C12-AMINO-(dT)₁₈-ATGCAGTGATTATCACCAGCACCAGTTCGT-3' |
| 5-BP DUP,NT3320 | 5'-C12-AMINO-(dT)₁₈-TCATCTTGTTACAAGCTTAAAAGGACTATG-3' |
| 1-BP DEL, 3293A | 5'-C12-AMINO-(dT)₁₈-ATTTCACAGGCAGGAGTCCAATTTTCACTC-3' |
| 1-BP INS, 3622T | 5'-C12-AMINO-(dT)₁₈-TTTCAGATGCGATCTGTGAGCCGAGTCTTT-3' |
| 1-BP DEL, 3659C | 5'-C12-AMINO-(dT)₁₈-TGACTGTCAAAGATCTCACAGCAAAATACA-3' |
| 4-BP INS, NT3667 | 5'-C12-AMINO-(dT)₁₈-TGCCAACAGAAGGTAAACCTACCAAGTCAA-3' |
| 1-BP DEL, 3876A | 5'-C12-AMINO-(dT)₁₈-ATAGGTGGGCCTCTTGGGAAGAACTGGATC-3' |
| 1-BP INS, 3898C | 5'-C12-AMINO-(dT)₁₈-TGGATCAGGGAAGAGTACTTTGTTATCAGC-3' |
| Spotting control | 5'-C12-AMINO-(dT)₁₈-ACG TAC GTA CGT ACG TAC GTA CGT-Cy5-3' |

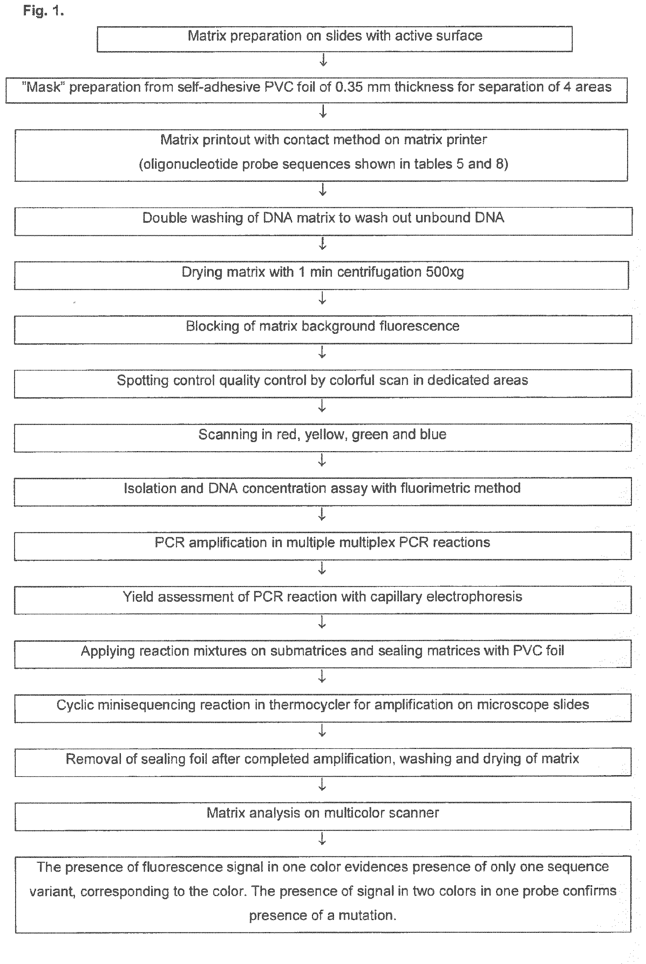

Matrices were prepared on slides with SUPEREPOXY surface. Before taking out of the box, the case and the box containing slides were cleaned with Microarray Cleanroom Wipes and a 95% ethanol solution and with air from a Microarray Air Jet compressor. Slides were unpacked in a class II laminar air flow chamber and a "mask" with dimensions of 76 x 26 mm, cut out from self-adhesive PVC foil of 0.35 mm thickness with a laser plotter, was applied to the active surface. In every fragment of the foil a circle of 14 mm diameter was cut out, 2,5 mm from the edge of the slide and at regular distances of 4 mm (from the edges of circles). Thereby 4 submatrices of 50 µl volume, resistant to the solutions used in the procedure and to temperature changes in the range used in the procedure, were formed on the slide surface - a substrate of the future matrix. Individual submatrices are dedicated to analysis of a positive control, a negative control and two examined samples, wherein analysis of two solutions of the same sample in separate submatrices of one matrix is assumed, so that the analysis result is always confirmed in two tests. The matrix is shown in fig. 1. Slides were placed on a SpotBot 2 Array Printer (Arraylt) working top.

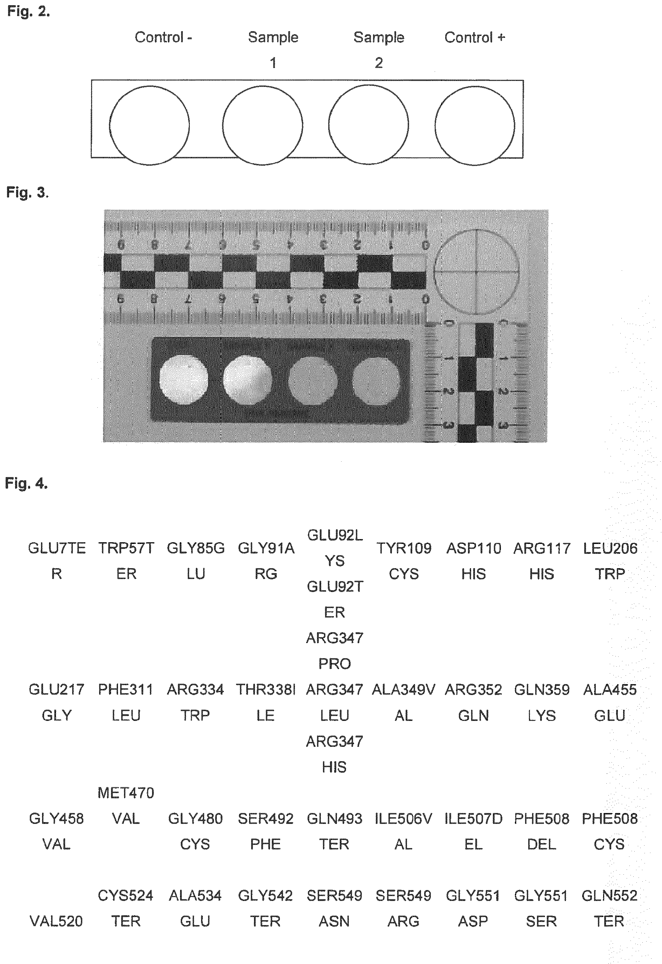

100 µM oligonucleotide solutions in buffer were prepared in a 96-well plate by adding 25 µl of 200 µM oligonucleotide solution and 25 µl of 2x Micro Spotting Solution Plus (Arraylt) solution. Oligonucleotide solutions were mixed by 10x pipetting with 40 µl volume and the plate was centrifuged in a centrifuge for plate centrifugation (2 min., 4000 rpm). Matrices were printed with contact method. Each submatrix was printed in the same probe layout. Said probe layout is shown in fig. 2. The printed matrices were placed in a climatic chamber with relative humidity <30%, at room temperature (∼25°C) for 12 hours, after which the matrices were incubated 1 hour in 80°C in a laboratory incubator. To wash out unbound DNA, matrices were washed twice for 2 min. at room temperature (22-25°C) in 500 ml of 0,1% SDS solution and once in 500 ml of deionized water with intense mixing in a High Throughput Wash Station. Matrices were dried by 1 min. centrifugation, 500xg in a Microarray High Speed Centrifuge. To block background fluorescence the matrices were incubated for 60 min. at 60°C in 500 ml Blocklt Buffer and dried by 1 min. centrifugation, 500xg in a Microarray High Speed Centrifuge.

### 2. Examined material

The examined material was a dried blood specimen on a FTA card for proficiency testing as part of "Newborn Screening Quality Assurance Program", Cystic Fibrosis DNA Mutation Detection Proficiency Testing (CFDNAPT), PT Quarter 3 - 2016, denoted as: 316C1 and 316C2. Blood dried on a FTA card denoted as EXDNAPC was used as positive control, which was verified in the course of a test with DNA sequencing method to contain no CFTR gene mutations. Two fragments were cut out from the FTA card containing the blood specimen with a chisel of 3 mm diameter to two test tubes, and denoted as "Control +".

### 3. DNA isolation

DNA was isolated on a QIAsymphony (QIAGEN) automatic workstation using a QIAsymphony DNA Investigator Kit (Qiagen) reagent kit, according to the manufacturer instructions. Isolation was performed in presence of the negative control, containing all reagent components without the addition of biological material, denoted containing no DNA, denoted "Control -".

### 4. DNA concentration assesment

DNA concentration was assayed with fluorymetric method with a Qubit 2.0 (Life Technologies) apparatus, using a Qubit ®dsDNA BR (Life Technologies) kit, according to the manufacturer instructions.

Water solutions of working concentration 1 ng/ml were prepared from the isolated DNA based on the results of DNA concentration measurement. Sample "Control -" is not subject to analysis or dilution.

### 5. PCR amplification

PCR amplification was carried out in 4 multiplex PCR reactions in 50 µl volume in PCR tubes of 200 µl volume, said tubes denoted as A, B, C, D, containing: 1x PCR buffer, MgCl₂ 2,5 mM, dNTPs 250 µM (A&A Biotechnology), BSA fraction V 0,16 mg/ml (Sigma), glycerol (10% w/v), 5U TaqPol (A&A Biotechnology), starters as in table 6, and 10 µl DNA, in multiplex reactions of table 6; PCR reaction temperature profile: initial denaturation 95°C - 3 min., followed by 35 cycles: denaturation 94°C - 1 min., annealing 55°C - 1 min., elongation 72°C - 1 min., final elongation 60°C - 15 min. Two examined samples, negative control and positive control underwent amplification. The amplification was carried out in a GeneAmp PCR System 9700 thermocycler.

**Table 6. Starters for multiplex amplification of CFTR gene**

| gene | exon | sequence | PCR reaction | concentration nM |
|---|---|---|---|---|
| CFTR | 1 | 5'-CAGCACTCGGCTTTTAAC-3' | A | 700 |
| | | 5'-ATACACACGCCCTCCTCT-3' | | 700 |
| | 2 | 5'-TCCAAATCTGTATGGAGACC-3' | B | 600 |
| | | 5'-TGAATTTCTCTCTTCAACTAAACA-3' | | 600 |
| | 3 | 5'-CAACTTATTGGTCCCACTTT-3' | C | 800 |
| | | 5'-CACCTATTCACCAGATTTCG-3' | | 800 |
| | 4 | 5'-TTGTAGGAAGTCACCAAAGC-3' | D | 400 |
| | | 5'-TACGATACAGAATATATGTGCCA-3' | | 400 |
| | 5 | 5'-TTGAAATTATCTAACTTTCCATTTT-3' | A | 600 |
| | | 5'-CGCCTTTCCAGTTGTATAAT-3' | | 600 |
| | 6 | 5'-GCTGTGCTTTTATTTTCCAG-3' | B | 600 |
| | | 5'-ACTAAAGTGGGCTTTTTGAA-3' | | 600 |
| | | 5'-CTTAAAACCTTGAGCAGTTCT-3' | C | 600 |
| | | 5'-CAATATTGAAATTATTGGAACAAC-3' | | 600 |
| | 7 | 5'-AGATCTTCCATTCCAAGATC-3' | D | 500 |
| | | 5'-TGCAGCATTATGGTACATTA-3' | | 500 |
| | 8 | 5'-AAGATGTAGCACAATGAGAGTATAAA-3' | A | 700 |
| | | 5'-GAAAACAGTTAGGTGTTTAGAGCAA-3' | | 700 |
| | 9 | 5'-TGGGGAATTATTTGAGAAAG-3' | B | 400 |
| | | 5'-CTTCCAGCACTACAAACTAGAAA-3' | | 400 |
| | 10 | 5'-GCGTGATTTGATAATGACCT-3' | C | 500 |
| | | 5'-TGGGTAGTGTGAAGGGTTC-3' | | 500 |
| | 11 | 5'-AGATTGAGCATACTAAAAGTGAC-3' | D | 500 |
| | | 5'-TGCTTGCTAGACCAATAATTAG-3' | | 500 |
| | 12 | 5'-CCAGGAAATAGAGAGGAAATG-3' | A | 500 |
| | | 5'-CATACCAACAATGGTGAACA-3' | | 500 |
| | 13 | 5'-GCTAAAATACGAGACATATTGC-3' | B | 600 |
| | | 5'-ATTCTGTGGGGTGAAATACC-3' | | 600 |
| | 14 | 5'-GGTGGCATGAAACTGTACT-3' | C | 600 |
| | | 5'-ATACATCCCCAAACTATCTTAAT-3' | | 600 |
| | | 5'-ATGGGAGGAATAGGTGAAGA-3' | D | 800 |
| | | 5'-CAAAGTGGATTACAATACATACA-3' | | 800 |
| | 15 | 5'-TGCCAAATAACGATTTCCTA-3' | A | 600 |
| | | 5'-GTGGATCAGCAGTTTCATTT-3' | | 600 |
| | 16 | 5'-TTGAGGAATTTGTCATCTTGT-3' | B | 600 |
| | | 5'-GACTTCAACCCTCAATCAAA-3' | | 600 |
| | 17 | 5'-CACTGACACACTTTGTCCAC-3' | C | 500 |
| | | 5'-ATCGCACATTCACTGTCATA-3' | | 500 |
| | | 5'-ATTTGCAATGTTTTCTATGG-3' | D | 500 |
| | | 5'-TGCTTAGCTAAAGTTAATGAGTTC-3' | | 500 |
| | 18 | 5'-TTCATTTACGTCTTTTGTGC-3' | A | 600 |
| | | 5'-GGTATATAGTTCTTCCTCATGC-3' | | 600 |
| | 19 | 5'-GTGAAATTGTCTGCCATTCT-3' | B | 800 |
| | | 5'-CAAGCAGTGTTCAAATCTCA-3' | | 800 |
| | 20 | 5'-TGATCCCATCACTTTTACCT-3' | C | 600 |
| | | 5'-TTTCTGGCTAAGTCCTTTTG-3' | | 600 |
| | 21 | 5'-AGAACTTGATGGTAAGTACATG-3' | D | 600 |
| | | 5'-CATTTCAGTTAGCAGCCTTA-3' | | 600 |
| | 22 | 5'-TCTGAACTATCTTCTCTAACTGC-3' | A | 500 |
| | | 5'-AATGATTCTGTTCCCACTGT-3' | | 500 |
| | 23 | 5'-TTCTGTGATATTATGTGTGGTATT-3' | B | 500 |
| | | 5'-AAGAATTACAAGGGCAATGA-3' | | 500 |
| | 24 | 5'-CAGATCTCACTAACAGCCATT-3' | C | 500 |
| | | 5'-TGTCAACATTTATGCTGCTC-3' | | 500 |

### 6. PCR amplification assessment

PCR products were assessed for yield by capillary electrophoresis with a QIAxcelAdvanced (QIAGEN) analyzer and a QIAxcel DNA High Resolution Kit, and markers: QX Intensity Calibration Marker and QX Alignment Marker, according to manufacturer instructions. Amplification yield was assessed based on the positive control. The condition to continue the examination was to achieve the examined samples amplification yield at least at the level of the positive control yield.

### 7. Purification

Amplification products of multiplex reactions A, B, C, D, control -, control +, sample 1 and sample 2 were mixed in one tube, obtaining around 200 µl of PCR amplification products of each sample. Amplification products were purified from starters and nucleotides with a precipitation method on Clean-up columns (A&A Biotechnology), according to manufacturer instructions. Purified products were collected from the column in volume of 50 µl.

### 8. Matrix minisequencing

Matrix minisequencing mixtures were prepared in 4 tubes (negative control, positive control, examined sample 1, examined sample 2) in 70 µl volume comprising: 50 mM Tris-HCl, pH 9,5, 3 mM MgCl₂, 0,75µM 7-Propargylamino-7-deaza-2',3'-dideoxyadenosine-5'thiphosphate-6-FAM, 5-Propargylamino-2',3'-dideoxycytidine+5'thiphosphate-5/6-TAMRA, 7-Propargylamino-7-deaza-2'3'dideoxyguanosine-5'triphosphate-6-ROX, 5-Propargylamino-2',3'-dideoxyuridine-5'triphosphate-6-JOE, 5U thermostable DNA polymerase adding dideoxynucleotides, and 20 µl of purified PCR product. Reaction mixtures were placed in the submatrix fields and sealed with foil cut to matrix dimensions (76 x 26 mm). The matrix was placed in a thermocycler for amplification on a microscopic slide and amplification was run under the following conditions: 35 cycles: denaturation 94°C - 1 min., annealing/elongation 50°C - 1 min., cover temperature 50°C.

After finished amplification the sealing foil was removed and the matrix was placed in a wash station and washed 2x 2 min. in deionized water and then dried by centrifugation in a minicentrifuge for matrix centrifugation.

### 9. Minisequencing

The matrix was analysed on a multicolor scanner GenePix 4300A (Molecular Devices) with 4 scans conducted under the following conditions:
- for 7-Propargylamino-7-deaza-2',3'- dideoxyadenosine-5'thiphosphate-6-FAM excitation wavelength: 492 nm, emission wavelength: 517 nm
- for 5-Propargylamino-2',3'-dideoxycytidine+5'thiphosphate-5/6-TAMRA excitation wavelength: 545 nm, emission wavelength: 575 nm
- for 7-Propargylamino-7-deaza-2'3'dideoxyguanosine-5'triphosphate-6-ROX excitation wavelength: 575 nm, emission wavelength: 600 nm
- for 5-Propargylamino-2',3'-dideoxyuridine-5'triphosphate-6-JOE excitation wavelength: 520 nm, emission wavelength: 548 nm

The presence of fluorescence signal in one color evidences presence of only one sequence variant, corresponding to the color. The presence of signal in two colors in one probe confirms presence of a mutation. The matrix was constructed for identification of mutations in germline and thus signal intensity should be similar; signal intensity variation for one probe is admissible at the level of +/- 20%.

The samples for proficiency tests for "Newborn Screening Quality Assurance Program" Cystic Fibrosis DNA Mutation Detection Proficiency Testing (CFDNAPT), PT Quarter 3 - 2016, denoted: 316C1 and 316C2 were analyzed for mutations in exons 4, 8, 11, 12 of the CFTR gene by DNA enzymatic sequencing with reaction termination by fluorescently labeled dideoxy terminators. Samples were also analyzed by DNA IMAGING method with use of CFTR matrices, which resulted in:

**Table 7**

| Sample | Detected mutations DNA sequencing method | Detected mutations DNA IMAGING method |
|---|---|---|
| 316C1 | F508del (c.1521_1523delCTT) / F508del (c.1521_1523delCTT) | F508del (c.1521_1523delCTT) / F508del / (c.1521_1523delCTT) |
| 316C2 | F508del (c.1521_1523delCTT) / WT | F508del (c.1521_1523delCTT) / WT |

The obtained results are consistent for the DNA method and the reference (DNA sequencing) method. The obtained results are consistent with the sample characteristics.

### Mutation analysis in BRCA1 and BRCA2 genes

### 1. Matrix preparation

Oligonucleotides for matrix preparation were purchased from GENOMED S.A. Synthesis was carried out in 0.2 µmol scale, HPLC purified. Sequences of the individual probes are shown in table 8. Oligonucleotides were dissolved in water in an appropriate water volume for obtaining 200 µM concentration.

**Table 8. Nucleotide sequences of matrix probes for the detection of BRCA 1 and BRCA2 gene mutations**

| gene | mutation | probe |
|---|---|---|
| BRCA1 | c.1016_1016dupA | 5'-C12-AMINO-(dT)₁₈-GACTCCCAGCACAGAAAAAAA-3' |
| | c.1292_1292dupT | 5'-C12-AMINO-(dT)18- ATTCTGGTTCTTCAGAGAAAATAGACTT-3' |
| | c.1380_1380dupA | 5'-C12-AMINO-(dT)18- CAAATCAGTAGAGAGTAATATTGAAGACAAAATA-3' |
| | c.1556deIA | 5'-C12-AMINO-(dT)18-GCCTTCATCCTGAGGATTTTATCA- 3' |
| | c.1687C>T | 5'-C12-AMINO-(dT)18- CATGAGAATAAAACAAAAGGTGATTCTATT-3' |
| | c.181T>G | 5'-C12-AMINO-(dT)₁₈-CAGAAGAAAGGGCCTTCACAG-3' |
| | c.211A>G | 5'-C12-AMINO-(dT)18- GTGTCCTTTATGTAAGAATGATATAACCAAA-3' |
| | c.2197_2201delGAGAA | 5'-C12-AMINO-(dT)₁₈-CAGCATTATTAGACACTTTAACTGTTTCTAGT-3' |
| | c.2338C>T | 5'-C12-AMINO-(dT)18-GTACCTGGTACTGATTATGGCACT- 3' |
| | c.2475delC | 5'-C12-AMINO-(dT)18- CCAATGGATACTTAAAGCCTTCTGT-3' |
| | c.2685_2686delAA | 5'-C12-AMINO-(dT)₁₈-CACTCTGGGTCCTTAAAGAAACA-3' |
| | c.2722G>T | 5'-C12-AMINO-(dT)18- CACTTTTGAATGTGAACAAAAGGAA-3' |
| | c.3052_3056dupAACAT | 5'-C12-AMINO-(dT)18- CACTTTTGAATGTGAACAAAAGGAA-3' |
| | c.3228_3229delAG | 5'-C12-AMINO-(dT)18- GAAAGAGAAATGGGAAATGAGAACAT-3' |
| | c.3485delA | 5'-C12-AMINO-(dT)18- CATTCAAGCAGAACTAGGTAGAAACAG-3' |
| | c.3626delT | 5'-C12-AMINO-(dT)18- CTGTTAGATGATGGTGAAATAAAGGAAG-3' |
| | c.3700_3704delGTAAA | 5'-C12-AMINO-(dT)₁₈-CGAAGAGGGGCCAAGAAAT-3' |
| | c.4035delA | 5'-C12-AMINO-(dT)18- GCTTCCAACACTTGTTATTTGGTAAA-3' |
| | c.4097-2A>G | 5'-C12-AMINO-(dT)18-CAAGGAATTGGTTTCAGATGATGA- 3' |
| | c.4327C>T | 5'-C12-AMINO-(dT)18- TTTTACATCTGAACCTCTGTTTTTGTTATTTA-3' |
| | c.470_471 deICT | 5'-C12-AMINO-(dT)₁₈-TTCTGCCCTTGAGGACCTG-3' |
| | c.4964_4982deI19 | 5'-C12-AMINO-(dT)₁₈-AACCAGTCTCAGTGTCCAACTCT-3' |
| | c.5123C>A | 5'-C12-AMINO-(dT)18- GGAGATACATATGGATACACTCACAAAT-3' |
| | c.5251C>T | 5'-C12-AMINO-(dT)18- CGGACACTGAAATATTTTCTAGGAATTG-3' |
| | c.5266dupC | 5'-C12-AMINO-(dT)₁₈-AGAAACCACCAAGGTCCAAAG-3' |
| | c.5277+1G>A | 5'-C12-AMINO-(dT)₁₈-AAAGCGAGCAAGAGAATCCC-3' |
| | c.5419delA | 5'-C12-AMINO-(dT)₁₈-CAACTTGAGGGAGGGAGCTTT A-3' |
| | c.66_66dupA | 5'-C12-AMINO-(dT)₁₈-TGACTTACCAGATGGGACACTCT-3' |
| | c.68_69delAG | 5'-C12-AMINO-(dT)₁₈-CTGACTTACCAGATGGGACACT-3' |
| | c.697_698delGT | 5'-C12-AMINO-(dT)₁₈-CTTGTGAATTTTCTGAGACGGAT-3' |
| | c.70_73dupTGTC | 5'-C12-AMINO-(dT)₁₈-GCTGACTTACCAGATGGGACA-3' |
| BRCA2 | c.2808_2811 delACAA | 5'-C12-AMINO-(dT)18- GGTTTTATATGGAGACACAGGTGATAA-3' |
| | c.3847_3848delGT | 5'-C12-AMINO-(dT)18- GTTTAAGATAGAAAATCATAATGATAAAACT-3' |
| | c.5351dupA | 5'-C12-AMINO-(dT)18- CAGTATTGAAGAATGTTGAAGATCAAAAAAA-3' |
| | c.5946delT | 5'-C12-AMINO-(dT)₁₈-GTGGGATTTTTAGCACAGCAAG-3' |
| | c.6275_6276delTT | 5'-C12-AMINO-(dT)18- TTTGATTTAATCAGAACTGAGCATAGTC-3' |
| | c.6468_6469delTC | 5'-C12-AMINO-(dT)18- CACTCTATTAAAGTTTCTCCATATCTCTC-3' |
| | c.7480C>T | 5'-C12-AMINO-(dT)18- CAGAATGCCAGAGATATACAGGATATG-3' |
| | c.771_775delTCAAA | 5'-C12-AMINO-(dT)18- CTGTGACAGACAGTGAAAACACAAA-3' |
| | c.8327T>G | 5'-C12-AMINO-(dT)₁₈-GAAGCCCCAGAATCTCTTATGT-3' |
| | c.8537_8538delAG | 5'-C12-AMINO-(dT)18- GGATTATACATATTTCGCAATGAAAGAG-3' |
| | c.9026_9030delATCAT | 5'-C12-AMINO-(dT)18- TGTTAACAGAAGGAAAGAGATACAGAATTT-3' |
| | c.9117+G>T | 5'-C12-AMINO-(dT)18- AAAAAAAACTCAGTATCAACAACTACCG-3' |
| | c.9118-2A>G | 5'-C12-AMINO-(dT)18- CATATGTTGAATTTTTGTTTTGTTTTCTGT-3' |
| | c.9403delC | 5'-C12AMINO-(dT)₁₈-GACCAGAATCCAAATCAGGC-3' |
| | Spotting control | 5'-C12-AMINO-(dT)18-ACG TAC GTA CGT ACG TAC GTA CGT-Cy5-3' |

Matrices were prepared on slides with SUPEREPOXY surface. Before taking out of the box, the case and the box containing slides were cleaned with Microarray Cleanroom Wipes and a 95% ethanol solution and with air from a Microarray Air Jet compressor. Slides were unpacked in a class II laminar air flow chamber and a "mask" with dimensions of 76 x 26 mm, cut out with a laser plotter from self-adhesive PVC foil of 0.35 mm thickness, was applied to the active surface. In every fragment of the foil a circle of 14 mm diameter was cut out, 2.5 mm from the edge of the slide and at regular distances of 4 mm (from the edges of circles). Thereby 4 submatrices of 50 µl volume, resistant to the solutions used in the procedure and to temperature changes in the range used in the procedure, were formed on the slide surface - a substrate of future matrix. Individual matrices are dedicated to analysis of positive control, negative control and two examined samples, wherein analysis of two solutions of the same sample in separate submatrices of one matrix is assumed, so that the analysis result is always confirmed in two tests. The matrix is shown in fig. 1. Slides were placed on a SpotBot 2 Array Printer (Arraylt) working top.

100 µM oligonucleotide solutions in buffer were prepared in a 96-well plate by adding 25 µl of 200 µM oligonucleotide solution and 25 µl of 2x Micro Spotting Solution Plus (Arraylt) solution. Oligonucleotide solutions were mixed by 10x pipetting with 40 µl volume and the plate was centrifuged in a centrifuge for plate centrifugation (2 min., 4000 rpm). Matrices were printed with contact method. Each submatrix was printed in the same probe layout. Said probe layout is shown in fig. 2. The printed matrices were placed in a climatic chamber with relative humidity <30%, at room temperature (∼25°C) for 12 hours after which the matrices were incubated 1 hour in 80°C in laboratory incubator. To wash out unbound DNA, matrices were washed twice for 2 min. at room temperature (22-25°C) in 500 ml of 0,1% SDS solution and once in 500 ml of deionized water with intense mixing in a High Throughput Wash Station. Matrices were dried with 1 min. centrifugation, 500xg in a Microarray High Speed Centrifuge. To block background fluorescence, the matrices were incubated for 60 min. at 60°C in 500 ml Blocklt Buffer and dried with 1 min. centrifugation, 500xg in a Microarray High Speed Centrifuge.

### 2. Examined material

Testing material comprised cheek mucosa swab samples protected on swab pearls for diagnostic examination in respect of BRCA1 and BRCA2 gene mutations, denoted as D832 and D994. Swabs of cheek mucosa were collected on two swab sticks for each sample. Blood dried on a FTA card denoted as EXDNAPC was used as positive control, confirmed in the course of examination by DNA sequencing method to contain no BRCA1 or BRCA2 gene mutations. Swab sticks were broken away to test tubes denoted: "Sample 1" and "Sample 2". 2 fragments of the EXDNAPC card were collected with a chisel of 3 mm diameter and denoted as "Control +".

### 3. DNA isolation

DNA was isolated on a QIAsymphony (QIAGEN) automatic workstation using a QIAsymphony DNA Investigator Kit (Qiagen) reagent kit according to the manufacturer instructions. Isolation was performed in the presence of the negative control, containing all reagent components without the addition of biological material, denoted as containing no DNA, denoted "Control -".

### 4. DNA concentration assesment

DNA concentration was assayed with fluorimetric method with a Qubit 2.0 (Life Technologies) apparatus, using a Qubit ®dsDNA BR (Life Technologies) kit, according to the manufacturer instructions.

Water solutions of working concentration 1 ng/ml were prepared from the isolated DNA based on the results of DNA concentration measurement. Sample "Control -" is not subject to analysis or dilution.

### 5. PCR amplification

PCR amplification was carried out in 8 multiplex PCR reactions in 50 µl volume in PCR tubes of 200 µl volume, said tubes denoted as A, B, C, D, E, F, G, H containing: 1x PCR buffer, MgCl₂ 2,5 mM, dNTPs 250 µM (A&A Biotechnology), BSA fraction V 0,16 mg/ml (Sigma), glycerol (10% w/v), 5U TaqPol (A&A Biotechnology), starters according to table and 10 µl DNA, in multiplex reactions of table 9; PCR reaction temperature profile: initial denaturation 95°C - 3 min., followed by 35 cycles: denaturation 94°C - 1 min., annealing 55°C - 1 min., elongation 72°C - 1 min., final elongation 60°C - 15 min. Two examined samples, negative control and positive control underwent amplification. The amplification was carried out in a GeneAmp PCR System 9700 thermocycler.

**Table 9. Starters for multiplex amplification of BRCA1 and BRCA2 gene fragments:**

| Gene | exon | sequence | PCR reaction | Concentration nM |
|---|---|---|---|---|
| BRCA1 | 2 | 5'-GACGTTGTCATTAGTTCTTTGG-3' | A | 600 |
| | | 5'-GGTCAATTCTGTTCATTTGC-3' | | 600 |
| | 5 | 5'-CTCTTAAGGGCAGTTGTGAG-3' | B | 500 |
| | | 5'-ATGGTTTTATAGGAACGCTATG-3' | | 500 |
| | 8 | 5'-TGTTAGCTGACTGATGATGGT-3' | C | 700 |
| | | 5'-AACCCAGCAATTATTATTAAATAC-3' | | 700 |
| | 11 | 5-GATTTCCACCTCCAAGGTGTATGA-3' | A | 600 |
| | | 5'-CATGGCTCCACATGCAAG-3' | | 600 |
| | | 5'-CAGCTGAGAGGCATCCA-3' | B | 800 |
| | | 5'-GGA TTCTCTGAGCA TGGCA-3' | | 800 |
| | | 5'-GATCCCCTGTGTGAGAGAA-3' | C | 400 |
| | | 5'-TTACTCTCTACTGATTTGGAGTGA-3' | | 400 |
| | | 5'-AGTGATCCTCATGAGGCTTT-3' | D | 600 |
| | | 5'-AGGCCTGA TGTAGGTCTCC-3' | | 600 |
| | | 5'-TACAAGAGCGTCCCCTCAC-3' | E | 600 |
| | | 5'-TTTTCGAGTGATTCTATTGG-3' | | 600 |
| | | 5'-GAGCAGAATGGTCAAGTGAT-3' | F | 600 |
| | | 5'-CAAGCGCATGAATATGCCT-3' | | 600 |
| | | 5'-TGTCAATCCTAGCCTTCCA-3' | G | 500 |
| | | 5'-AAAGCCTTCTGTGTCATTTCT-3' | | 500 |
| | | 5'-CCAAGGGACTAATTCATGGT-3' | H | 700 |
| | | 5'-TTAACTGTCTGTACAGGCTTGAT-3' | | 700 |
| | | 5'-GAAACAAAGTCCAAAAGTCACT-3' | A | 400 |
| | | 5'-ACTTGATGGGAAAAAGTGG-3' | | 400 |
| | | 5'-CATTACTCCAAATAAACATGGACT-3' | B | 500 |
| | | 5'-TACTGGAGCCCACTTCATT-3' | | 500 |
| | | 5'-TGGGAAATGAGAACATTCC-3' | C | 500 |
| | | 5'-CAGGAAGACTTTGTTTATAGACC-3' | | 500 |
| | | 5'-GCAGAACTAGGTAGAAACAGAGG-3' | D | 500 |
| | | 5'-TTTCCTTAATGTCATTTTCAGCA-3' | | 500 |
| | | 5'-TTAGATGATGGTGAAATAAAGGA-3' | E | 600 |
| | | 5'-CAATGATAATAAATTCTCCTCTGT-3' | | 600 |
| | | 5'-TAGCACCGTTGCTACCGAGT-3' | F | 600 |
| | | 5'-TTTCTTCCAAGCCCGTT-3' | | 600 |
| | | 5'-AGCCAGGGAGTTGGTCTG-3' | G | 800 |
| | | 5'-CCCAAAAGCATAAACATTTAGC-3' | | 800 |
| | 12 | 5'-GCGTTTATAGTCTGCTTTTACA-3' | H | 600 |
| | | 5'-TGTCAGCAAACCTAAGAATGT-3' | | 600 |
| | 13 | 5'-AATGGAAAGCTTCTCAAAGTA-3' | D | 600 |
| | | 5'-ATGTTGGAGCTAGGTCCTTAC-3' | | 600 |
| | 16 | 5'-AATTCTTAACAGAGACCAGAAC-3' | E | 500 |
| | | 5'-AAAACTCTTTCCAGAATGTTGT-3' | | 500 |
| | 18 | 5'-GGCTCTTTAGCTTCTTAGGAC-3' | F | 500 |
| | | 5'-CTCAGACTCAAGCATCAGC-3' | | 500 |
| | 20 | 5'-ATATGACGTGTCTGCTCCAC-3' | G | 500 |
| | | 5'-AGTCTTACAAAATGAAGCGG-3' | | 500 |
| | 23 | 5'-CAGAGCAAGACCCTGTCTC-3' | H | 500 |
| | | 5'-GACATTTTAGCCATTCA-3' | | 500 |
| BRCA2 | 9 | 5'-ATAACTGAAATCACCAAAAGTG-3' | A | 600 |
| | | 5'-CTGT AGTTCAACT AAACAGAGG-3' | | 600 |
| | 11 | 5'-AACCCATTTTCAAGAACTCTACCA-3' | A | 500 |
| | | 5'-CTGAAGCTACCTCCAAAACTGTG-3' | | 500 |
| | | 5'-CTTCAAGTAAATGTCATGATTCTGTC- 3' | B | 500 |
| | | 5'-CTGGCAGCAGTATATTTGTTATCT-3' | | 400 |
| | | 5'-AACCAGAAAGAATAAATACT-3' | C | 600 |
| | | 5'-TCCTCAACGCAAATATCTTCAT-3' | | 600 |
| | | 5'-CACCTTGTGATGTTAGTTT-3' | D | 600 |
| | | 5'-TTGGGATATTAAATGTTCTGGAGTA- 3' | | 600 |
| | | 5'-AAAGTAACGAACATTCAGACCA-3' | E | 600 |
| | | 5'-CTGGGTTTCTCTTATCAACACG-3' | | 600 |
| | | 5'-AGTCTTCACTATTCACCTACG-3' | F | 600 |
| | | 5'-GTGAGACTTTGGTTCCT AAT-3' | | 600 |
| | | 5'-TTCAACAAGACAAACAACAGT-3' | G | 600 |
| | | 5'-TGTCAGTTCATCATCTTCCATAAA-3' | | 600 |
| | 15 | 5'-GGCCAGGGGTTGTGCTTTTT-3' | B | 500 |
| | | 5'-AGGATACTAGTTAATGAAATA-3' | | 500 |
| | 18 | 5'-GCAGATACCCAAAAAGTGGC-3' | C | 800 |
| | | 5'-TCTGGACCTCCCAAAAACTG-3' | | 800 |
| | 20 | 5'-CACTGTGCCTGGCCTGATAC-3' | D | 600 |
| | | 5'-ATGTTAAATTCAAAGTCTCTA-3' | | 600 |
| | 23 | 5'-ACTTCTTCCATTGCATCTTTCTCA-3' | E | 600 |
| | | 5'-AAAACAAAACAAAAATTCAACATA-3' | | 600 |
| | 24 | 5'-GCAGCGACAAAAAAAACTCA-3' | F | 600 |
| | | 5'-ATTTGCCAACTGGTAGCTCC-3' | | 600 |
| | 25 | 5'-GCTTTCGCCAAATTCAGCTA-3' | G | 600 |
| | | 5'-TACCAAAATGTGTGGTGATGC-3' | | 600 |

### 6. PCR amplification assessment

PCR products were assessed for yield with capillary electrophoresis with a QIAxcelAdvanced (QIAGEN) analyzer and a QIAxcel DNA High Resolution Kit, and markers: QX Intensity Calibration Marker and QX Alignment Marker, according to manufacturer instructions. Amplification yield was assessed based on positive control. The condition to continue the examination was to achieve the examined samples amplification yield at least at the level of the positive control yield.

### 7. Purification

Amplification products of multiplex reactions A, B, C, D, E, F, G, H, control -, control +, sample 1 and sample 2 were mixed in one tube, obtaining around 200 µl of PCR amplification products of each sample. Amplification products were purified from starters and nucleotides with a precipitation method on Clean-up columns (A&A Biotechnology), according to manufacturer instructions. Purified products were collected from the column in volume of 50 µl

### 8. Matrix minisequencing

Matrix minisequencing mixtures were prepared in 4 tubes (negative control, positive control, examined sample 1, examined sample 2) in 70 µl volume comprising: 50 mM Tris-HCl, pH 9,5, 3 mM MgCl₂, 0,75µM 7-Propargylamino-7-deaza-2',3'-dideoxyadenosine-5'thiphosphate-6-FAM, 5-Propargylamino-2',3'-dideoxycytidine+5'thiphosphate-5/6-TAMRA, 7-Propargylamino-7-deaza-2'3'dideoxyguanosine-5'triphosphate-6-ROX, 5-Propargylamino-2',3'-dideoxyuridine-5'triphosphate-6-JOE, 5U thermostable DNA polymerase (DynaSeq DNA Polymerase, Finnzyme) adding dideoxynucleotides, and 20 µl of purified PCR product. Reaction mixtures were placed in the submatrix fields and sealed with foil cut to matrix dimensions (76 x 26 mm). The matrix was placed in a thermocycler for amplification on microscope slides and amplification was run under the following conditions: 35 cycles: denaturation 94°C - 1 min., annealing/elongation 50°C - 1 min., cover temperature 50°C.

After finished amplification, the sealing foil was removed and the matrix was placed in a wash station and washed 2x 2 min. in deionized water and then dried by centrifugation in a minicentrifuge for matrix centrifugation.

### 9. Minisequencing

The matrix was analyzed on a multicolor scanner GenePix 4300A (Molecular Devices) with 4 scans conducted under the following conditions:
- for 7-Propargylamino-7-deaza-2',3'- dideoxyadenosine-5'thiphosphate-6-FAM excitation wavelength: 492 nm, emission wavelength: 517 nm
- for 5-Propargylamino-2',3'-dideoxycytidine+5'thiphosphate-5/6-TAMRA excitation wavelength: 545 nm, emission wavelength: 575 nm
- for 7-Propargylamino-7-deaza-2'3'dideoxyguanosine-5'triphosphate-6-ROX excitation wavelength: 575 nm, emission wavelength: 600 nm
- for 5-Propargylamino-2',3'-dideoxyuridine-5'triphosphate-6-JOE excitation wavelength: 520 nm, emission wavelength: 548 nm

The presence of fluorescence signal in one color evidences presence of only one sequence variant, corresponding to the color. The presence of signal in two colors in one probe confirms the presence of a mutation. The matrix was constructed for identification of mutations in germline and thus signal intensity should be similar; signal intensity variation for one probe is admissible at the level of +/- 20%.

Samples D832 and D994 were analyzed for presence of mutations in coding part of BRCA1 and BRCA2 genes by enzymatic DNA sequencing with reaction termination by fluorescently labeled dideoxy terminators. The samples were also analyzed with DNA IMAGING using BRCA1/BRCA2 matrices, which resulted in:

**Table 10**

| Sample | Detected mutations DNA sequencing method | Detected mutations DNA IMAGING method |
|---|---|---|
| D832 | c.5266dupC/ WT | c.5266dupC / WT |
| D994 | c.68_69deIAG / WT | c.68_69delAG / WT |

The obtained results are consistent for the DNA method and the reference (DNA sequencing) method.

### Application examples:

1) Proficiency testing samples for "Newborn Screening Quality Assurance Program", Cystic Fibrosis DNA Mutation Detection Proficiency Testing (CFDNAPT), PT Quarter 3 - 2016, denoted: 316C1 and 316C2 were analyzed for CFTR gene mutations in exons 4, 8, 11, 12 with enzymatic DNA sequencing with reaction termination by fluorescently labeled dideoxy terminators. The samples were also analyzed with DNA IMAGING using CFTR matrix, which resulted in:

**Table 11**

| Sample | Detected mutations DNA sequencing method | Detected mutations DNA IMAGING method |
|---|---|---|
| 316C1 | F508del (c.1521_1523delCTT)/ F508del (c.1521_1523delCTT) | F508del (c.1521_1523delCTT)/ F508del / (c.1521_1523delCTT) |
| 316C2 | F508del (c.1521_1523delCTT) /WT | F508del (c.1521_1523delCTT) / WT |

The obtained results are consistent for the DNA method and the reference (DNA sequencing) method. The obtained results are consistent with the sample characteristics.
2) Proficiency testing samples for "Newborn Screening Quality Assurance Program", Cystic Fibrosis DNA Mutation Detection Proficiency Testing (CFDNAPT), PT Quarter 4 - 2016, denoted: 416C3 and 416C5, were analyzed for CFTR gene mutations in exons 4, 8, 11, 12 with enzymatic DNA sequencing with reaction termination by fluorescently labeled dideoxy terminators. The samples were also analyzed with DNA IMAGING using CFTR matrix, which resulted in:

**Table 12**

| Sample | Detected mutations DNA sequencing method | Detected mutations DNA IMAGING method |
|---|---|---|
| 416C3 | F508del (c.1521_1523delCTT)/ F508del (c.1521_1523delCTT) | F508del (c.1521_1523delCTT)/ F508del / (c.1521_1523delCTT) |
| 416C5 | F508del (c.1521_1523delCTT)/ G542X (c.1624G>T) | F508del (c.1521_1523delCTT)/ G542X (c.1624G>T) |

The obtained results are consistent for the DNA method and the reference (DNA sequencing) method. The obtained results are consistent with the sample characteristics.

## Claims

1. Method for the detection of mutations, polymorphisms and specific DNA sequences on DNA matrices, which comprises performing a reaction of binding oligonucleotides of the 3' region complementary sequence, said 3' region directly preceding the detected sequence, with a probe of such a sequence that after addition of one nucleotide the mutated variant and the wild type variant differ in the kind of the complementary nucleotide next in the 3' direction of the probe sequence, followed by matrix analysis wherein the bound molecules are amplified with PCR amplification using thermostable DNA polymerase specific towards dideoxynucleotide triphosphates, which performs matrix sequence conditioned elongation by one nucleotide of oligonucleotides bound to the bed in cycles of alternating denaturation and addition, whereby matrix minisequencing is performed on the collected and purified amplification products by scanning with cut-off filters, which leads to the labeling of presence of the examined sequence.

2. Method according to claim 1, wherein the examined sequence is a mutation.

3. Method according to claim 2, wherein said mutation is a CFTR gene or BRCA1 and/or BRCA2 gene mutation.

4. Method according to claims 1 or 2, wherein each probe comprises at the 5' end C12-AMINO modification and 18dT sequence preceding the proper oligonucleotide sequence.

5. Starters for use in the detection of CFTR and BRCA1 and BRCA2 gene mutations comprising CFTR starters denoted as sequences SEQ ID NO: 235-288, and/or BRCA1 and BRCA2 starters denoted as sequences SEQ ID NO: 159-234, respectively.

## Amended claims

### Amended claims in accordance with Rule 137(2) EPC.

1. Use of the method for the detection of mutations, polymorphisms and specific DNA sequences on DNA matrices, which comprises performing a reaction of binding oligonucleotides of the 3' region complementary sequence, said 3' region directly preceding the detected sequence, with a probe of such a sequence that after addition of one nucleotide the mutated variant and the wild type variant differ in the kind of the complementary nucleotide next in the 3' direction of the probe sequence, followed by matrix analysis wherein the bound molecules are amplified with PCR amplification using thermostable DNA polymerase specific towards dideoxynucleotide triphosphates, which performs matrix sequence conditioned elongation by one nucleotide of oligonucleotides bound to the bed in cycles of alternating denaturation and addition, whereby matrix minisequencing is performed on the collected and purified amplification products by scanning with cut-off filters, which leads to the labeling of presence of the examined sequence, and wherein said mutation is a CFTR gene or BRCA1 and/or BRCA2 gene mutation.

2. Use according to claim 1, wherein each probe comprises at the 5' end C12-AMINO modification and 18dT sequence preceding the proper oligonucleotide sequence.

3. CFTR starters denoted as sequences SEQ ID NO: 235-288, and/or BRCA1 and BRCA2 starters denoted as sequences SEQ ID NO: 159-234, respectively, with exclusion of sequences Nos. 161 and 176, for use in the detection of CFTR and BRCA1 and BRCA2 gene mutations.


SEQUENCE LISTING

<110>  INSTYTUT GENETYKI S¥DOWEJ JOLANTA POWIERSKA - CZARNY

<120>  Method for detection of mutations, polymorphisms and specific DNA
        sequences on DNA matrices with DNA IMAGING techniques for the
       use in medical diagnostics and forensic genetics

<130>  27632.EPO.17

<140>  00
<141>  2017-08-21

<160>  577

<170>  PatentIn version 3.5

<210>  1
<211>  21
<212>  DNA
<213>  Artificial Sequence

<220>
<223>  SNaPShot probe BRCA1 gene mutation

<400>  1
gactcccagc acagaaaaaa a                                                21


<210>  2
<211>  28
<212>  DNA
<213>  Artificial Sequence

<220>
<223>  SNaPShot probe BRCA1 gene mutation

<400>  2
attctggttc ttcagagaaa atagactt                                         28


<210>  3
<211>  34
<212>  DNA
<213>  Artificial Sequence

<220>
<223>  SNaPShot probe BRCA1 gene mutation

<400>  3
caaatcagta gagagtaata ttgaagacaa aata                                  34


<210>  4
<211>  24
<212>  DNA
<213>  Artificial Sequence

<220>
<223>  SNaPShot probe BRCA1 gene mutation

<400>  4
gccttcatcc tgaggatttt atca                                             24

<210>  5
<211>  30
<212>  DNA
<213>  Artificial Sequence

<220>
<223>  SNaPShot probe BRCA1 gene mutation

<400>  5
catgagaata aaacaaaagg tgattctatt                                        30


<210>  6
<211>  21
<212>  DNA
<213>  Artificial Sequence

<220>
<223>  SNaPShot probe BRCA1 gene mutation

<400>  6
cagaagaaag ggccttcaca g                                                 21


<210>  7
<211>  31
<212>  DNA
<213>  Artificial Sequence

<220>
<223>  SNaPShot probe BRCA1 gene mutation

<400>  7
gtgtccttta tgtaagaatg atataaccaa a                                      31


<210>  8
<211>  32
<212>  DNA
<213>  Artificial Sequence

<220>
<223>  SNaPShot probe BRCA1 gene mutation

<400>  8
cagcattatt agacacttta actgtttcta gt                                     32


<210>  9
<211>  24
<212>  DNA
<213>  Artificial Sequence

<220>
<223>  SNaPShot probe BRCA1 gene mutation

<400>  9
gtacctggta ctgattatgg cact                                              24


<210>  10

<211>  25
<212>  DNA
<213>  Artificial Sequence

<220>
<223>  SNaPShot probe BRCA1 gene mutation

<400>  10
ccaatggata cttaaagcct tctgt                                          25


<210>  11
<211>  23
<212>  DNA
<213>  Artificial Sequence

<220>
<223>  SNaPShot probe BRCA1 gene mutation

<400>  11
cactctgggt ccttaaagaa aca                                            23


<210>  12
<211>  25
<212>  DNA
<213>  Artificial Sequence

<220>
<223>  SNaPShot probe BRCA1 gene mutation

<400>  12
cacttttgaa tgtgaacaaa aggaa                                          25


<210>  13
<211>  25
<212>  DNA
<213>  Artificial Sequence

<220>
<223>  SNaPShot probe BRCA1 gene mutation

<400>  13
cacttttgaa tgtgaacaaa aggaa                                          25


<210>  14
<211>  26
<212>  DNA
<213>  Artificial Sequence

<220>
<223>  SNaPShot probe BRCA1 gene mutation

<400>  14
gaaagagaaa tgggaaatga gaacat                                         26


<210>  15
<211>  27
<212>  DNA
<213>  Artificial Sequence

<220>
<223>  SNaPShot probe BRCA1 gene mutation

<400>  15
cattcaagca gaactaggta gaaacag                                          27


<210>  16
<211>  28
<212>  DNA
<213>  Artificial Sequence

<220>
<223>  SNaPShot probe BRCA1 gene mutation

<400>  16
ctgttagatg atggtgaaat aaaggaag                                         28


<210>  17
<211>  19
<212>  DNA
<213>  Artificial Sequence

<220>
<223>  SNaPShot probe BRCA1 gene mutation

<400>  17
cgaagagggg ccaagaaat                                                   19


<210>  18
<211>  26
<212>  DNA
<213>  Artificial Sequence

<220>
<223>  SNaPShot probe BRCA1 gene mutation

<400>  18
gcttccaaca cttgttattt ggtaaa                                           26


<210>  19
<211>  24
<212>  DNA
<213>  Artificial Sequence

<220>
<223>  SNaPShot probe BRCA1 gene mutation

<400>  19
caaggaattg gtttcagatg atga                                             24


<210>  20
<211>  32
<212>  DNA
<213>  Artificial Sequence

<220>
<223>  SNaPShot probe BRCA1 gene mutation

<400>  20
ttttacatct gaacctctgt ttttgttatt ta                                    32


<210>  21
<211>  19
<212>  DNA
<213>  Artificial Sequence

<220>
<223>  SNaPShot probe BRCA1 gene mutation

<400>  21
ttctgccctt gaggacctg                                                  19


<210>  22
<211>  23
<212>  DNA
<213>  Artificial Sequence

<220>
<223>  SNaPShot probe BRCA1 gene mutation

<400>  22
aaccagtctc agtgtccaac tct                                             23


<210>  23
<211>  28
<212>  DNA
<213>  Artificial Sequence

<220>
<223>  SNaPShot probe BRCA1 gene mutation

<400>  23
ggagatacat atggatacac tcacaaat                                        28


<210>  24
<211>  28
<212>  DNA
<213>  Artificial Sequence

<220>
<223>  SNaPShot probe BRCA1 gene mutation

<400>  24
   cggacactga aatattttct aggaattg                                         28


<210>  25
<211>  21
<212>  DNA
<213>  Artificial Sequence

<220>
<223>  SNaPShot probe BRCA1 gene mutation

<400>  25
agaaaccacc aaggtccaaa g                                               21

<210>  26
<211>  20
<212>  DNA
<213>  Artificial Sequence

<220>
<223>  SNaPShot probe BRCA1 gene mutation

<400>  26
aaagcgagca agagaatccc                                                    20


<210>  27
<211>  22
<212>  DNA
<213>  Artificial Sequence

<220>
<223>  SNaPShot probe BRCA1 gene mutation

<400>  27
caacttgagg gagggagctt ta                                                 22


<210>  28
<211>  23
<212>  DNA
<213>  Artificial Sequence

<220>
<223>  SNaPShot probe BRCA1 gene mutation

<400>  28
tgacttacca gatgggacac tct                                                23


<210>  29
<211>  22
<212>  DNA
<213>  Artificial Sequence

<220>
<223>  SNaPShot probe BRCA1 gene mutation

<400>  29
ctgacttacc agatgggaca ct                                                 22


<210>  30
<211>  23
<212>  DNA
<213>  Artificial Sequence

<220>
<223>  SNaPShot probe BRCA1 gene mutation

<400>  30
cttgtgaatt ttctgagacg gat                                                23


<210>  31

<211>  21
<212>  DNA
<213>  Artificial Sequence

<220>
<223>  SNaPShot probe BRCA1 gene mutation

<400>  31
gctgacttac cagatgggac a                                                21


<210>  32
<211>  27
<212>  DNA
<213>  Artificial Sequence

<220>
<223>  SNaPShot probe BRCA1 gene mutation

<400>  32
ggttttatat ggagacacag gtgataa                                          27


<210>  33
<211>  31
<212>  DNA
<213>  Artificial Sequence

<220>
<223>  SNaPShot probe BRCA1 gene mutation

<400>  33
gtttaagata gaaaatcata atgataaaac t                                     31


<210>  34
<211>  31
<212>  DNA
<213>  Artificial Sequence

<220>
<223>  SNaPShot probe BRCA1 gene mutation

<400>  34
cagtattgaa gaatgttgaa gatcaaaaaa a                                     31


<210>  35
<211>  22
<212>  DNA
<213>  Artificial Sequence

<220>
<223>  SNaPShot probe BRCA1 gene mutation

<400>  35
gtgggatttt tagcacagca ag                                               22


<210>  36
<211>  28
<212>  DNA
<213>  Artificial Sequence

<220>
<223>  SNaPShot probe BRCA1 gene mutation

<400>  36
tttgatttaa tcagaactga gcatagtc                                          28


<210>  37
<211>  29
<212>  DNA
<213>  Artificial Sequence

<220>
<223>  SNaPShot probe BRCA1 gene mutation

<400>  37
cactctatta aagtttctcc atatctctc                                         29


<210>  38
<211>  27
<212>  DNA
<213>  Artificial Sequence

<220>
<223>  SNaPShot probe BRCA1 gene mutation

<400>  38
cagaatgcca gagatataca ggatatg                                           27


<210>  39
<211>  25
<212>  DNA
<213>  Artificial Sequence

<220>
<223>  SNaPShot probe BRCA1 gene mutation

<400>  39
ctgtgacaga cagtgaaaac acaaa                                             25


<210>  40
<211>  22
<212>  DNA
<213>  Artificial Sequence

<220>
<223>  SNaPShot probe BRCA1 gene mutation

<400>  40
gaagccccag aatctcttat gt                                                22


<210>  41
<211>  28
<212>  DNA
<213>  Artificial Sequence

<220>
<223>  SNaPShot probe BRCA1 gene mutation

<400>  41
ggattataca tatttcgcaa tgaaagag                                          28


<210>  42
<211>  30
<212>  DNA
<213>  Artificial Sequence

<220>
<223>  SNaPShot probe BRCA1 gene mutation

<400>  42
tgttaacaga aggaaagaga tacagaattt                                        30


<210>  43
<211>  28
<212>  DNA
<213>  Artificial Sequence

<220>
<223>  SNaPShot probe BRCA1 gene mutation

<400>  43
aaaaaaaact cagtatcaac aactaccg                                          28


<210>  44
<211>  30
<212>  DNA
<213>  Artificial Sequence

<220>
<223>  SNaPShot probe BRCA1 gene mutation

<400>  44
catatgttga atttttgttt tgttttctgt                                        30


<210>  45
<211>  20
<212>  DNA
<213>  Artificial Sequence

<220>
<223>  SNaPShot probe BRCA1 gene mutation

<400>  45
gaccagaatc caaatcaggc                                                   20


<210>  46
<211>  25
<212>  DNA
<213>  Artificial Sequence

<220>
<223>  SNaPShot probe CFTR gene mutation

<400>  46
agagaccatg cagaggtcgc ctctg                                             25

<210>  47
<211>  30
<212>  DNA
<213>  Artificial Sequence

<220>
<223>  SNaPShot probe CFTR gene mutation

<400>  47
gtcccacttt ttattctttt gcagagaatg                                        30


<210>  48
<211>  30
<212>  DNA
<213>  Artificial Sequence

<220>
<223>  SNaPShot probe CFTR gene mutation

<400>  48
gatgtttttt ctggagattt atgttctatg                                        30


<210>  49
<211>  25
<212>  DNA
<213>  Artificial Sequence

<220>
<223>  SNaPShot probe CFTR gene mutation

<400>  49
gttctatgga atctttttat attta                                             25


<210>  50
<211>  25
<212>  DNA
<213>  Artificial Sequence

<220>
<223>  SNaPShot probe CFTR gene mutation

<400>  50
tttctctgtt tttccccttt tgtag                                             25


<210>  51
<211>  25
<212>  DNA
<213>  Artificial Sequence

<220>
<223>  SNaPShot probe CFTR gene mutation

<400>  51
tttctctgtt tttccccttt tgtag                                             25


<210>  52

<211>  30
<212>  DNA
<213>  Artificial Sequence

<220>
<223>  SNaPShot probe CFTR gene mutation

<400>  52
ctctcttact gggaagaatc atagcttcct                                        30


<210>  53
<211>  25
<212>  DNA
<213>  Artificial Sequence

<220>
<223>  SNaPShot probe CFTR gene mutation

<400>  53
actgggaaga atcatagctt cctat                                             25


<210>  54
<211>  25
<212>  DNA
<213>  Artificial Sequence

<220>
<223>  SNaPShot probe CFTR gene mutation

<400>  54
tatgacccgg ataacaagga ggaac                                             25


<210>  55
<211>  25
<212>  DNA
<213>  Artificial Sequence

<220>
<223>  SNaPShot probe CFTR gene mutation

<400>  55
gcacatttcg tgtggatcgc tcctt                                             25


<210>  56
<211>  25
<212>  DNA
<213>  Artificial Sequence

<220>
<223>  SNaPShot probe CFTR gene mutation

<400>  56
gcactcctca tggggctaat ctggg                                             25


<210>  57
<211>  30
<212>  DNA
<213>  Artificial Sequence

<220>
<223>  SNaPShot probe CFTR gene mutation

<400>  57
tgtgagatac ttcaatagct cagccttctt                                        30


<210>  58
<211>  30
<212>  DNA
<213>  Artificial Sequence

<220>
<223>  SNaPShot probe CFTR gene mutation

<400>  58
ccctatgcac taatcaaagg aatcatcctc                                        30


<210>  59
<211>  25
<212>  DNA
<213>  Artificial Sequence

<220>
<223>  SNaPShot probe CFTR gene mutation

<400>  59
ggaatcatcc tccggaaaat attca                                             25


<210>  60
<211>  25
<212>  DNA
<213>  Artificial Sequence

<220>
<223>  SNaPShot probe CFTR gene mutation

<400>  60
accatctcat tctgcattgt tctgc                                             25


<210>  61
<211>  25
<212>  DNA
<213>  Artificial Sequence

<220>
<223>  SNaPShot probe CFTR gene mutation

<400>  61
accatctcat tctgcattgt tctgc                                             25


<210>  62
<211>  25
<212>  DNA
<213>  Artificial Sequence

<220>
<223>  SNaPShot probe CFTR gene mutation

<400>  62
accatctcat tctgcattgt tctgc                                          25


<210>  63
<211>  25
<212>  DNA
<213>  Artificial Sequence

<220>
<223>  SNaPShot probe CFTR gene mutation

<400>  63
tcattctgca ttgttctgcg catgg                                          25


<210>  64
<211>  25
<212>  DNA
<213>  Artificial Sequence

<220>
<223>  SNaPShot probe CFTR gene mutation

<400>  64
attgttctgc gcatggcggt cactc                                          25


<210>  65
<211>  25
<212>  DNA
<213>  Artificial Sequence

<220>
<223>  SNaPShot probe CFTR gene mutation

<400>  65
cggcaatttc cctgggctgt acaaa                                          25


<210>  66
<211>  30
<212>  DNA
<213>  Artificial Sequence

<220>
<223>  SNaPShot probe CFTR gene mutation

<400>  66
accttgcctg ctccagtgga tccagcaacc                                     30


<210>  67
<211>  30
<212>  DNA
<213>  Artificial Sequence

<220>
<223>  SNaPShot probe CFTR gene mutation

<400>  67
tagaaagagg acagttgttg gcggttgctg                                     30

<210>  68
<211>  25
<212>  DNA
<213>  Artificial Sequence

<220>
<223>  SNaPShot probe CFTR gene mutation

<400>  68
ttatttccag acttcacttc taatg                                              25


<210>  69
<211>  30
<212>  DNA
<213>  Artificial Sequence

<220>
<223>  SNaPShot probe CFTR gene mutation

<400>  69
gtgattatgg gagaactgga gccttcagag                                         30


<210>  70
<211>  30
<212>  DNA
<213>  Artificial Sequence

<220>
<223>  SNaPShot probe CFTR gene mutation

<400>  70
ttaagcacag tggaagaatt tcattctgtt                                         30


<210>  71
<211>  25
<212>  DNA
<213>  Artificial Sequence

<220>
<223>  SNaPShot probe CFTR gene mutation

<400>  71
cagtggaaga atttcattct gttct                                              25


<210>  72
<211>  25
<212>  DNA
<213>  Artificial Sequence

<220>
<223>  SNaPShot probe CFTR gene mutation

<400>  72
tatgcctggc accattaaag aaaat                                              25


<210>  73

<211>  30
<212>  DNA
<213>  Artificial Sequence

<220>
<223>  SNaPshot probe for CFTR gene mutation

<400>  73
attatgcctg gcaccattaa agaaaatatc                                        30


<210>  74
<211>  30
<212>  DNA
<213>  Artificial Sequence

<220>
<223>  SNaPShot probe CFTR gene mutation

<400>  74
tatgcctggc accattaaag aaaatatcat                                        30


<210>  75
<211>  25
<212>  DNA
<213>  Artificial Sequence

<220>
<223>  SNaPShot probe CFTR gene mutation

<400>  75
ggcaccatta aagaaaatat catct                                             25


<210>  76
<211>  25
<212>  DNA
<213>  Artificial Sequence

<220>
<223>  SNaPShot probe CFTR gene mutation

<400>  76
ctatgatgaa tatagataca gaagc                                             25


<210>  77
<211>  30
<212>  DNA
<213>  Artificial Sequence

<220>
<223>  SNaPShot probe CFTR gene mutation

<400>  77
atatagatac agaagcgtca tcaaagcatg                                        30


<210>  78
<211>  30
<212>  DNA
<213>  Artificial Sequence

<220>
<223>  SNaPShot probe CFTR gene mutation

<400>  78
gtggaatcac actgagtgga ggtcaacgag                                        30


<210>  79
<211>  25
<212>  DNA
<213>  Artificial Sequence

<220>
<223>  SNaPShot probe CFTR gene mutation

<400>  79
tgcagagaaa gacaatatag ttctt                                             25


<210>  80
<211>  25
<212>  DNA
<213>  Artificial Sequence

<220>
<223>  SNaPShot probe CFTR gene mutation

<400>  80
cttggagaag gtggaatcac actga                                             25


<210>  81
<211>  25
<212>  DNA
<213>  Artificial Sequence

<220>
<223>  SNaPShot probe CFTR gene mutation

<400>  81
cttggagaag gtggaatcac actga                                             25


<210>  82
<211>  25
<212>  DNA
<213>  Artificial Sequence

<220>
<223>  SNaPShot probe CFTR gene mutation

<400>  82
tcttggagaa ggtggaatca cactg                                             25


<210>  83
<211>  25
<212>  DNA
<213>  Artificial Sequence

<220>
<223>  SNaPShot probe CFTR gene mutation

<400>  83
gaaggtggaa tcacactgag tggag                                          25


<210>  84
<211>  30
<212>  DNA
<213>  Artificial Sequence

<220>
<223>  SNaPShot probe CFTR gene mutation

<400>  84
cttggagaag gtggaatcac actgagtgga                                     30


<210>  85
<211>  30
<212>  DNA
<213>  Artificial Sequence

<220>
<223>  SNaPShot probe CFTR gene mutation

<400>  85
ggagaaggtg gaatcacact gagtggaggt                                     30


<210>  86
<211>  25
<212>  DNA
<213>  Artificial Sequence

<220>
<223>  SNaPShot probe CFTR gene mutation

<400>  86
tggaatcaca ctgagtggag gtcaa                                          25


<210>  87
<211>  30
<212>  DNA
<213>  Artificial Sequence

<220>
<223>  SNaPShot probe CFTR gene mutation

<400>  87
aaggtggaat cacactgagt ggaggtcaac                                     30


<210>  88
<211>  25
<212>  DNA
<213>  Artificial Sequence

<220>
<223>  SNaPShot probe CFTR gene mutation

<400>  88
actgagtgga ggtcaacgag caaga                                          25

<210>  89
<211>  25
<212>  DNA
<213>  Artificial Sequence

<220>
<223>  SNaPShot probe CFTR gene mutation

<400>  89
aggtcaacga gcaagaattt cttta                                              25


<210>  90
<211>  25
<212>  DNA
<213>  Artificial Sequence

<220>
<223>  SNaPShot probe CFTR gene mutation

<400>  90
caacgagcaa gaatttcttt agcaa                                              25


<210>  91
<211>  25
<212>  DNA
<213>  Artificial Sequence

<220>
<223>  SNaPShot probe CFTR gene mutation

<400>  91
caacgagcaa gaatttcttt agcaa                                              25


<210>  92
<211>  30
<212>  DNA
<213>  Artificial Sequence

<220>
<223>  SNaPShot probe CFTR gene mutation

<400>  92
tgtaatttaa tttccatttt ctttttagag                                         30


<210>  93
<211>  30
<212>  DNA
<213>  Artificial Sequence

<220>
<223>  SNaPShot probe CFTR gene mutation

<400>  93
tttaatttcc attttctttt tagagcagta                                         30


<210>  94

<211>  30
<212>  DNA
<213>  Artificial Sequence

<220>
<223>  SNaPShot probe CFTR gene mutation

<400>  94
aagatgctga tttgtattta ttagactctc                                        30


<210>  95
<211>  25
<212>  DNA
<213>  Artificial Sequence

<220>
<223>  SNaPShot probe CFTR gene mutation

<400>  95
ttgtatttat tagactctcc ttttg                                             25


<210>  96
<211>  30
<212>  DNA
<213>  Artificial Sequence

<220>
<223>  SNaPShot probe CFTR gene mutation

<400>  96
cagactttag ctcaaaactc atgggatgtg                                        30


<210>  97
<211>  30
<212>  DNA
<213>  Artificial Sequence

<220>
<223>  SNaPShot probe CFTR gene mutation

<400>  97
tctattctca atccaatcaa ctctatacga                                        30


<210>  98
<211>  30
<212>  DNA
<213>  Artificial Sequence

<220>
<223>  SNaPShot probe CFTR gene mutation

<400>  98
aactctatac gaaaattttc cattgtgcaa                                         30


<210>  99
<211>  30
<212>  DNA
<213>  Artificial Sequence

<220>
<223>  SNaPShot probe CFTR gene mutation

<400>  99
ggcttggaaa taagtgaaga aattaacgaa                                        30


<210>  100
<211>  30
<212>  DNA
<213>  Artificial Sequence

<220>
<223>  SNaPShot probe CFTR gene mutation

<400>  100
tatggagagc ataccagcag tgactacatg                                        30


<210>  101
<211>  25
<212>  DNA
<213>  Artificial Sequence

<220>
<223>  SNaPShot probe CFTR gene mutation

<400>  101
agtgactaca tggaacacat acctt                                             25


<210>  102
<211>  30
<212>  DNA
<213>  Artificial Sequence

<220>
<223>  SNaPShot probe CFTR gene mutation

<400>  102
atttcctatt tgctttacag cactcctctt                                        30


<210>  103
<211>  25
<212>  DNA
<213>  Artificial Sequence

<220>
<223>  SNaPShot probe CFTR gene mutation

<400>  103
gcagtgatta tcaccagcac cagtt                                             25


<210>  104
<211>  25
<212>  DNA
<213>  Artificial Sequence

<220>
<223>  SNaPShot probe CFTR gene mutation

<400>  104
gtgattatca ccagcaccag ttcgt                                          25


<210>  105
<211>  30
<212>  DNA
<213>  Artificial Sequence

<220>
<223>  SNaPShot probe CFTR gene mutation

<400>  105
   catactctaa tcacagtgtc gaaaatttta                                      30


<210>  106
<211>  30
<212>  DNA
<213>  Artificial Sequence

<220>
<223>  SNaPShot probe CFTR gene mutation

<400>  106
ttgttacaag cttaaaagga ctatggacac                                     30


<210>  107
<211>  30
<212>  DNA
<213>  Artificial Sequence

<220>
<223>  SNaPShot probe CFTR gene mutation

<400>  107
ttacaagctt aaaaggacta tggacacttc                                     30


<210>  108
<211>  25
<212>  DNA
<213>  Artificial Sequence

<220>
<223>  SNaPShot probe CFTR gene mutation

<400>  108
aagcttaaaa ggactatgga cactt                                          25


<210>  109
<211>  30
<212>  DNA
<213>  Artificial Sequence

<220>
<223>  SNaPShot probe CFTR gene mutation

<400>  109
acaagcttaa aaggactatg gacacttcgt                                     30

<210>  110
<211>  30
<212>  DNA
<213>  Artificial Sequence

<220>
<223>  SNaPShot probe CFTR gene mutation

<400>  110
gactatggac acttcgtgcc ttcggacggc                                        30


<210>  111
<211>  30
<212>  DNA
<213>  Artificial Sequence

<220>
<223>  SNaPShot probe CFTR gene mutation

<400>  111
aaactctgtt ccacaaagct ctgaatttac                                        30


<210>  112
<211>  25
<212>  DNA
<213>  Artificial Sequence

<220>
<223>  SNaPShot probe CFTR gene mutation

<400>  112
gctctgaatt tacatactgc caact                                             25


<210>  113
<211>  25
<212>  DNA
<213>  Artificial Sequence

<220>
<223>  SNaPShot probe CFTR gene mutation

<400>  113
tacatactgc caactggttc ttgta                                             25


<210>  114
<211>  25
<212>  DNA
<213>  Artificial Sequence

<220>
<223>  SNaPShot probe CFTR gene mutation

<400>  114
ctgtcaacac tgcgctggtt ccaaa                                             25


<210>  115

<211>  25
<212>  DNA
<213>  Artificial Sequence

<220>
<223>  SNaPShot probe CFTR gene mutation

<400>  115
aatgttgtta tttttatttc agatg                                          25


<210>  116
<211>  30
<212>  DNA
<213>  Artificial Sequence

<220>
<223>  SNaPShot probe CFTR gene mutation

<400>  116
ctgttggcat gtcaatgaac ttaaagactc                                     30


<210>  117
<211>  25
<212>  DNA
<213>  Artificial Sequence

<220>
<223>  SNaPShot probe CFTR gene mutation

<400>  117
cacacgtgaa gaaagatgac atctg                                          25


<210>  118
<211>  25
<212>  DNA
<213>  Artificial Sequence

<220>
<223>  SNaPShot probe CFTR gene mutation

<400>  118
gtcaaagatc tcacagcaaa ataca                                          25


<210>  119
<211>  25
<212>  DNA
<213>  Artificial Sequence

<220>
<223>  SNaPShot probe CFTR gene mutation

<400>  119
catattagag aacatttcct tctca                                          25


<210>  120
<211>  25
<212>  DNA
<213>  Artificial Sequence

<220>
<223>  SNaPShot probe CFTR gene mutation

<400>  120
catttccttc tcaataagtc ctggc                                          25


<210>  121
<211>  25
<212>  DNA
<213>  Artificial Sequence

<220>
<223>  SNaPShot probe CFTR gene mutation

<400>  121
gcagtgatta tcaccagcac cagtt                                          25


<210>  122
<211>  30
<212>  DNA
<213>  Artificial Sequence

<220>
<223>  SNaPShot probe CFTR gene mutation

<400>  122
aggtgggcct cttgggaaga actggatcag                                     30


<210>  123
<211>  25
<212>  DNA
<213>  Artificial Sequence

<220>
<223>  SNaPShot probe CFTR gene mutation

<400>  123
ttgggaagaa ctggatcagg gaaga                                          25


<210>  124
<211>  25
<212>  DNA
<213>  Artificial Sequence

<220>
<223>  SNaPShot probe CFTR gene mutation

<400>  124
ggatcaggga agagtacttt gttat                                          25


<210>  125
<211>  30
<212>  DNA
<213>  Artificial Sequence

<220>
<223>  SNaPShot probe CFTR gene mutation

<400>  125
agaactggat cagggaagag tactttgtta                                        30


<210>  126
<211>  25
<212>  DNA
<213>  Artificial Sequence

<220>
<223>  SNaPShot probe CFTR gene mutation

<400>  126
gaacactgaa ggagaaatcc agatc                                             25


<210>  127
<211>  25
<212>  DNA
<213>  Artificial Sequence

<220>
<223>  SNaPShot probe CFTR gene mutation

<400>  127
gggattcaat aactttgcaa cagtg                                             25


<210>  128
<211>  25
<212>  DNA
<213>  Artificial Sequence

<220>
<223>  SNaPShot probe CFTR gene mutation

<400>  128
gattcaataa ctttgcaaca gtgga                                             25


<210>  129
<211>  30
<212>  DNA
<213>  Artificial Sequence

<220>
<223>  SNaPShot probe CFTR gene mutation

<400>  129
caataacttt gcaacagtgg aggaaagcct                                        30


<210>  130
<211>  25
<212>  DNA
<213>  Artificial Sequence

<220>
<223>  SNaPShot probe CFTR gene mutation

<400>  130
ggaaagcctt tggagtgata ccaca                                             25

<210>  131
<211>  25
<212>  DNA
<213>  Artificial Sequence

<220>
<223>  SNaPShot probe CFTR gene mutation

<400>  131
   ttttttctgg aacatttaga aaaaa                                                25


<210>  132
<211>  25
<212>  DNA
<213>  Artificial Sequence

<220>
<223>  SNaPShot probe CFTR gene mutation

<400>  132
   ttttttctgg aacatttaga aaaaa                                                25


<210>  133
<211>  25
<212>  DNA
<213>  Artificial Sequence

<220>
<223>  SNaPShot probe CFTR gene mutation

<400>  133
ggatccctat gaacagtgga gtgat                                               25


<210>  134
<211>  30
<212>  DNA
<213>  Artificial Sequence

<220>
<223>  SNaPShot probe CFTR gene mutation

<400>  134
cctatgaaca gtggagtgat caagaaatat                                          30


<210>  135
<211>  25
<212>  DNA
<213>  Artificial Sequence

<220>
<223>  SNaPShot probe CFTR gene mutation

<400>  135
gtgtcctaag ccatggccac aagca                                               25


<210>  136

<211>  30
<212>  DNA
<213>  Artificial Sequence

<220>
<223>  SNaPShot probe CFTR gene mutation

<400>  136
ttctggagat ttatgttcta tggaatcttt                                        30


<210>  137
<211>  30
<212>  DNA
<213>  Artificial Sequence

<220>
<223>  SNaPShot probe CFTR gene mutation

<400>  137
attgtgagga cactgctcct acacccagcc                                        30


<210>  138
<211>  30
<212>  DNA
<213>  Artificial Sequence

<220>
<223>  SNaPShot probe CFTR gene mutation

<400>  138
gaggacactg ctcctacacc cagccatttt                                        30


<210>  139
<211>  30
<212>  DNA
<213>  Artificial Sequence

<220>
<223>  SNaPShot probe CFTR gene mutation

<400>  139
cttgtgatta cctcagaaat gattgaaaat                                        30


<210>  140
<211>  30
<212>  DNA
<213>  Artificial Sequence

<220>
<223>  SNaPShot probe CFTR gene mutation

<400>  140
ggaagaagca atggaaaaaa tgattgaaaa                                        30


<210>  141
<211>  30
<212>  DNA
<213>  Artificial Sequence

<220>
<223>  SNaPShot probe CFTR gene mutation

<400>  141
tagctcagcc ttcttcttct cagggttctt                                        30


<210>  142
<211>  30
<212>  DNA
<213>  Artificial Sequence

<220>
<223>  SNaPShot probe CFTR gene mutation

<400>  142
catcctccgg aaaatattca ccaccatctc                                        30


<210>  143
<211>  30
<212>  DNA
<213>  Artificial Sequence

<220>
<223>  SNaPShot probe CFTR gene mutation

<400>  143
actcggcaat ttccctgggc tgtacaaaca                                        30


<210>  144
<211>  30
<212>  DNA
<213>  Artificial Sequence

<220>
<223>  SNaPShot probe CFTR gene mutation

<400>  144
tcggcaattt ccctgggctg tacaaacatg                                        30


<210>  145
<211>  30
<212>  DNA
<213>  Artificial Sequence

<220>
<223>  SNaPShot probe CFTR gene mutation

<400>  145
ccctgggctg tacaaacatg gtatgactct                                        30


<210>  146
<211>  30
<212>  DNA
<213>  Artificial Sequence

<220>
<223>  SNaPShot probe CFTR gene mutation

<400>  146
aagcacagtg gaagaatttc attctgttct                                        30


<210>  147
<211>  30
<212>  DNA
<213>  Artificial Sequence

<220>
<223>  SNaPShot probe CFTR gene mutation

<400>  147
tatcatcttt ggtgtttcct atgatgaata                                        30


<210>  148
<211>  30
<212>  DNA
<213>  Artificial Sequence

<220>
<223>  SNaPShot probe CFTR gene mutation

<400>  148
aagactccct tacaaatgaa tggcatcgaa                                        30


<210>  149
<211>  30
<212>  DNA
<213>  Artificial Sequence

<220>
<223>  SNaPShot probe CFTR gene mutation

<400>  149
tgatcagcac tggccccacg cttcaggcac                                        30


<210>  150
<211>  30
<212>  DNA
<213>  Artificial Sequence

<220>
<223>  SNaPShot probe CFTR gene mutation

<400>  150
ctcaggcaaa cttgactgaa ctggatatat                                        30


<210>  151
<211>  30
<212>  DNA
<213>  Artificial Sequence

<220>
<223>  SNaPShot probe CFTR gene mutation

<400>  151
atgcagtgat tatcaccagc accagttcgt                                        30

<210>  152
<211>  30
<212>  DNA
<213>  Artificial Sequence

<220>
<223>  SNaPShot probe CFTR gene mutation

<400>  152
tcatcttgtt acaagcttaa aaggactatg                                        30


<210>  153
<211>  30
<212>  DNA
<213>  Artificial Sequence

<220>
<223>  SNaPShot probe CFTR gene mutation

<400>  153
atttcacagg caggagtcca attttcactc                                        30


<210>  154
<211>  30
<212>  DNA
<213>  Artificial Sequence

<220>
<223>  SNaPShot probe CFTR gene mutation

<400>  154
tttcagatgc gatctgtgag ccgagtcttt                                        30


<210>  155
<211>  30
<212>  DNA
<213>  Artificial Sequence

<220>
<223>  SNaPShot probe CFTR gene mutation

<400>  155
tgactgtcaa agatctcaca gcaaaataca                                        30


<210>  156
<211>  30
<212>  DNA
<213>  Artificial Sequence

<220>
<223>  SNaPShot probe CFTR gene mutation

<400>  156
tgccaacaga aggtaaacct accaagtcaa                                        30


<210>  157

<211>  30
<212>  DNA
<213>  Artificial Sequence

<220>
<223>  SNaPShot probe CFTR gene mutation

<400>  157
ataggtgggc ctcttgggaa gaactggatc                                        30


<210>  158
<211>  30
<212>  DNA
<213>  Artificial Sequence

<220>
<223>  SNaPShot probe CFTR gene mutation

<400>  158
tggatcaggg aagagtactt tgttatcagc                                        30


<210>  159
<211>  22
<212>  DNA
<213>  Artificial Sequence

<220>
<223>  PCR primer for BRCA1 and BRCA2 gene regions

<400>  159
gacgttgtca ttagttcttt gg                                                22


<210>  160
<211>  20
<212>  DNA
<213>  Artificial Sequence

<220>
<223>  PCR primer for BRCA1 and BRCA2 gene regions

<400>  160
ggtcaattct gttcatttgc                                                   20


<210>  161
<211>  20
<212>  DNA
<213>  Artificial Sequence

<220>
<223>  PCR primer for BRCA1 and BRCA2 gene regions

<400>  161
ctcttaaggg cagttgtgag                                                   20


<210>  162
<211>  22
<212>  DNA
<213>  Artificial Sequence

<220>
<223>  PCR primer for BRCA1 and BRCA2 gene regions

<400>  162
atggttttat aggaacgcta tg                                                22


<210>  163
<211>  21
<212>  DNA
<213>  Artificial Sequence

<220>
<223>  PCR primer for BRCA1 and BRCA2 gene regions

<400>  163
tgttagctga ctgatgatgg t                                                 21


<210>  164
<211>  24
<212>  DNA
<213>  Artificial Sequence

<220>
<223>  PCR primer for BRCA1 and BRCA2 gene regions

<400>  164
aacccagcaa ttattattaa atac                                              24


<210>  165
<211>  24
<212>  DNA
<213>  Artificial Sequence

<220>
<223>  PCR primer for BRCA1 and BRCA2 gene regions

<400>  165
gatttccacc tccaaggtgt atga                                              24


<210>  166
<211>  18
<212>  DNA
<213>  Artificial Sequence

<220>
<223>  PCR primer for BRCA1 and BRCA2 gene regions

<400>  166
catggctcca catgcaag                                                     18


<210>  167
<211>  17
<212>  DNA
<213>  Artificial Sequence

<220>
<223>  PCR primer for BRCA1 and BRCA2 gene regions

<400>  167
cagctgagag gcatcca                                                      17


<210>  168
<211>  19
<212>  DNA
<213>  Artificial Sequence

<220>
<223>  PCR primer for BRCA1 and BRCA2 gene regions

<400>  168
ggattctctg agcatggca                                                    19


<210>  169
<211>  19
<212>  DNA
<213>  Artificial Sequence

<220>
<223>  PCR primer for BRCA1 and BRCA2 gene regions

<400>  169
gatcccctgt gtgagagaa                                                    19


<210>  170
<211>  24
<212>  DNA
<213>  Artificial Sequence

<220>
<223>  PCR primer for BRCA1 and BRCA2 gene regions

<400>  170
ttactctcta ctgatttgga gtga                                              24


<210>  171
<211>  20
<212>  DNA
<213>  Artificial Sequence

<220>
<223>  PCR primer for BRCA1 and BRCA2 gene regions

<400>  171
agtgatcctc atgaggcttt                                                   20


<210>  172
<211>  19
<212>  DNA
<213>  Artificial Sequence

<220>
<223>  PCR primer for BRCA1 and BRCA2 gene regions

<400>  172
aggcctgatg taggtctcc                                                    19

<210>  173
<211>  19
<212>  DNA
<213>  Artificial Sequence

<220>
<223>  PCR primer for BRCA1 and BRCA2 gene regions

<400>  173
tacaagagcg tcccctcac                                                    19


<210>  174
<211>  20
<212>  DNA
<213>  Artificial Sequence

<220>
<223>  PCR primer for BRCA1 and BRCA2 gene regions

<400>  174
ttttcgagtg attctattgg                                                   20


<210>  175
<211>  20
<212>  DNA
<213>  Artificial Sequence

<220>
<223>  PCR primer for BRCA1 and BRCA2 gene regions

<400>  175
gagcagaatg gtcaagtgat                                                   20


<210>  176
<211>  19
<212>  DNA
<213>  Artificial Sequence

<220>
<223>  PCR primer for BRCA1 and BRCA2 gene regions

<400>  176
caagcgcatg aatatgcct                                                    19


<210>  177
<211>  19
<212>  DNA
<213>  Artificial Sequence

<220>
<223>  PCR primer for BRCA1 and BRCA2 gene regions

<400>  177
tgtcaatcct agccttcca                                                    19


<210>  178

<211>  21
<212>  DNA
<213>  Artificial Sequence

<220>
<223>  PCR primer for BRCA1 and BRCA2 gene regions

<400>  178
aaagccttct gtgtcatttc t                                                21


<210>  179
<211>  20
<212>  DNA
<213>  Artificial Sequence

<220>
<223>  PCR primer for BRCA1 and BRCA2 gene regions

<400>  179
ccaagggact aattcatggt                                                  20


<210>  180
<211>  23
<212>  DNA
<213>  Artificial Sequence

<220>
<223>  PCR primer for BRCA1 and BRCA2 gene regions

<400>  180
ttaactgtct gtacaggctt gat                                              23


<210>  181
<211>  22
<212>  DNA
<213>  Artificial Sequence

<220>
<223>  PCR primer for BRCA1 and BRCA2 gene regions

<400>  181
gaaacaaagt ccaaaagtca ct                                               22


<210>  182
<211>  19
<212>  DNA
<213>  Artificial Sequence

<220>
<223>  PCR primer for BRCA1 and BRCA2 gene regions

<400>  182
acttgatggg aaaaagtgg                                                   19


<210>  183
<211>  24
<212>  DNA
<213>  Artificial Sequence

<220>
<223>  PCR primer for BRCA1 and BRCA2 gene regions

<400>  183
cattactcca aataaacatg gact                                              24


<210>  184
<211>  19
<212>  DNA
<213>  Artificial Sequence

<220>
<223>  PCR primer for BRCA1 and BRCA2 gene regions

<400>  184
tactggagcc cacttcatt                                                    19


<210>  185
<211>  19
<212>  DNA
<213>  Artificial Sequence

<220>
<223>  PCR primer for BRCA1 and BRCA2 gene regions

<400>  185
tgggaaatga gaacattcc                                                    19


<210>  186
<211>  23
<212>  DNA
<213>  Artificial Sequence

<220>
<223>  PCR primer for BRCA1 and BRCA2 gene regions

<400>  186
caggaagact ttgtttatag acc                                               23


<210>  187
<211>  23
<212>  DNA
<213>  Artificial Sequence

<220>
<223>  PCR primer for BRCA1 and BRCA2 gene regions

<400>  187
gcagaactag gtagaaacag agg                                               23


<210>  188
<211>  23
<212>  DNA
<213>  Artificial Sequence

<220>
<223>  PCR primer for BRCA1 and BRCA2 gene regions

<400>  188
tttccttaat gtcattttca gca                                              23


<210>  189
<211>  23
<212>  DNA
<213>  Artificial Sequence

<220>
<223>  PCR primer for BRCA1 and BRCA2 gene regions

<400>  189
ttagatgatg gtgaaataaa gga                                              23


<210>  190
<211>  24
<212>  DNA
<213>  Artificial Sequence

<220>
<223>  PCR primer for BRCA1 and BRCA2 gene regions

<400>  190
caatgataat aaattctcct ctgt                                             24


<210>  191
<211>  20
<212>  DNA
<213>  Artificial Sequence

<220>
<223>  PCR primer for BRCA1 and BRCA2 gene regions

<400>  191
tagcaccgtt gctaccgagt                                                  20


<210>  192
<211>  17
<212>  DNA
<213>  Artificial Sequence

<220>
<223>  PCR primer for BRCA1 and BRCA2 gene regions

<400>  192
tttcttccaa gcccgtt                                                     17


<210>  193
<211>  18
<212>  DNA
<213>  Artificial Sequence

<220>
<223>  PCR primer for BRCA1 and BRCA2 gene regions

<400>  193
agccagggag ttggtctg                                                    18

<210>  194
<211>  22
<212>  DNA
<213>  Artificial Sequence

<220>
<223>  PCR primer for BRCA1 and BRCA2 gene regions

<400>  194
cccaaaagca taaacattta gc                                                22


<210>  195
<211>  22
<212>  DNA
<213>  Artificial Sequence

<220>
<223>  PCR primer for BRCA1 and BRCA2 gene regions

<400>  195
gcgtttatag tctgctttta ca                                                22


<210>  196
<211>  21
<212>  DNA
<213>  Artificial Sequence

<220>
<223>  PCR primer for BRCA1 and BRCA2 gene regions

<400>  196
tgtcagcaaa cctaagaatg t                                                 21


<210>  197
<211>  21
<212>  DNA
<213>  Artificial Sequence

<220>
<223>  PCR primer for BRCA1 and BRCA2 gene regions

<400>  197
aatggaaagc ttctcaaagt a                                                 21


<210>  198
<211>  21
<212>  DNA
<213>  Artificial Sequence

<220>
<223>  PCR primer for BRCA1 and BRCA2 gene regions

<400>  198
atgttggagc taggtcctta c                                                 21


<210>  199

<211>  22
<212>  DNA
<213>  Artificial Sequence

<220>
<223>  PCR primer for BRCA1 and BRCA2 gene regions

<400>  199
aattcttaac agagaccaga ac                                                22


<210>  200
<211>  22
<212>  DNA
<213>  Artificial Sequence

<220>
<223>  PCR primer for BRCA1 and BRCA2 gene regions

<400>  200
aaaactcttt ccagaatgtt gt                                                22


<210>  201
<211>  21
<212>  DNA
<213>  Artificial Sequence

<220>
<223>  PCR primer for BRCA1 and BRCA2 gene regions

<400>  201
ggctctttag cttcttagga c                                                 21


<210>  202
<211>  19
<212>  DNA
<213>  Artificial Sequence

<220>
<223>  PCR primer for BRCA1 and BRCA2 gene regions

<400>  202
ctcagactca agcatcagc                                                    19


<210>  203
<211>  20
<212>  DNA
<213>  Artificial Sequence

<220>
<223>  PCR primer for BRCA1 and BRCA2 gene regions

<400>  203
atatgacgtg tctgctccac                                                   20


<210>  204
<211>  20
<212>  DNA
<213>  Artificial Sequence

<220>
<223>  PCR primer for BRCA1 and BRCA2 gene regions

<400>  204
agtcttacaa aatgaagcgg                                                   20


<210>  205
<211>  19
<212>  DNA
<213>  Artificial Sequence

<220>
<223>  PCR primer for BRCA1 and BRCA2 gene regions

<400>  205
cagagcaaga ccctgtctc                                                    19


<210>  206
<211>  17
<212>  DNA
<213>  Artificial Sequence

<220>
<223>  PCR primer for BRCA1 and BRCA2 gene regions

<400>  206
gacattttag ccattca                                                      17


<210>  207
<211>  22
<212>  DNA
<213>  Artificial Sequence

<220>
<223>  PCR primer for BRCA1 and BRCA2 gene regions

<400>  207
ataactgaaa tcaccaaaag tg                                                22


<210>  208
<211>  22
<212>  DNA
<213>  Artificial Sequence

<220>
<223>  PCR primer for BRCA1 and BRCA2 gene regions

<400>  208
ctgtagttca actaaacaga gg                                                22


<210>  209
<211>  24
<212>  DNA
<213>  Artificial Sequence

<220>
<223>  PCR primer for BRCA1 and BRCA2 gene regions

<400>  209
aacccatttt caagaactct acca                                              24


<210>  210
<211>  23
<212>  DNA
<213>  Artificial Sequence

<220>
<223>  PCR primer for BRCA1 and BRCA2 gene regions

<400>  210
ctgaagctac ctccaaaact gtg                                               23


<210>  211
<211>  26
<212>  DNA
<213>  Artificial Sequence

<220>
<223>  PCR primer for BRCA1 and BRCA2 gene regions

<400>  211
cttcaagtaa atgtcatgat tctgtc                                            26


<210>  212
<211>  24
<212>  DNA
<213>  Artificial Sequence

<220>
<223>  PCR primer for BRCA1 and BRCA2 gene regions

<400>  212
ctggcagcag tatatttgtt atct                                              24


<210>  213
<211>  20
<212>  DNA
<213>  Artificial Sequence

<220>
<223>  PCR primer for BRCA1 and BRCA2 gene regions

<400>  213
aaccagaaag aataaatact                                                   20


<210>  214
<211>  22
<212>  DNA
<213>  Artificial Sequence

<220>
<223>  PCR primer for BRCA1 and BRCA2 gene regions

<400>  214
tcctcaacgc aaatatcttc at                                                22

<210>  215
<211>  19
<212>  DNA
<213>  Artificial Sequence

<220>
<223>  PCR primer for BRCA1 and BRCA2 gene regions

<400>  215
caccttgtga tgttagttt                                                    19


<210>  216
<211>  25
<212>  DNA
<213>  Artificial Sequence

<220>
<223>  PCR primer for BRCA1 and BRCA2 gene regions

<400>  216
ttgggatatt aaatgttctg gagta                                             25


<210>  217
<211>  22
<212>  DNA
<213>  Artificial Sequence

<220>
<223>  PCR primer for BRCA1 and BRCA2 gene regions

<400>  217
aaagtaacga acattcagac ca                                                22


<210>  218
<211>  22
<212>  DNA
<213>  Artificial Sequence

<220>
<223>  PCR primer for BRCA1 and BRCA2 gene regions

<400>  218
ctgggtttct cttatcaaca cg                                                22


<210>  219
<211>  21
<212>  DNA
<213>  Artificial Sequence

<220>
<223>  PCR primer for BRCA1 and BRCA2 gene regions

<400>  219
agtcttcact attcacctac g                                                 21


<210>  220

<211>  20
<212>  DNA
<213>  Artificial Sequence

<220>
<223>  PCR primer for BRCA1 and BRCA2 gene regions

<400>  220
gtgagacttt ggttcctaat                                                   20


<210>  221
<211>  21
<212>  DNA
<213>  Artificial Sequence

<220>
<223>  PCR primer for BRCA1 and BRCA2 gene regions

<400>  221
ttcaacaaga caaacaacag t                                                 21


<210>  222
<211>  24
<212>  DNA
<213>  Artificial Sequence

<220>
<223>  PCR primer for BRCA1 and BRCA2 gene regions

<400>  222
tgtcagttca tcatcttcca taaa                                              24


<210>  223
<211>  20
<212>  DNA
<213>  Artificial Sequence

<220>
<223>  PCR primer for BRCA1 and BRCA2 gene regions

<400>  223
ggccaggggt tgtgcttttt                                                   20


<210>  224
<211>  21
<212>  DNA
<213>  Artificial Sequence

<220>
<223>  PCR primer for BRCA1 and BRCA2 gene regions

<400>  224
aggatactag ttaatgaaat a                                                 21


<210>  225
<211>  20
<212>  DNA
<213>  Artificial Sequence

<220>
<223>  PCR primer for BRCA1 and BRCA2 gene regions

<400>  225
gcagataccc aaaaagtggc                                                    20


<210>  226
<211>  20
<212>  DNA
<213>  Artificial Sequence

<220>
<223>  PCR primer for BRCA1 and BRCA2 gene regions

<400>  226
tctggacctc ccaaaaactg                                                    20


<210>  227
<211>  20
<212>  DNA
<213>  Artificial Sequence

<220>
<223>  PCR primer for BRCA1 and BRCA2 gene regions

<400>  227
cactgtgcct ggcctgatac                                                    20


<210>  228
<211>  21
<212>  DNA
<213>  Artificial Sequence

<220>
<223>  PCR primer for BRCA1 and BRCA2 gene regions

<400>  228
atgttaaatt caaagtctct a                                                  21


<210>  229
<211>  24
<212>  DNA
<213>  Artificial Sequence

<220>
<223>  PCR primer for BRCA1 and BRCA2 gene regions

<400>  229
acttcttcca ttgcatcttt ctca                                               24


<210>  230
<211>  24
<212>  DNA
<213>  Artificial Sequence

<220>
<223>  PCR primer for BRCA1 and BRCA2 gene regions

<400>  230
aaaacaaaac aaaaattcaa cata                                              24


<210>  231
<211>  20
<212>  DNA
<213>  Artificial Sequence

<220>
<223>  PCR primer for BRCA1 and BRCA2 gene regions

<400>  231
gcagcgacaa aaaaactca                                                    20


<210>  232
<211>  20
<212>  DNA
<213>  Artificial Sequence

<220>
<223>  PCR primer for BRCA1 and BRCA2 gene regions

<400>  232
atttgccaac tggtagctcc                                                   20


<210>  233
<211>  20
<212>  DNA
<213>  Artificial Sequence

<220>
<223>  PCR primer for BRCA1 and BRCA2 gene regions

<400>  233
gctttcgcca aattcagcta                                                   20


<210>  234
<211>  21
<212>  DNA
<213>  Artificial Sequence

<220>
<223>  PCR primer for BRCA1 and BRCA2 gene regions

<400>  234
taccaaaatg tgtggtgatg c                                                 21


<210>  235
<211>  18
<212>  DNA
<213>  Artificial Sequence

<220>
<223>  PCR primer for CFTR gene regions

<400>  235
cagcactcgg cttttaac                                                     18

<210>  236
<211>  18
<212>  DNA
<213>  Artificial Sequence

<220>
<223>  PCR primer for CFTR gene regions

<400>  236
atacacacgc cctcctct                                                    18


<210>  237
<211>  20
<212>  DNA
<213>  Artificial Sequence

<220>
<223>  PCR primer for CFTR gene regions

<400>  237
tccaaatctg tatggagacc                                                  20


<210>  238
<211>  24
<212>  DNA
<213>  Artificial Sequence

<220>
<223>  PCR primer for CFTR gene regions

<400>  238
tgaatttctc tcttcaacta aaca                                             24


<210>  239
<211>  20
<212>  DNA
<213>  Artificial Sequence

<220>
<223>  PCR primer for CFTR gene regions

<400>  239
caacttattg gtcccacttt                                                  20


<210>  240
<211>  20
<212>  DNA
<213>  Artificial Sequence

<220>
<223>  PCR primer for CFTR gene regions

<400>  240
cacctattca ccagatttcg                                                  20


<210>  241

<211>  20
<212>  DNA
<213>  Artificial Sequence

<220>
<223>  PCR primer for CFTR gene regions

<400>  241
ttgtaggaag tcaccaaagc                                                   20


<210>  242
<211>  23
<212>  DNA
<213>  Artificial Sequence

<220>
<223>  PCR primer for CFTR gene regions

<400>  242
tacgatacag aatatatgtg cca                                               23


<210>  243
<211>  25
<212>  DNA
<213>  Artificial Sequence

<220>
<223>  PCR primer for CFTR gene regions

<400>  243
ttgaaattat ctaactttcc atttt                                             25


<210>  244
<211>  20
<212>  DNA
<213>  Artificial Sequence

<220>
<223>  PCR primer for CFTR gene regions

<400>  244
cgcctttcca gttgtataat                                                   20


<210>  245
<211>  20
<212>  DNA
<213>  Artificial Sequence

<220>
<223>  PCR primer for CFTR gene regions

<400>  245
gctgtgcttt tattttccag                                                   20


<210>  246
<211>  20
<212>  DNA
<213>  Artificial Sequence

<220>
<223>  PCR primer for CFTR gene regions

<400>  246
actaaagtgg gctttttgaa                                                   20


<210>  247
<211>  21
<212>  DNA
<213>  Artificial Sequence

<220>
<223>  PCR primer for CFTR gene regions

<400>  247
cttaaaacct tgagcagttc t                                                 21


<210>  248
<211>  24
<212>  DNA
<213>  Artificial Sequence

<220>
<223>  PCR primer for CFTR gene regions

<400>  248
caatattgaa attattggaa caac                                              24


<210>  249
<211>  20
<212>  DNA
<213>  Artificial Sequence

<220>
<223>  PCR primer for CFTR gene regions

<400>  249
agatcttcca ttccaagatc                                                   20


<210>  250
<211>  20
<212>  DNA
<213>  Artificial Sequence

<220>
<223>  PCR primer for CFTR gene regions

<400>  250
tgcagcatta tggtacatta                                                   20


<210>  251
<211>  26
<212>  DNA
<213>  Artificial Sequence

<220>
<223>  PCR primer for CFTR gene regions

<400>  251
aagatgtagc acaatgagag tataaa                                          26


<210>  252
<211>  25
<212>  DNA
<213>  Artificial Sequence

<220>
<223>  PCR primer for CFTR gene regions

<400>  252
gaaaacagtt aggtgtttag agcaa                                           25


<210>  253
<211>  20
<212>  DNA
<213>  Artificial Sequence

<220>
<223>  PCR primer for CFTR gene regions

<400>  253
tggggaatta tttgagaaag                                                 20


<210>  254
<211>  23
<212>  DNA
<213>  Artificial Sequence

<220>
<223>  PCR primer for CFTR gene regions

<400>  254
cttccagcac tacaaactag aaa                                             23


<210>  255
<211>  20
<212>  DNA
<213>  Artificial Sequence

<220>
<223>  PCR primer for CFTR gene regions

<400>  255
gcgtgatttg ataatgacct                                                 20


<210>  256
<211>  19
<212>  DNA
<213>  Artificial Sequence

<220>
<223>  PCR primer for CFTR gene regions

<400>  256
tgggtagtgt gaagggttc                                                  19

<210>  257
<211>  23
<212>  DNA
<213>  Artificial Sequence

<220>
<223>  PCR primer for CFTR gene regions

<400>  257
agattgagca tactaaaagt gac                                               23


<210>  258
<211>  22
<212>  DNA
<213>  Artificial Sequence

<220>
<223>  PCR primer for CFTR gene regions

<400>  258
tgcttgctag accaataatt ag                                                22


<210>  259
<211>  21
<212>  DNA
<213>  Artificial Sequence

<220>
<223>  PCR primer for CFTR gene regions

<400>  259
ccaggaaata gagaggaaat g                                                 21


<210>  260
<211>  20
<212>  DNA
<213>  Artificial Sequence

<220>
<223>  PCR primer for CFTR gene regions

<400>  260
cataccaaca atggtgaaca                                                   20


<210>  261
<211>  22
<212>  DNA
<213>  Artificial Sequence

<220>
<223>  PCR primer for CFTR gene regions

<400>  261
gctaaaatac gagacatatt gc                                                22


<210>  262

<211>  20
<212>  DNA
<213>  Artificial Sequence

<220>
<223>  PCR primer for CFTR gene regions

<400>  262
attctgtggg gtgaaatacc                                                    20


<210>  263
<211>  19
<212>  DNA
<213>  Artificial Sequence

<220>
<223>  PCR primer for CFTR gene regions

<400>  263
ggtggcatga aactgtact                                                     19


<210>  264
<211>  23
<212>  DNA
<213>  Artificial Sequence

<220>
<223>  PCR primer for CFTR gene regions

<400>  264
atacatcccc aaactatctt aat                                                23


<210>  265
<211>  20
<212>  DNA
<213>  Artificial Sequence

<220>
<223>  PCR primer for CFTR gene regions

<400>  265
atgggaggaa taggtgaaga                                                    20


<210>  266
<211>  23
<212>  DNA
<213>  Artificial Sequence

<220>
<223>  PCR primer for CFTR gene regions

<400>  266
caaagtggat tacaatacat aca                                                23


<210>  267
<211>  20
<212>  DNA
<213>  Artificial Sequence

<220>
<223>  PCR primer for CFTR gene regions

<400>  267
tgccaaataa cgatttccta                                                   20


<210>  268
<211>  20
<212>  DNA
<213>  Artificial Sequence

<220>
<223>  PCR primer for CFTR gene regions

<400>  268
gtggatcagc agtttcattt                                                   20


<210>  269
<211>  21
<212>  DNA
<213>  Artificial Sequence

<220>
<223>  PCR primer for CFTR gene regions

<400>  269
ttgaggaatt tgtcatcttg t                                                 21


<210>  270
<211>  20
<212>  DNA
<213>  Artificial Sequence

<220>
<223>  PCR primer for CFTR gene regions

<400>  270
gacttcaacc ctcaatcaaa                                                   20


<210>  271
<211>  20
<212>  DNA
<213>  Artificial Sequence

<220>
<223>  PCR primer for CFTR gene regions

<400>  271
cactgacaca ctttgtccac                                                   20


<210>  272
<211>  20
<212>  DNA
<213>  Artificial Sequence

<220>
<223>  PCR primer for CFTR gene regions

<400>  272
atcgcacatt cactgtcata                                                   20


<210>  273
<211>  20
<212>  DNA
<213>  Artificial Sequence

<220>
<223>  PCR primer for CFTR gene regions

<400>  273
atttgcaatg ttttctatgg                                                   20


<210>  274
<211>  24
<212>  DNA
<213>  Artificial Sequence

<220>
<223>  PCR primer for CFTR gene regions

<400>  274
tgcttagcta aagttaatga gttc                                              24


<210>  275
<211>  20
<212>  DNA
<213>  Artificial Sequence

<220>
<223>  PCR primer for CFTR gene regions

<400>  275
ttcatttacg tcttttgtgc                                                   20


<210>  276
<211>  22
<212>  DNA
<213>  Artificial Sequence

<220>
<223>  PCR primer for CFTR gene regions

<400>  276
ggtatatagt tcttcctcat gc                                                22


<210>  277
<211>  20
<212>  DNA
<213>  Artificial Sequence

<220>
<223>  PCR primer for CFTR gene regions

<400>  277
gtgaaattgt ctgccattct                                                   20

<210>  278
<211>  20
<212>  DNA
<213>  Artificial Sequence

<220>
<223>  PCR primer for CFTR gene regions

<400>  278
caagcagtgt tcaaatctca                                                    20


<210>  279
<211>  20
<212>  DNA
<213>  Artificial Sequence

<220>
<223>  PCR primer for CFTR gene regions

<400>  279
tgatcccatc acttttacct                                                    20


<210>  280
<211>  20
<212>  DNA
<213>  Artificial Sequence

<220>
<223>  PCR primer for CFTR gene regions

<400>  280
tttctggcta agtccttttg                                                    20


<210>  281
<211>  22
<212>  DNA
<213>  Artificial Sequence

<220>
<223>  PCR primer for CFTR gene regions

<400>  281
agaacttgat ggtaagtaca tg                                                 22


<210>  282
<211>  20
<212>  DNA
<213>  Artificial Sequence

<220>
<223>  PCR primer for CFTR gene regions

<400>  282
catttcagtt agcagcctta                                                    20


<210>  283

<211>  23
<212>  DNA
<213>  Artificial Sequence

<220>
<223>  PCR primer for CFTR gene regions

<400>  283
tctgaactat cttctctaac tgc                                              23


<210>  284
<211>  20
<212>  DNA
<213>  Artificial Sequence

<220>
<223>  PCR primer for CFTR gene regions

<400>  284
aatgattctg ttcccactgt                                                  20


<210>  285
<211>  24
<212>  DNA
<213>  Artificial Sequence

<220>
<223>  PCR primer for CFTR gene regions

<400>  285
ttctgtgata ttatgtgtgg tatt                                             24


<210>  286
<211>  20
<212>  DNA
<213>  Artificial Sequence

<220>
<223>  PCR primer for CFTR gene regions

<400>  286
aagaattaca agggcaatga                                                  20


<210>  287
<211>  21
<212>  DNA
<213>  Artificial Sequence

<220>
<223>  PCR primer for CFTR gene regions

<400>  287
cagatctcac taacagccat t                                                21


<210>  288
<211>  20
<212>  DNA
<213>  Artificial Sequence

<220>
<223>  PCR primer for CFTR gene regions

<400>  288
tgtcaacatt tatgctgctc                                                    20


<210>  289
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  289
nagagaccat gcagaggtcg cctctg                                             26


<210>  290
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  290
ngtcccactt tttattcttt tgcagagaat g                                       31


<210>  291
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  291
ngatgttttt tctggagatt tatgttctat g                                       31


<210>  292

<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  292
ngttctatgg aatcttttta tattta                                          26


<210>  293
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  293
   ntttctctgt ttttcccctt ttgtag                                           26


<210>  294
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  294
   ntttctctgt ttttcccctt ttgtag                                           26


<210>  295
<211>  31
<212>  DNA
<213>  Artificial Sequence

<220>
<223>  probe for CFTR mutations


<220>

<221>  misc_feature
<222>  (1)..(1)
<223>  n is C12-AMINO and dT18 at 5′

<400>  295
nctctcttac tgggaagaat catagcttcc t                                    31


<210>  296
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  296
nactgggaag aatcatagct tcctat                                          26


<210>  297
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  297
ntatgacccg gataacaagg aggaac                                          26


<210>  298
<211>  26
<212>  DNA
<213>  Artificial Sequence

<220>
<223>  nucleotide sequence of matrix probe for detection of mutations in
       CFTR gene


<220>
<221>  misc_feature
<222>  (1)..(1)
<223>  n is C12-AMINO and dT18 at 5′

<400>  298
ngcacatttc gtgtggatcg ctcctt                                          26

<210>  299
<211>  26
<212>  DNA
<213>  Artificial Sequence

<220>
<223>  nucleotide sequence of matrix probe for detection of mutations in
       CFTR gene


<220>
<221>  misc_feature
<222>  (1)..(1)
<223>  n is C12-AMINO and dT18 at 5′

<400>  299
ngcactcctc atggggctaa tctggg                                          26


<210>  300
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  300
ntgtgagata cttcaatagc tcagccttct t                                    31


<210>  301
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  301
nccctatgca ctaatcaaag gaatcatcct c                                    31


<210>  302
<211>  26
<212>  DNA
<213>  Artificial Sequence

<220>
<223>  probe for CFTR mutations

<220>
<221>  misc_feature
<222>  (1)..(1)
<223>  n is C12-AMINO and dT18 at 5′

<400>  302
nggaatcatc ctccggaaaa tattca                                          26


<210>  303
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  303
naccatctca ttctgcattg ttctgc                                          26


<210>  304
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  304
naccatctca ttctgcattg ttctgc                                          26


<210>  305
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  305
naccatctca ttctgcattg ttctgc                                          26

<210>  306
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  306
ntcattctgc attgttctgc gcatgg                                            26


<210>  307
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  307
nattgttctg cgcatggcgg tcactc                                            26


<210>  308
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  308
ncggcaattt ccctgggctg tacaaa                                            26


<210>  309
<211>  31
<212>  DNA
<213>  Artificial Sequence

<220>
<223>  probe for CFTR mutations

<220>
<221>  misc_feature
<222>  (1)..(1)
<223>  n is C12-AMINO and dT18 at 5′

<400>  309
naccttgcct gctccagtgg atccagcaac c                                    31


<210>  310
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  310
ntagaaagag gacagttgtt ggcggttgct g                                    31


<210>  311
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  311
nttatttcca gacttcactt ctaatg                                          26


<210>  312
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  312
ngtgattatg ggagaactgg agccttcaga g                                    31

<210>  313
<211>  31
<212>  DNA
<213>  Artificial Sequence

<220>
<223>  nucleotide sequence of matrix probe for detection of mutations in
       CFTR gene


<220>
<221>  misc_feature
<222>  (1)..(1)
<223>  n is C12-AMINO and dT18 at 5′

<400>  313
nttaagcaca gtggaagaat ttcattctgt t                                    31


<210>  314
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  314
ncagtggaag aatttcattc tgttct                                          26


<210>  315
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  315
ntatgcctgg caccattaaa gaaaat                                          26


<210>  316
<211>  31
<212>  DNA
<213>  Artificial Sequence

<220>
<223>  probe for CFTR mutations

<220>
<221>  misc_feature
<222>  (1)..(1)
<223>  n is C12-AMINO and dT18 at 5′

<400>  316
nattatgcct ggcaccatta aagaaaatat c                                    31


<210>  317
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  317
ntatgcctgg caccattaaa gaaaatatca t                                    31


<210>  318
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  318
nggcaccatt aaagaaaata tcatct                                          26


<210>  319
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  319
nctatgatga atatagatac agaagc                                          26

<210>  320
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  320
natatagata cagaagcgtc atcaaagcat g                                    31


<210>  321
<211>  31
<212>  DNA
<213>  Artificial Sequence

<220>
<223>  probe for CFTR mutations


<220>
<221>  misc_feature
<223>  n is C12-AMINO and dT18 at 5'

<220>
<221>  misc_feature
<222>  (1)..(1)
<223>  n is a, c, g, or t

<400>  321
ngtggaatca cactgagtgg aggtcaacga g                                    31


<210>  322
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  322
ntgcagagaa agacaatata gttctt                                          26


<210>  323
<211>  26
<212>  DNA

<213>  Artificial Sequence

<220>
<223>  probe for CFTR mutations


<220>
<221>  misc_feature
<222>  (1)..(1)
<223>  n is C12-AMINO and dT18 at 5'

<400>  323
ncttggagaa ggtggaatca cactga                                          26


<210>  324
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  324
ncttggagaa ggtggaatca cactga                                          26


<210>  325
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  325
ntcttggaga aggtggaatc acactg                                          26


<210>  326
<211>  26
<212>  DNA
<213>  Artificial Sequence

<220>
<223>  probe for CFTR mutations


<220>
<221>  misc_feature
<222>  (1)..(1)

<223>  n is C12-AMINO and dT18 at 5′

<400>  326
ngaaggtgga atcacactga gtggag                                          26


<210>  327
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  327
ncttggagaa ggtggaatca cactgagtgg a                                    31


<210>  328
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  328
nggagaaggt ggaatcacac tgagtggagg t                                    31


<210>  329
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  329
ntggaatcac actgagtgga ggtcaa                                          26


<210>  330
<211>  31
<212>  DNA

<213>  Artificial Sequence

<220>
<223>  probe for CFTR mutations


<220>
<221>  misc_feature
<222>  (1)..(1)
<223>  n is C12-AMINO and dT18 at 5'

<400>  330
naaggtggaa tcacactgag tggaggtcaa c                                    31


<210>  331
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  331
nactgagtgg aggtcaacga gcaaga                                          26


<210>  332
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  332
naggtcaacg agcaagaatt tcttta                                          26


<210>  333
<211>  26
<212>  DNA
<213>  Artificial Sequence

<220>
<223>  probe for CFTR mutations


<220>
<221>  misc_feature
<222>  (1)..(1)

<223>  n is C12-AMINO and dT18 at 5′

<400>  333
ncaacgagca agaatttctt tagcaa                                            26


<210>  334
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  334
ncaacgagca agaatttctt tagcaa                                            26


<210>  335
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  335
ntgtaattta atttccattt tctttttaga g                                      31


<210>  336
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  336
ntttaatttc cattttcttt ttagagcagt a                                      31


<210>  337
<211>  31
<212>  DNA

<213>  Artificial Sequence

<220>
<223>  probe for CFTR mutations


<220>
<221>  misc_feature
<222>  (1)..(1)
<223>  n is C12-AMINO and dT18 at 5'

<400>  337
naagatgctg atttgtattt attagactct c                                    31


<210>  338
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  338
nttgtattta ttagactctc cttttg                                          26


<210>  339
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  339
ncagacttta gctcaaaact catgggatgt g                                    31


<210>  340
<211>  31
<212>  DNA
<213>  Artificial Sequence

<220>
<223>  probe for CFTR mutations


<220>
<221>  misc_feature
<222>  (1)..(1)

<223>  n is C12-AMINO and dT18 at 5′

<400>  340
ntctattctc aatccaatca actctatacg a                                    31


<210>  341
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  341
naactctata cgaaaatttt ccattgtgca a                                    31


<210>  342
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  342
nggcttggaa ataagtgaag aaattaacga a                                    31


<210>  343
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  343
ntatggagag cataccagca gtgactacat g                                    31


<210>  344
<211>  26
<212>  DNA

<213>  Artificial Sequence

<220>
<223>  probe for CFTR mutations


<220>
<221>  misc_feature
<222>  (1)..(1)
<223>  n is C12-AMINO and dT18 at 5'

<400>  344
nagtgactac atggaacaca tacctt                                          26


<210>  345
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  345
natttcctat ttgctttaca gcactcctct t                                    31


<210>  346
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  346
ngcagtgatt atcaccagca ccagtt                                          26


<210>  347
<211>  26
<212>  DNA
<213>  Artificial Sequence

<220>
<223>  probe for CFTR mutations


<220>
<221>  misc_feature
<222>  (1)..(1)

<223>  n is C12-AMINO and dT18 at 5′

<400>  347
ngtgattatc accagcacca gttcgt                                            26


<210>  348
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  348
ncatactcta atcacagtgt cgaaaatttt a                                      31


<210>  349
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  349
nttgttacaa gcttaaaagg actatggaca c                                      31


<210>  350
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  350
nttacaagct taaaaggact atggacactt c                                      31


<210>  351
<211>  26
<212>  DNA

<213>  Artificial Sequence

<220>
<223>  probe for CFTR mutations


<220>
<221>  misc_feature
<222>  (1)..(1)
<223>  n is C12-AMINO and dT18 at 5′

<400>  351
naagcttaaa aggactatgg acactt                                          26


<210>  352
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  352
nacaagctta aaaggactat ggacacttcg t                                    31


<210>  353
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  353
ngactatgga cacttcgtgc cttcggacgg c                                    31


<210>  354
<211>  31
<212>  DNA
<213>  Artificial Sequence

<220>
<223>  probe for CFTR mutations


<220>
<221>  misc_feature
<222>  (1)..(1)

<223>  n is C12-AMINO and dT18 at 5′

<400>  354
naaactctgt tccacaaagc tctgaattta c                                    31


<210>  355
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  355
ngctctgaat ttacatactg ccaact                                          26


<210>  356
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  356
ntacatactg ccaactggtt cttgta                                          26


<210>  357
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  357
nctgtcaaca ctgcgctggt tccaaa                                          26


<210>  358
<211>  26
<212>  DNA

<213>  Artificial Sequence

<220>
<223>  probe for CFTR mutations


<220>
<221>  misc_feature
<222>  (1)..(1)
<223>  n is C12-AMINO and dT18 at 5'

<400>  358
naatgttgtt atttttattt cagatg                                          26


<210>  359
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  359
nctgttggca tgtcaatgaa cttaaagact c                                    31


<210>  360
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  360
ncacacgtga agaaagatga catctg                                          26


<210>  361
<211>  26
<212>  DNA
<213>  Artificial Sequence

<220>
<223>  probe for CFTR mutations


<220>
<221>  misc_feature
<222>  (1)..(1)

<223>  n is C12-AMINO and dT18 at 5′

<400>  361
ngtcaaagat ctcacagcaa aataca                                          26


<210>  362
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  362
ncatattaga gaacatttcc ttctca                                          26


<210>  363
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  363
ncatttcctt ctcaataagt cctggc                                          26


<210>  364
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  364
ngcagtgatt atcaccagca ccagtt                                          26


<210>  365
<211>  31
<212>  DNA

<213>  Artificial Sequence

<220>
<223>  probe for CFTR mutations


<220>
<221>  misc_feature
<222>  (1)..(1)
<223>  n is C12-AMINO and dT18 at 5′

<400>  365
naggtgggcc tcttgggaag aactggatca g                                    31


<210>  366
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  366
nttgggaaga actggatcag ggaaga                                          26


<210>  367
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  367
nggatcaggg aagagtactt tgttat                                          26


<210>  368
<211>  31
<212>  DNA
<213>  Artificial Sequence

<220>
<223>  probe for CFTR mutations


<220>
<221>  misc_feature
<222>  (1)..(1)

<223>  n is C12-AMINO and dT18 at 5'

<400>  368
nagaactgga tcagggaaga gtactttgtt a                                    31


<210>  369
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  369
ngaacactga aggagaaatc cagatc                                          26


<210>  370
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  370
ngggattcaa taactttgca acagtg                                          26


<210>  371
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  371
ngattcaata actttgcaac agtgga                                          26


<210>  372
<211>  31
<212>  DNA

<213>  Artificial Sequence

<220>
<223>  probe for CFTR mutations


<220>
<221>  misc_feature
<222>  (1)..(1)
<223>  n is C12-AMINO and dT18 at 5′

<400>  372
ncaataactt tgcaacagtg gaggaaagcc t                                    31


<210>  373
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  373
nggaaagcct ttggagtgat accaca                                          26


<210>  374
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  374
ntttttctg gaacatttag aaaaaa                                           26


<210>  375
<211>  26
<212>  DNA
<213>  Artificial Sequence

<220>
<223>  probe for CFTR mutations


<220>
<221>  misc_feature
<222>  (1)..(1)

<223>  n is C12-AMINO and dT18 at 5'

<400>  375
ntttttctg gaacatttag aaaaaa                                          26


<210>  376
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  376
nggatcccta tgaacagtgg agtgat                                          26


<210>  377
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  377
ncctatgaac agtggagtga tcaagaaata t                                    31


<210>  378
<211>  26
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  378
ngtgtcctaa gccatggcca caagca                                          26


<210>  379
<211>  31
<212>  DNA

<213>  Artificial Sequence

<220>
<223>  probe for CFTR mutations


<220>
<221>  misc_feature
<222>  (1)..(1)
<223>  n is C12-AMINO and dT18 at 5'

<400>  379
nttctggaga tttatgttct atggaatctt t                                    31


<210>  380
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  380
nattgtgagg acactgctcc tacacccagc c                                    31


<210>  381
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  381
ngaggacact gctcctacac ccagccattt t                                    31


<210>  382
<211>  31
<212>  DNA
<213>  Artificial Sequence

<220>
<223>  probe for CFTR mutations


<220>
<221>  misc_feature
<222>  (1)..(1)

<223>  n is C12-AMINO and dT18 at 5′

<400>  382
ncttgtgatt acctcagaaa tgattgaaaa t                                    31


<210>  383
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  383
nggaagaagc aatggaaaaa atgattgaaa a                                    31


<210>  384
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  384
ntagctcagc cttcttcttc tcagggttct t                                    31


<210>  385
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  385
ncatcctccg gaaaatattc accaccatct c                                    31


<210>  386
<211>  31
<212>  DNA

<213>  Artificial Sequence

<220>
<223>  probe for CFTR mutations


<220>
<221>  misc_feature
<222>  (1)..(1)
<223>  n is C12-AMINO and dT18 at 5'

<400>  386
nactcggcaa tttccctggg ctgtacaaac a                                    31


<210>  387
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  387
ntcggcaatt tccctgggct gtacaaacat g                                    31


<210>  388
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  388
nccctgggct gtacaaacat ggtatgactc t                                    31


<210>  389
<211>  31
<212>  DNA
<213>  Artificial Sequence

<220>
<223>  probe for CFTR mutations


<220>
<221>  misc_feature
<222>  (1)..(1)

<223>  n is C12-AMINO and dT18 at 5′

<400>  389
naagcacagt ggaagaattt cattctgttc t                                    31


<210>  390
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  390
ntatcatctt tggtgtttcc tatgatgaat a                                    31


<210>  391
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  391
naagactccc ttacaaatga atggcatcga a                                    31


<210>  392
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  392
ntgatcagca ctggccccac gcttcaggca c                                    31


<210>  393
<211>  31
<212>  DNA

<213>  Artificial Sequence

<220>
<223>  probe for CFTR mutations


<220>
<221>  misc_feature
<222>  (1)..(1)
<223>  n is C12-AMINO and dT18 at 5'

<400>  393
nctcaggcaa acttgactga actggatata t                                    31


<210>  394
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  394
natgcagtga ttatcaccag caccagttcg t                                    31


<210>  395
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  395
ntcatcttgt tacaagctta aaaggactat g                                    31


<210>  396
<211>  31
<212>  DNA
<213>  Artificial Sequence

<220>
<223>  probe for CFTR mutations


<220>
<221>  misc_feature
<222>  (1)..(1)

<223>  n is C12-AMINO and dT18 at 5′

<400>  396
natttcacag gcaggagtcc aattttcact c                                    31


<210>  397
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  397
ntttcagatg cgatctgtga gccgagtctt t                                    31


<210>  398
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  398
ntgactgtca aagatctcac agcaaaatac a                                    31


<210>  399
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5′

<400>  399
ntgccaacag aaggtaaacc taccaagtca a                                    31


<210>  400
<211>  31
<212>  DNA

<213>  Artificial Sequence

<220>
<223>  probe for CFTR mutations


<220>
<221>  misc_feature
<222>  (1)..(1)
<223>  n is C12-AMINO and dT18 at 5'

<400>  400
nataggtggg cctcttggga agaactggat c                                    31


<210>  401
<211>  31
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  401
ntggatcagg gaagagtact ttgttatcag c                                    31


<210>  402
<211>  27
<212>  DNA
<213>  Artificial Sequence

<220>
<223>  probe for CFTR mutations


<220>
<221>  misc_feature
<222>  (1)..(1)
<223>  n is C12-AMINO and dT18 at 5'

<400>  402
nacgtacgta cgtacgtacg tacgtcy                                         27


<210>  403
<211>  18
<212>  DNA
<213>  Artificial Sequence

<220>
<223>  starter for multiplex amplification of CFTR gene fragments

<400>  403
cagcactcgg cttttaac                                                   18

<210>  404
<211>  18
<212>  DNA
<213>  Artificial Sequence

<220>
<223>  starter for multiplex amplification of CFTR gene fragments

<400>  404
atacacacgc cctcctct                                                    18


<210>  405
<211>  20
<212>  DNA
<213>  Artificial Sequence

<220>
<223>  starter for multiplex amplification of CFTR gene fragments

<400>  405
tccaaatctg tatggagacc                                                  20


<210>  406
<211>  24
<212>  DNA
<213>  Artificial Sequence

<220>
<223>  starter for multiplex amplification of CFTR gene fragments

<400>  406
tgaatttctc tcttcaacta aaca                                             24


<210>  407
<211>  21
<212>  DNA
<213>  Artificial Sequence

<220>
<223>  starter for multiplex amplification of CFTR gene fragments

<400>  407
caacttattg gtcccacttt c                                                21


<210>  408
<211>  20
<212>  DNA
<213>  Artificial Sequence

<220>
<223>  starter for multiplex amplification of CFTR gene fragments

<400>  408
cacctattca ccagatttcg                                                  20


<210>  409
<211>  20
<212>  DNA

<213>  Artificial Sequence

<220>
<223>  starter for multiplex amplification of CFTR gene fragments

<400>  409
ttgtaggaag tcaccaaagc                                                   20


<210>  410
<211>  23
<212>  DNA
<213>  Artificial Sequence

<220>
<223>  starter for multiplex amplification of CFTR gene fragments

<400>  410
tacgatacag aatatatgtg cca                                               23


<210>  411
<211>  25
<212>  DNA
<213>  Artificial Sequence

<220>
<223>  starter for multiplex amplification of CFTR gene fragments

<400>  411
ttgaaattat ctaactttcc atttt                                             25


<210>  412
<211>  20
<212>  DNA
<213>  Artificial Sequence

<220>
<223>  starter for multiplex amplification of CFTR gene fragments

<400>  412
cgcctttcca gttgtataat                                                   20


<210>  413
<211>  21
<212>  DNA
<213>  Artificial Sequence

<220>
<223>  starter for multiplex amplification of CFTR gene fragments

<400>  413
gctgtgcttt tattttccag b                                                 21


<210>  414
<211>  20
<212>  DNA
<213>  Artificial Sequence

<220>

<223>  starter for multiplex amplification of CFTR gene fragments

<400>  414
actaaagtgg gctttttgaa                                                    20


<210>  415
<211>  21
<212>  DNA
<213>  Artificial Sequence

<220>
<223>  starter for multiplex amplification of CFTR gene fragments

<400>  415
cttaaaacct tgagcagttc t                                                  21


<210>  416
<211>  24
<212>  DNA
<213>  Artificial Sequence

<220>
<223>  starter for multiplex amplification of CFTR gene fragments

<400>  416
caatattgaa attattggaa caac                                               24


<210>  417
<211>  20
<212>  DNA
<213>  Artificial Sequence

<220>
<223>  starter for multiplex amplification of CFTR gene fragments

<400>  417
agatcttcca ttccaagatc                                                    20


<210>  418
<211>  20
<212>  DNA
<213>  Artificial Sequence

<220>
<223>  starter for multiplex amplification of CFTR gene fragments

<400>  418
tgcagcatta tggtacatta                                                    20


<210>  419
<211>  26
<212>  DNA
<213>  Artificial Sequence

<220>
<223>  starter for multiplex amplification of CFTR gene fragments

<400>  419

aagatgtagc acaatgagag tataaa 26

<210> 420
<211> 25
<212> DNA
<213> Artificial Sequence

<220>
<223> starter for multiplex amplification of CFTR gene fragments

<400> 420
gaaaacagtt aggtgtttag agcaa 25

<210> 421
<211> 20
<212> DNA
<213> Artificial Sequence

<220>
<223> starter for multiplex amplification of CFTR gene fragments

<400> 421
tggggaatta tttgagaaag 20

<210> 422
<211> 23
<212> DNA
<213> Artificial Sequence

<220>
<223> starter for multiplex amplification of CFTR gene fragments

<400> 422
cttccagcac tacaaactag aaa 23

<210> 423
<211> 20
<212> DNA
<213> Artificial Sequence

<220>
<223> starter for multiplex amplification of CFTR gene fragments

<400> 423
gcgtgatttg ataatgacct 20

<210> 424
<211> 19
<212> DNA
<213> Artificial Sequence

<220>
<223> starter for multiplex amplification of CFTR gene fragments

<400> 424
tgggtagtgt gaagggttc 19

<210>  425
<211>  23
<212>  DNA
<213>  Artificial Sequence

<220>
<223>  starter for multiplex amplification of CFTR gene fragments

<400>  425
agattgagca tactaaaagt gac                                               23


<210>  426
<211>  22
<212>  DNA
<213>  Artificial Sequence

<220>
<223>  starter for multiplex amplification of CFTR gene fragments

<400>  426
tgcttgctag accaataatt ag                                                22


<210>  427
<211>  21
<212>  DNA
<213>  Artificial Sequence

<220>
<223>  starter for multiplex amplification of CFTR gene fragments

<400>  427
ccaggaaata gagaggaaat g                                                 21


<210>  428
<211>  20
<212>  DNA
<213>  Artificial Sequence

<220>
<223>  starter for multiplex amplification of CFTR gene fragments

<400>  428
cataccaaca atggtgaaca                                                   20


<210>  429
<211>  22
<212>  DNA
<213>  Artificial Sequence

<220>
<223>  starter for multiplex amplification of CFTR gene fragments

<400>  429
gctaaaatac gagacatatt gc                                                22


<210>  430
<211>  20
<212>  DNA

<213>  Artificial Sequence

<220>
<223>  starter for multiplex amplification of CFTR gene fragments

<400>  430
attctgtggg gtgaaatacc                                                   20


<210>  431
<211>  20
<212>  DNA
<213>  Artificial Sequence

<220>
<223>  starter for multiplex amplification of CFTR gene fragments

<400>  431
ggtggcatga aactgtactc                                                   20


<210>  432
<211>  23
<212>  DNA
<213>  Artificial Sequence

<220>
<223>  starter for multiplex amplification of CFTR gene fragments

<400>  432
atacatcccc aaactatctt aat                                               23


<210>  433
<211>  20
<212>  DNA
<213>  Artificial Sequence

<220>
<223>  starter for multiplex amplification of CFTR gene fragments

<400>  433
atgggaggaa taggtgaaga                                                   20


<210>  434
<211>  23
<212>  DNA
<213>  Artificial Sequence

<220>
<223>  starter for multiplex amplification of CFTR gene fragments

<400>  434
caaagtggat tacaatacat aca                                               23


<210>  435
<211>  20
<212>  DNA
<213>  Artificial Sequence

<220>

<223>  starter for multiplex amplification of CFTR gene fragments

<400>  435
tgccaaataa cgatttccta                                                   20


<210>  436
<211>  20
<212>  DNA
<213>  Artificial Sequence

<220>
<223>  starter for multiplex amplification of CFTR gene fragments

<400>  436
gtggatcagc agtttcattt                                                   20


<210>  437
<211>  21
<212>  DNA
<213>  Artificial Sequence

<220>
<223>  starter for multiplex amplification of CFTR gene fragments

<400>  437
ttgaggaatt tgtcatcttg t                                                 21


<210>  438
<211>  20
<212>  DNA
<213>  Artificial Sequence

<220>
<223>  starter for multiplex amplification of CFTR gene fragments

<400>  438
gacttcaacc ctcaatcaaa                                                   20


<210>  439
<211>  20
<212>  DNA
<213>  Artificial Sequence

<220>
<223>  starter for multiplex amplification of CFTR gene fragments

<400>  439
cactgacaca ctttgtccac                                                   20


<210>  440
<211>  20
<212>  DNA
<213>  Artificial Sequence

<220>
<223>  starter for multiplex amplification of CFTR gene fragments

<400>  440

atcgcacatt cactgtcata                                                    20

<210>  441
<211>  20
<212>  DNA
<213>  Artificial Sequence

<220>
<223>  starter for multiplex amplification of CFTR gene fragments

<400>  441
atttgcaatg ttttctatgg                                                    20

<210>  442
<211>  24
<212>  DNA
<213>  Artificial Sequence

<220>
<223>  starter for multiplex amplification of CFTR gene fragments

<400>  442
tgcttagcta aagttaatga gttc                                               24

<210>  443
<211>  21
<212>  DNA
<213>  Artificial Sequence

<220>
<223>  starter for multiplex amplification of CFTR gene fragments

<400>  443
ttcatttacg tcttttgtgc a                                                  21

<210>  444
<211>  22
<212>  DNA
<213>  Artificial Sequence

<220>
<223>  starter for multiplex amplification of CFTR gene fragments

<400>  444
ggtatatagt tcttcctcat gc                                                 22

<210>  445
<211>  20
<212>  DNA
<213>  Artificial Sequence

<220>
<223>  starter for multiplex amplification of CFTR gene fragments

<400>  445
gtgaaattgt ctgccattct                                                    20

<210>  446
<211>  20
<212>  DNA
<213>  Artificial Sequence

<220>
<223>  starter for multiplex amplification of CFTR gene fragments

<400>  446
caagcagtgt tcaaatctca                                                    20


<210>  447
<211>  21
<212>  DNA
<213>  Artificial Sequence

<220>
<223>  starter for multiplex amplification of CFTR gene fragments

<400>  447
tgatcccatc acttttacct c                                                  21


<210>  448
<211>  20
<212>  DNA
<213>  Artificial Sequence

<220>
<223>  starter for multiplex amplification of CFTR gene fragments

<400>  448
tttctggcta agtccttttg                                                    20


<210>  449
<211>  22
<212>  DNA
<213>  Artificial Sequence

<220>
<223>  starter for multiplex amplification of CFTR gene fragments

<400>  449
agaacttgat ggtaagtaca tg                                                 22


<210>  450
<211>  20
<212>  DNA
<213>  Artificial Sequence

<220>
<223>  starter for multiplex amplification of CFTR gene fragments

<400>  450
catttcagtt agcagcctta                                                    20


<210>  451
<211>  23
<212>  DNA

<213>  Artificial Sequence

<220>
<223>  starter for multiplex amplification of CFTR gene fragments

<400>  451
tctgaactat cttctctaac tgc                                               23


<210>  452
<211>  20
<212>  DNA
<213>  Artificial Sequence

<220>
<223>  starter for multiplex amplification of CFTR gene fragments

<400>  452
aatgattctg ttcccactgt                                                   20


<210>  453
<211>  24
<212>  DNA
<213>  Artificial Sequence

<220>
<223>  starter for multiplex amplification of CFTR gene fragments

<400>  453
ttctgtgata ttatgtgtgg tatt                                              24


<210>  454
<211>  20
<212>  DNA
<213>  Artificial Sequence

<220>
<223>  starter for multiplex amplification of CFTR gene fragments

<400>  454
aagaattaca agggcaatga                                                   20


<210>  455
<211>  21
<212>  DNA
<213>  Artificial Sequence

<220>
<223>  starter for multiplex amplification of CFTR gene fragments

<400>  455
cagatctcac taacagccat t                                                 21


<210>  456
<211>  20
<212>  DNA
<213>  Artificial Sequence

<220>

<223>  starter for multiplex amplification of CFTR gene fragments

<400>  456
tgtcaacatt tatgctgctc                                                    20


<210>  457
<211>  22
<212>  DNA
<213>  Artificial Sequence

<220>
<223>  nucleotide sequence of matrix probe for detecting mutations in
       BRCA1 gene


<220>
<221>  misc_feature
<222>  (1)..(1)
<223>  n is C12-AMINO and dT18 at 5′

<400>  457
ngactcccag cacagaaaaa aa                                                 22


<210>  458
<211>  29
<212>  DNA
<213>  Artificial Sequence

<220>
<223>  nucleotide sequence of matrix probe for detecting mutations in
       BRCA1 gene


<220>
<221>  misc_feature
<222>  (1)..(1)
<223>  n is C12-AMINO and dT18 at 5′

<400>  458
nattctggtt cttcagagaa aatagactt                                          29


<210>  459
<211>  35
<212>  DNA
<213>  Artificial Sequence

<220>
<223>  nucleotide sequence of matrix probe for detecting mutations in
       BRCA1 gene


<220>
<221>  misc_feature
<222>  (1)..(1)
<223>  n is C12-AMINO and dT18 at 5′

<400>  459
ncaaatcagt agagagtaat attgaagaca aaata                                   35

<210>  460
<211>  25
<212>  DNA
<213>  Artificial Sequence

<220>
<223>  nucleotide sequence of matrix probe for detecting mutations in
       BRCA1 gene


<220>
<221>  misc_feature
<222>  (1)..(1)
<223>  n is C12-AMINO and dT18 at 5'

<400>  460
ngccttcatc ctgaggattt tatca                                          25


<210>  461
<211>  31
<212>  DNA
<213>  Artificial Sequence

<220>
<223>  nucleotide sequence of matrix probe for detecting mutations in
       BRCA1 gene


<220>
<221>  misc_feature
<222>  (1)..(1)
<223>  n is C12-AMINO and dT18 at 5'

<400>  461
ncatgagaat aaaacaaaag gtgattctat t                                   31


<210>  462
<211>  22
<212>  DNA
<213>  Artificial Sequence

<220>
<223>  nucleotide sequence of matrix probe for detecting mutations in
       BRCA1 gene


<220>
<221>  misc_feature
<222>  (1)..(1)
<223>  n is C12-AMINO and dT18 at 5'

<400>  462
ncagaagaaa gggccttcac ag                                             22


<210>  463
<211>  32
<212>  DNA
<213>  Artificial Sequence

<220>

<223>  nucleotide sequence of matrix probe for detecting mutations in
       BRCA1 gene


<220>
<221>  misc_feature
<222>  (1)..(1)
<223>  n is C12-AMINO and dT18 at 5'

<400>  463
ngtgtccttt atgtaagaat gatataacca aa                                    32


<210>  464
<211>  33
<212>  DNA
<213>  Artificial Sequence

<220>
<223>  nucleotide sequence of matrix probe for detecting mutations in
       BRCA1 gene


<220>
<221>  misc_feature
<222>  (1)..(1)
<223>  n is C12-AMINO and dT18 at 5'

<400>  464
ncagcattat tagacacttt aactgtttct agt                                   33


<210>  465
<211>  25
<212>  DNA
<213>  Artificial Sequence

<220>
<223>  nucleotide sequence of matrix probe for detecting mutations in
       BRCA1 gene


<220>
<221>  misc_feature
<222>  (1)..(1)
<223>  n is C12-AMINO and dT18 at 5'

<400>  465
ngtacctggt actgattatg gcact                                            25


<210>  466
<211>  26
<212>  DNA
<213>  Artificial Sequence

<220>
<223>  nucleotide sequence of matrix probe for detecting mutations in
       BRCA1 gene


<220>
<221>  misc_feature

<222>  (1)..(1)
<223>  n is C12-AMINO and dT18 at 5'

<400>  466
nccaatggat acttaaagcc ttctgt                                          26


<210>  467
<211>  24
<212>  DNA
<213>  Artificial Sequence

<220>
<223>  nucleotide sequence of matrix probe for detecting mutations in
       BRCA1 gene


<220>
<221>  misc_feature
<222>  (1)..(1)
<223>  n is C12-AMINO and dT18 at 5'

<400>  467
ncactctggg tccttaaaga aaca                                            24


<210>  468
<211>  26
<212>  DNA
<213>  Artificial Sequence

<220>
<223>  nucleotide sequence of matrix probe for detecting mutations in
       BRCA1 gene


<220>
<221>  misc_feature
<222>  (1)..(1)
<223>  n is C12-AMINO and dT18 at 5'

<400>  468
ncacttttga atgtgaacaa aaggaa                                          26


<210>  469
<211>  26
<212>  DNA
<213>  Artificial Sequence

<220>
<223>  nucleotide sequence of matrix probe for detecting mutations in
       BRCA1 gene


<220>
<221>  misc_feature
<222>  (1)..(1)
<223>  n is C12-AMINO and dT18 at 5'

<400>  469
ncacttttga atgtgaacaa aaggaa                                          26

<210>  470
<211>  27
<212>  DNA
<213>  Artificial Sequence

<220>
<223>  nucleotide sequence of matrix probe for detecting mutations in
       BRCA1 gene


<220>
<221>  misc_feature
<222>  (1)..(1)
<223>  n is C12-AMINO and dT18 at 5′

<400>  470
ngaaagagaa atgggaaatg agaacat                                          27


<210>  471
<211>  28
<212>  DNA
<213>  Artificial Sequence

<220>
<223>  nucleotide sequence of matrix probe for detecting mutations in
       BRCA1 gene


<220>
<221>  misc_feature
<222>  (1)..(1)
<223>  n is C12-AMINO and dT18 at 5′

<400>  471
ncattcaagc agaactaggt agaaacag                                         28


<210>  472
<211>  29
<212>  DNA
<213>  Artificial Sequence

<220>
<223>  nucleotide sequence of matrix probe for detecting mutations in
       BRCA1 gene


<220>
<221>  misc_feature
<222>  (1)..(1)
<223>  n is C12-AMINO and dT18 at 5′

<400>  472
nctgttagat gatggtgaaa taaaggaag                                        29


<210>  473
<211>  20
<212>  DNA
<213>  Artificial Sequence

<220>
<223>  nucleotide sequence of matrix probe for detecting mutations in
      BRCA1 gene


<220>
<221>  misc_feature
<222>  (1)..(1)
<223>  n is C12-AMINO and dT18 at 5'

<400>  473
ncgaagaggg gccaagaaat                                                   20


<210>  474
<211>  27
<212>  DNA
<213>  Artificial Sequence

<220>
<223>  nucleotide sequence of matrix probe for detecting mutations in
      BRCA1 gene


<220>
<221>  misc_feature
<222>  (1)..(1)
<223>  n is C12-AMINO and dT18 at 5'

<400>  474
ngcttccaac acttgttatt tggtaaa                                           27


<210>  475
<211>  25
<212>  DNA
<213>  Artificial Sequence

<220>
<223>  nucleotide sequence of matrix probe for detecting mutations in
      BRCA1 gene


<220>
<221>  misc_feature
<222>  (1)..(1)
<223>  n is C12-AMINO and dT18 at 5'

<400>  475
ncaaggaatt ggtttcagat gatga                                             25


<210>  476
<211>  33
<212>  DNA
<213>  Artificial Sequence

<220>
<223>  nucleotide sequence of matrix probe for detecting mutations in
      BRCA1 gene


<220>

<221>  misc_feature
<222>  (1)..(1)
<223>  n is C12-AMINO and dT18 at 5'

<400>  476
nttttacatc tgaacctctg tttttgttat tta                                    33


<210>  477
<211>  20
<212>  DNA
<213>  Artificial Sequence

<220>
<223>  nucleotide sequence of matrix probe for detecting mutations in
      BRCA1 gene


<220>
<221>  misc_feature
<222>  (1)..(1)
<223>  n is C12-AMINO and dT18 at 5'

<400>  477
nttctgccct tgaggacctg                                                   20


<210>  478
<211>  24
<212>  DNA
<213>  Artificial Sequence

<220>
<223>  nucleotide sequence of matrix probe for detecting mutations in
      BRCA1 gene


<220>
<221>  misc_feature
<222>  (1)..(1)
<223>  n is C12-AMINO and dT18 at 5'

<400>  478
naaccagtct cagtgtccaa ctct                                              24


<210>  479
<211>  29
<212>  DNA
<213>  Artificial Sequence

<220>
<223>  nucleotide sequence of matrix probe for detecting mutations in
      BRCA1 gene


<220>
<221>  misc_feature
<222>  (1)..(1)
<223>  n is C12-AMINO and dT18 at 5'

<400>  479
nggagataca tatggataca ctcacaaat                                         29

<210>  480
<211>  29
<212>  DNA
<213>  Artificial Sequence

<220>
<223>  nucleotide sequence of matrix probe for detecting mutations in
       BRCA1 gene


<220>
<221>  misc_feature
<222>  (1)..(1)
<223>  n is C12-AMINO and dT18 at 5′

<400>  480
ncggacactg aaatattttc taggaattg                                         29


<210>  481
<211>  22
<212>  DNA
<213>  Artificial Sequence

<220>
<223>  nucleotide sequence of matrix probe for detecting mutations in
       BRCA1 gene


<220>
<221>  misc_feature
<222>  (1)..(1)
<223>  n is C12-AMINO and dT18 at 5′

<400>  481
nagaaaccac caaggtccaa ag                                                22


<210>  482
<211>  21
<212>  DNA
<213>  Artificial Sequence

<220>
<223>  nucleotide sequence of matrix probe for detecting mutations in
       BRCA1 gene


<220>
<221>  misc_feature
<222>  (1)..(1)
<223>  n is C12-AMINO and dT18 at 5′

<400>  482
naaagcgagc aagagaatcc c                                                 21


<210>  483
<211>  23
<212>  DNA
<213>  Artificial Sequence

<220>
<223>  nucleotide sequence of matrix probe for detecting mutations in
      BRCA1 gene


<220>
<221>  misc_feature
<222>  (1)..(1)
<223>  n is C12-AMINO and dT18 at 5'

<400>  483
ncaacttgag ggagggagct tta                                              23


<210>  484
<211>  24
<212>  DNA
<213>  Artificial Sequence

<220>
<223>  nucleotide sequence of matrix probe for detecting mutations in
      BRCA1 gene


<220>
<221>  misc_feature
<222>  (1)..(1)
<223>  n is C12-AMINO and dT18 at 5'

<400>  484
ntgacttacc agatgggaca ctct                                             24


<210>  485
<211>  23
<212>  DNA
<213>  Artificial Sequence

<220>
<223>  nucleotide sequence of matrix probe for detecting mutations in
      BRCA1 gene


<220>
<221>  misc_feature
<222>  (1)..(1)
<223>  n is C12-AMINO and dT18 at 5'

<400>  485
nctgacttac cagatgggac act                                              23


<210>  486
<211>  22
<212>  DNA
<213>  Artificial Sequence

<220>
<223>  nucleotide sequence of matrix probe for detecting mutations in
      BRCA1 gene

<220>
<221>  misc_feature
<222>  (1)..(1)
<223>  n is C12-AMINO and dT18 at 5'

<400>  486
ngctgactta ccagatggga ca                                              22


<210>  487
<211>  28
<212>  DNA
<213>  Artificial Sequence

<220>
<223>  nucleotide sequence of matrix probe for detecting mutations in
       BRCA2 gene


<220>
<221>  misc_feature
<222>  (1)..(1)
<223>  n is C12-AMINO and dT18 at 5'

<400>  487
nggttttata tggagacaca ggtgataa                                        28


<210>  488
<211>  32
<212>  DNA
<213>  Artificial Sequence

<220>
<223>  nucleotide sequence of matrix probe for detecting mutations in
       BRCA2 gene


<220>
<221>  misc_feature
<222>  (1)..(1)
<223>  n is C12-AMINO and dT18 at 5'

<400>  488
ngtttaagat agaaaatcat aatgataaaa ct                                   32


<210>  489
<211>  32
<212>  DNA
<213>  Artificial Sequence

<220>
<223>  nucleotide sequence of matrix probe for detecting mutations in
       BRCA2 gene


<220>
<221>  misc_feature
<222>  (1)..(1)
<223>  n is C12-AMINO and dT18 at 5'

<400>  489

ncagtattga agaatgttga agatcaaaaa aa                                    32


<210>  490
<211>  23
<212>  DNA
<213>  Artificial Sequence

<220>
<223>  nucleotide sequence of matrix probe for detecting mutations in
       BRCA2 gene


<220>
<221>  misc_feature
<222>  (1)..(1)
<223>  n is C12-AMINO and dT18 at 5′

<400>  490
ngtgggattt ttagcacagc aag                                              23


<210>  491
<211>  29
<212>  DNA
<213>  Artificial Sequence

<220>
<223>  nucleotide sequence of matrix probe for detecting mutations in
       BRCA2 gene


<220>
<221>  misc_feature
<222>  (1)..(1)
<223>  n is C12-AMINO and dT18 at 5′

<400>  491
ntttgattta atcagaactg agcatagtc                                        29


<210>  492
<211>  30
<212>  DNA
<213>  Artificial Sequence

<220>
<223>  nucleotide sequence of matrix probe for detecting mutations in
       BRCA2 gene


<220>
<221>  misc_feature
<222>  (1)..(1)
<223>  n is C12-AMINO and dT18 at 5′

<400>  492
ncactctatt aaagtttctc catatctctc                                       30


<210>  493
<211>  28
<212>  DNA

<213>  Artificial Sequence

<220>
<223>  nucleotide sequence of matrix probe for detecting mutations in
       BRCA2 gene


<220>
<221>  misc_feature
<222>  (1)..(1)
<223>  n is C12-AMINO and dT18 at 5′

<400>  493
ncagaatgcc agagatatac aggatatg                                          28


<210>  494
<211>  26
<212>  DNA
<213>  Artificial Sequence

<220>
<223>  nucleotide sequence of matrix probe for detecting mutations in
       BRCA2 gene


<220>
<221>  misc_feature
<222>  (1)..(1)
<223>  n is C12-AMINO and dT18 at 5′

<400>  494
nctgtgacag acagtgaaaa cacaaa                                            26


<210>  495
<211>  23
<212>  DNA
<213>  Artificial Sequence

<220>
<223>  nucleotide sequence of matrix probe for detecting mutations in
       BRCA2 gene


<220>
<221>  misc_feature
<222>  (1)..(1)
<223>  n is C12-AMINO and dT18 at 5′

<400>  495
ngaagcccca gaatctctta tgt                                               23


<210>  496
<211>  29
<212>  DNA
<213>  Artificial Sequence

<220>
<223>  nucleotide sequence of matrix probe for detecting mutations in
       BRCA2 gene

<220>
<221>  misc_feature
<222>  (1)..(1)
<223>  n is C12-AMINO and dT18 at 5'

<400>  496
nggattatac atatttcgca atgaaagag                                         29


<210>  497
<211>  31
<212>  DNA
<213>  Artificial Sequence

<220>
<223>  nucleotide sequence of matrix probe for detecting mutations in
      BRCA2 gene


<220>
<221>  misc_feature
<222>  (1)..(1)
<223>  n is C12-AMINO and dT18 at 5'

<400>  497
ntgttaacag aaggaaagag atacagaatt t                                      31


<210>  498
<211>  29
<212>  DNA
<213>  Artificial Sequence

<220>
<223>  nucleotide sequence of matrix probe for detecting mutations in
      BRCA2 gene


<220>
<221>  misc_feature
<222>  (1)..(1)
<223>  n is C12-AMINO and dT18 at 5'

<400>  498
naaaaaaaac tcagtatcaa caactaccg                                         29


<210>  499
<211>  31
<212>  DNA
<213>  Artificial Sequence

<220>
<223>  nucleotide sequence of matrix probe for detecting mutations in
      BRCA2 gene


<220>
<221>  misc_feature
<222>  (1)..(1)
<223>  n is C12-AMINO and dT18 at 5'

<400>  499
ncatatgttg aatttttgtt ttgttttctg t                                    31


<210>  500
<211>  21
<212>  DNA
<213>  Artificial Sequence

<220>
<223>  nucleotide sequence of matrix probe for detecting mutations in
       BRCA2 gene


<220>
<221>  misc_feature
<222>  (1)..(1)
<223>  n is C12-AMINO and dT18 at 5′

<400>  500
ngaccagaat ccaaatcagg c                                               21


<210>  501
<211>  27
<212>  DNA
<213>  Artificial Sequence

<220>
<223>  nucleotide sequence of matrix probe for detecting mutations in
       BRCA1 and/or BRCA2 gene


<220>
<221>  misc_feature
<222>  (1)..(1)
<223>  n is C12-AMINO and dT18 at 5′

<400>  501
nacgtacgta cgtacgtacg tacgtcy                                         27


<210>  502
<211>  22
<212>  DNA
<213>  Artificial Sequence

<220>
<223>  starter for multiplex amplificantion of BRCA1 gene fragments

<400>  502
gacgttgtca ttagttcttt gg                                              22


<210>  503
<211>  20
<212>  DNA
<213>  Artificial Sequence

<220>
<223>  starter for multiplex amplificantion of BRCA1 gene fragments

<400>  503

ggtcaattct gttcatttgc                                                    20

<210>  504
<211>  20
<212>  DNA
<213>  Artificial Sequence

<220>
<223>  starter for multiplex amplificantion of BRCA1 gene fragments

<400>  504
ctcttaaggg cagttgtgag                                                    20

<210>  505
<211>  22
<212>  DNA
<213>  Artificial Sequence

<220>
<223>  starter for multiplex amplificantion of BRCA1 gene fragments

<400>  505
atggttttat aggaacgcta tg                                                 22

<210>  506
<211>  21
<212>  DNA
<213>  Artificial Sequence

<220>
<223>  starter for multiplex amplificantion of BRCA1 gene fragments

<400>  506
tgttagctga ctgatgatgg t                                                  21

<210>  507
<211>  24
<212>  DNA
<213>  Artificial Sequence

<220>
<223>  starter for multiplex amplificantion of BRCA1 gene fragments

<400>  507
aacccagcaa ttattattaa atac                                               24

<210>  508
<211>  24
<212>  DNA
<213>  Artificial Sequence

<220>
<223>  starter for multiplex amplificantion of BRCA1 gene fragments

<400>  508
gatttccacc tccaaggtgt atga                                               24

<210>  509
<211>  18
<212>  DNA
<213>  Artificial Sequence

<220>
<223>  starter for multiplex amplificantion of BRCA1 gene fragments

<400>  509
catggctcca catgcaag                                                     18


<210>  510
<211>  17
<212>  DNA
<213>  Artificial Sequence

<220>
<223>  starter for multiplex amplificantion of BRCA1 gene fragments

<400>  510
cagctgagag gcatcca                                                      17


<210>  511
<211>  19
<212>  DNA
<213>  Artificial Sequence

<220>
<223>  starter for multiplex amplificantion of BRCA1 gene fragments

<400>  511
ggattctctg agcatggca                                                    19


<210>  512
<211>  19
<212>  DNA
<213>  Artificial Sequence

<220>
<223>  starter for multiplex amplificantion of BRCA1 gene fragments

<400>  512
gatcccctgt gtgagagaa                                                    19


<210>  513
<211>  24
<212>  DNA
<213>  Artificial Sequence

<220>
<223>  starter for multiplex amplificantion of BRCA1 gene fragments

<400>  513
ttactctcta ctgatttgga gtga                                              24


<210>  514
<211>  20
<212>  DNA

<213>  Artificial Sequence

<220>
<223>  starter for multiplex amplificantion of BRCA1 gene fragments

<400>  514
agtgatcctc atgaggcttt                                                   20


<210>  515
<211>  19
<212>  DNA
<213>  Artificial Sequence

<220>
<223>  starter for multiplex amplificantion of BRCA1 gene fragments

<400>  515
aggcctgatg taggtctcc                                                    19


<210>  516
<211>  19
<212>  DNA
<213>  Artificial Sequence

<220>
<223>  starter for multiplex amplificantion of BRCA1 gene fragments

<400>  516
tacaagagcg tcccctcac                                                    19


<210>  517
<211>  20
<212>  DNA
<213>  Artificial Sequence

<220>
<223>  starter for multiplex amplificantion of BRCA1 gene fragments

<400>  517
ttttcgagtg attctattgg                                                   20


<210>  518
<211>  20
<212>  DNA
<213>  Artificial Sequence

<220>
<223>  starter for multiplex amplificantion of BRCA1 gene fragments

<400>  518
gagcagaatg gtcaagtgat                                                   20


<210>  519
<211>  19
<212>  DNA
<213>  Artificial Sequence

<220>

<223>  starter for multiplex amplificantion of BRCA1 gene fragments

<400>  519
caagcgcatg aatatgcct                                                    19


<210>  520
<211>  19
<212>  DNA
<213>  Artificial Sequence

<220>
<223>  starter for multiplex amplificantion of BRCA1 gene fragments

<400>  520
tgtcaatcct agccttcca                                                    19


<210>  521
<211>  21
<212>  DNA
<213>  Artificial Sequence

<220>
<223>  starter for multiplex amplificantion of BRCA1 gene fragments

<400>  521
aaagccttct gtgtcatttc t                                                 21


<210>  522
<211>  20
<212>  DNA
<213>  Artificial Sequence

<220>
<223>  starter for multiplex amplificantion of BRCA1 gene fragments

<400>  522
ccaagggact aattcatggt                                                   20


<210>  523
<211>  23
<212>  DNA
<213>  Artificial Sequence

<220>
<223>  starter for multiplex amplificantion of BRCA1 gene fragments

<400>  523
ttaactgtct gtacaggctt gat                                               23


<210>  524
<211>  22
<212>  DNA
<213>  Artificial Sequence

<220>
<223>  starter for multiplex amplificantion of BRCA1 gene fragments

<400>  524

gaaacaaagt ccaaaagtca ct                                                22

<210>  525
<211>  19
<212>  DNA
<213>  Artificial Sequence

<220>
<223>  starter for multiplex amplificantion of BRCA1 gene fragments

<400>  525
acttgatggg aaaaagtgg                                                   19

<210>  526
<211>  24
<212>  DNA
<213>  Artificial Sequence

<220>
<223>  starter for multiplex amplificantion of BRCA1 gene fragments

<400>  526
cattactcca aataaacatg gact                                             24

<210>  527
<211>  19
<212>  DNA
<213>  Artificial Sequence

<220>
<223>  starter for multiplex amplificantion of BRCA1 gene fragments

<400>  527
tactggagcc cacttcatt                                                   19

<210>  528
<211>  19
<212>  DNA
<213>  Artificial Sequence

<220>
<223>  starter for multiplex amplificantion of BRCA1 gene fragments

<400>  528
tgggaaatga gaacattcc                                                   19

<210>  529
<211>  23
<212>  DNA
<213>  Artificial Sequence

<220>
<223>  starter for multiplex amplificantion of BRCA1 gene fragments

<400>  529
caggaagact ttgtttatag acc                                              23

<210>  530
<211>  23
<212>  DNA
<213>  Artificial Sequence

<220>
<223>  starter for multiplex amplificantion of BRCA1 gene fragments

<400>  530
gcagaactag gtagaaacag agg                                              23


<210>  531
<211>  23
<212>  DNA
<213>  Artificial Sequence

<220>
<223>  starter for multiplex amplificantion of BRCA1 gene fragments

<400>  531
tttccttaat gtcattttca gca                                              23


<210>  532
<211>  23
<212>  DNA
<213>  Artificial Sequence

<220>
<223>  starter for multiplex amplificantion of BRCA1 gene fragments

<400>  532
ttagatgatg gtgaaataaa gga                                              23


<210>  533
<211>  24
<212>  DNA
<213>  Artificial Sequence

<220>
<223>  starter for multiplex amplificantion of BRCA1 gene fragments

<400>  533
caatgataat aaattctcct ctgt                                             24


<210>  534
<211>  20
<212>  DNA
<213>  Artificial Sequence

<220>
<223>  starter for multiplex amplificantion of BRCA1 gene fragments

<400>  534
tagcaccgtt gctaccgagt                                                  20


<210>  535
<211>  17
<212>  DNA

<213>  Artificial Sequence

<220>
<223>  starter for multiplex amplificantion of BRCA1 gene fragments

<400>  535
tttcttccaa gcccgtt                                                    17


<210>  536
<211>  19
<212>  DNA
<213>  Artificial Sequence

<220>
<223>  starter for multiplex amplificantion of BRCA1 gene fragments

<400>  536
agccagggag ttggtctgg                                                  19


<210>  537
<211>  22
<212>  DNA
<213>  Artificial Sequence

<220>
<223>  starter for multiplex amplificantion of BRCA1 gene fragments

<400>  537
cccaaaagca taaacattta gc                                              22


<210>  538
<211>  22
<212>  DNA
<213>  Artificial Sequence

<220>
<223>  starter for multiplex amplificantion of BRCA1 gene fragments

<400>  538
gcgtttatag tctgctttta ca                                              22


<210>  539
<211>  21
<212>  DNA
<213>  Artificial Sequence

<220>
<223>  starter for multiplex amplificantion of BRCA1 gene fragments

<400>  539
tgtcagcaaa cctaagaatg t                                               21


<210>  540
<211>  21
<212>  DNA
<213>  Artificial Sequence

<220>

<223>  starter for multiplex amplificantion of BRCA1 gene fragments

<400>  540
aatggaaagc ttctcaaagt a                                                21


<210>  541
<211>  21
<212>  DNA
<213>  Artificial Sequence

<220>
<223>  starter for multiplex amplificantion of BRCA1 gene fragments

<400>  541
atgttggagc taggtcctta c                                                21


<210>  542
<211>  22
<212>  DNA
<213>  Artificial Sequence

<220>
<223>  starter for multiplex amplificantion of BRCA1 gene fragments

<400>  542
aattcttaac agagaccaga ac                                               22


<210>  543
<211>  22
<212>  DNA
<213>  Artificial Sequence

<220>
<223>  starter for multiplex amplificantion of BRCA1 gene fragments

<400>  543
aaaactcttt ccagaatgtt gt                                               22


<210>  544
<211>  21
<212>  DNA
<213>  Artificial Sequence

<220>
<223>  starter for multiplex amplificantion of BRCA1 gene fragments

<400>  544
ggctctttag cttcttagga c                                                21


<210>  545
<211>  19
<212>  DNA
<213>  Artificial Sequence

<220>
<223>  starter for multiplex amplificantion of BRCA1 gene fragments

<400>  545

ctcagactca agcatcagc                                                    19


<210>  546
<211>  20
<212>  DNA
<213>  Artificial Sequence

<220>
<223>  starter for multiplex amplificantion of BRCA1 gene fragments

<400>  546
atatgacgtg tctgctccac                                                   20


<210>  547
<211>  20
<212>  DNA
<213>  Artificial Sequence

<220>
<223>  starter for multiplex amplificantion of BRCA1 gene fragments

<400>  547
agtcttacaa aatgaagcgg                                                   20


<210>  548
<211>  19
<212>  DNA
<213>  Artificial Sequence

<220>
<223>  starter for multiplex amplificantion of BRCA1 gene fragments

<400>  548
cagagcaaga ccctgtctc                                                    19


<210>  549
<211>  17
<212>  DNA
<213>  Artificial Sequence

<220>
<223>  starter for multiplex amplificantion of BRCA1 gene fragments

<400>  549
gacattttag ccattca                                                      17


<210>  550
<211>  22
<212>  DNA
<213>  Artificial Sequence

<220>
<223>  starter for multiplex amplificantion of BRCA2 gene fragments

<400>  550
ataactgaaa tcaccaaaag tg                                                22

<210>  551
<211>  22
<212>  DNA
<213>  Artificial Sequence

<220>
<223>  starter for multiplex amplificantion of BRCA2 gene fragments

<400>  551
ctgtagttca actaaacaga gg                                                22


<210>  552
<211>  24
<212>  DNA
<213>  Artificial Sequence

<220>
<223>  starter for multiplex amplificantion of BRCA2 gene fragments

<400>  552
aacccatttt caagaactct acca                                              24


<210>  553
<211>  23
<212>  DNA
<213>  Artificial Sequence

<220>
<223>  starter for multiplex amplificantion of BRCA2 gene fragments

<400>  553
ctgaagctac ctccaaaact gtg                                               23


<210>  554
<211>  26
<212>  DNA
<213>  Artificial Sequence

<220>
<223>  starter for multiplex amplificantion of BRCA2 gene fragments

<400>  554
cttcaagtaa atgtcatgat tctgtc                                            26


<210>  555
<211>  24
<212>  DNA
<213>  Artificial Sequence

<220>
<223>  starter for multiplex amplificantion of BRCA2 gene fragments

<400>  555
ctggcagcag tatatttgtt atct                                              24


<210>  556
<211>  20
<212>  DNA

<213>  Artificial Sequence

<220>
<223>  starter for multiplex amplificantion of BRCA2 gene fragments

<400>  556
aaccagaaag aataaatact                                                    20


<210>  557
<211>  22
<212>  DNA
<213>  Artificial Sequence

<220>
<223>  starter for multiplex amplificantion of BRCA2 gene fragments

<400>  557
tcctcaacgc aaatatcttc at                                                 22


<210>  558
<211>  19
<212>  DNA
<213>  Artificial Sequence

<220>
<223>  starter for multiplex amplificantion of BRCA2 gene fragments

<400>  558
caccttgtga tgttagttt                                                     19


<210>  559
<211>  25
<212>  DNA
<213>  Artificial Sequence

<220>
<223>  starter for multiplex amplificantion of BRCA2 gene fragments

<400>  559
ttgggatatt aaatgttctg gagta                                              25


<210>  560
<211>  22
<212>  DNA
<213>  Artificial Sequence

<220>
<223>  starter for multiplex amplificantion of BRCA2 gene fragments

<400>  560
aaagtaacga acattcagac ca                                                 22


<210>  561
<211>  22
<212>  DNA
<213>  Artificial Sequence

<220>

<223>  starter for multiplex amplificantion of BRCA2 gene fragments

<400>  561
ctgggtttct cttatcaaca cg                                                22


<210>  562
<211>  21
<212>  DNA
<213>  Artificial Sequence

<220>
<223>  starter for multiplex amplificantion of BRCA2 gene fragments

<400>  562
agtcttcact attcacctac g                                                 21


<210>  563
<211>  20
<212>  DNA
<213>  Artificial Sequence

<220>
<223>  starter for multiplex amplificantion of BRCA2 gene fragments

<400>  563
gtgagacttt ggttcctaat                                                   20


<210>  564
<211>  21
<212>  DNA
<213>  Artificial Sequence

<220>
<223>  starter for multiplex amplificantion of BRCA2 gene fragments

<400>  564
ttcaacaaga caaacaacag t                                                 21


<210>  565
<211>  24
<212>  DNA
<213>  Artificial Sequence

<220>
<223>  starter for multiplex amplificantion of BRCA2 gene fragments

<400>  565
tgtcagttca tcatcttcca taaa                                              24


<210>  566
<211>  20
<212>  DNA
<213>  Artificial Sequence

<220>
<223>  starter for multiplex amplificantion of BRCA2 gene fragments

<400>  566

ggccaggggt tgtgcttttt                                                    20

<210>  567
<211>  21
<212>  DNA
<213>  Artificial Sequence

<220>
<223>  starter for multiplex amplificantion of BRCA2 gene fragments

<400>  567
aggatactag ttaatgaaat a                                                  21

<210>  568
<211>  20
<212>  DNA
<213>  Artificial Sequence

<220>
<223>  starter for multiplex amplificantion of BRCA2 gene fragments

<400>  568
gcagataccc aaaaagtggc                                                    20

<210>  569
<211>  20
<212>  DNA
<213>  Artificial Sequence

<220>
<223>  starter for multiplex amplificantion of BRCA2 gene fragments

<400>  569
tctggacctc ccaaaaactg                                                    20

<210>  570
<211>  20
<212>  DNA
<213>  Artificial Sequence

<220>
<223>  starter for multiplex amplificantion of BRCA2 gene fragments

<400>  570
cactgtgcct ggcctgatac                                                    20

<210>  571
<211>  21
<212>  DNA
<213>  Artificial Sequence

<220>
<223>  starter for multiplex amplificantion of BRCA2 gene fragments

<400>  571
atgttaaatt caaagtctct a                                                  21

<210>  572
<211>  24
<212>  DNA
<213>  Artificial Sequence

<220>
<223>  starter for multiplex amplificantion of BRCA2 gene fragments

<400>  572
acttcttcca ttgcatcttt ctca                                              24


<210>  573
<211>  24
<212>  DNA
<213>  Artificial Sequence

<220>
<223>  starter for multiplex amplificantion of BRCA2 gene fragments

<400>  573
aaaacaaaac aaaaattcaa cata                                              24


<210>  574
<211>  20
<212>  DNA
<213>  Artificial Sequence

<220>
<223>  starter for multiplex amplificantion of BRCA2 gene fragments

<400>  574
gcagcgacaa aaaaaactca                                                   20


<210>  575
<211>  20
<212>  DNA
<213>  Artificial Sequence

<220>
<223>  starter for multiplex amplificantion of BRCA2 gene fragments

<400>  575
atttgccaac tggtagctcc                                                   20


<210>  576
<211>  20
<212>  DNA
<213>  Artificial Sequence

<220>
<223>  starter for multiplex amplificantion of BRCA2 gene fragments

<400>  576
gctttcgcca aattcagcta                                                   20


<210>  577
<211>  21
<212>  DNA

<213>  Artificial Sequence

<220>
<223>  starter for multiplex amplificantion of BRCA2 gene fragments

<400>  577
taccaaaatg tgtggtgatg c                                                21